# EUROPEAN PATENT SPECIFICATION

(11) **EP 3 911 766 B1**
(45) Date of publication and mention of the grant of the patent: **29.07.2026**
(21) Application number: 20706079.9
(22) Date of filing: 20.01.2020
(51) Int. Cl.: C12Q 1/689

(54) **MONITORING TOOLS AND DIAGNOSTIC METHODS FOR DETERMINING A CANID'S MICROBIOME AGE STATUS**
ÜBERWACHUNGSWERKZEUGE UND DIAGNOSEVERFAHREN ZUR BESTIMMUNG DES MIKROBIOM-ALTERSSTATUS EINES CANID
OUTILS DE SURVEILLANCE ET MÉTHODES DE DIAGNOSTIC POUR DÉTERMINER L'ÉTAT D'ÂGE DU MICROBIOME D'UN CANIDÉ

(30) Priority: 18.01.2019 GB 201900750
(43) Date of publication of application: 24.11.2021
(73) Proprietor: Mars, Incorporated, McLean, VA 22101 (US)
(72) Inventor: MARSHALL-JONES, Zoe V., Melton Mowbray Leicestershire LE14 4RS (GB); WRIGGLESWORTH, David J., Melton Mowbray Leicestershire LE14 4RS (GB); STAUNTON, Ruth, Melton Mowbray Leicestershire LE14 4RS (GB); LONSDALE, Zoe, Melton Mowbray Leicestershire LE14 4RS (GB); WATSON, Phillip, Melton Mowbray Leicestershire LE14 4RS (GB); HAYDOCK, Richard, Melton Mowbray Leicestershire LE14 4RS (GB)
(74) Representative: Haseltine Lake Kempner LLP
(86) International application number: PCT/US2020/014297
(87) International publication number: WO 2020/150717

(56) References cited:
- CONNIE A ROJAS ET AL: "ABSTRACT", FEMS MICROBIOLOGY ECOLOGY, vol. 96, no. 2, 1 February 2020 (2020-02-01), NL, XP055694361, ISSN: 0168-6496, DOI: 10.1093/femsec/fiaa007
- BLAKE C. GUARD ET AL: "Characterization of the fecal microbiome during neonatal and early pediatric development in puppies", PLOS ONE, vol. 12, no. 4, 27 April 2017 (2017-04-27), pages e0175718, XP055694651, DOI: 10.1371/journal.pone.0175718
- CHRISTINA D. MOON ET AL: "Metagenomic insights into the roles of Proteobacteria in the gastrointestinal microbiomes of healthy dogs and cats", MICROBIOLOGYOPEN, vol. 7, no. 5, 17 June 2018 (2018-06-17), pages e00677, XP055694653, ISSN: 2045-8827, DOI: 10.1002/mbo3.677
- ÅSA VILSON ET AL: "Disentangling factors that shape the gut microbiota in German Shepherd dogs", PLOS ONE, vol. 13, no. 3, 23 March 2018 (2018-03-23), pages e0193507, XP055694665, DOI: 10.1371/journal.pone.0193507
- MIZUKAMI KEIJIRO ET AL: "Age-related analysis of the gut microbiome in a purebred dog colony", vol. 366, no. 8, 1 April 2019 (2019-04-01), pages fnz095 - 1, XP009520370, ISSN: 1574-6968, Retrieved from the Internet <URL:http://academic.oup.com/femsle/advance-article-pdf/doi/10.1093/femsle/fnz095/28552648/fnz095.pdf> [retrieved on 20190502], DOI: 10.1093/FEMSLE/FNZ095

## Description

### TECHNICAL FIELD

This present disclosure is in the field of monitoring tools and diagnostic methods for determining a canid's microbiome age status, and comparing the microbiome biological age status of the canid to the chronological age of the canid, and therein assessing the health of the animal.

### BACKGROUND TO THE INVENTION

The understanding of the microbiome and its impact on health has increased significantly in recent years. Changes in the microbiome, and its interaction with the immune, endocrine and nervous systems are correlated with a wide array of health conditions and illnesses, ranging from inflammatory bowel disease [1-3] to cancer [4] and to behavioral aspects of host health [5;6].

The establishment of the microbiome occurs at the same time as development of the immune system and plays a role in intestinal physiology and regulation. The initial establishment of the gut microbiota is an essential step in neonatal development, influencing immunological development in infancy and health throughout life. As such in humans and many mammals a rapid increase in diversity occurs in the early establishment phase of gut microbiome development [7].

The adult gut microbiome can be resilient to large shifts in community structure and in humans and other mammals, it is considered to be relatively stable throughout adult life. This "adult microbiome" is considered to represent a healthy gut microbiome for dogs with enhanced resilience compared to other lifestages. In early lifestages, puppies have an undeveloped gut barrier, which includes the gastrointestinal microbiome as well as histological and gut associated immune functions. Puppies and young dogs are therefore are more prone to gastrointestinal illnesses such as diarrhoea and sickness, etc. Senior and geriatric dogs are also more prone to diarrhoea and gastrointestinal complications, which can occur in part as a result of a deterioration in the gut microbiome.

Given the importance of the microbiome to health and wellbeing, it is important to find ways to determine the status of the microbiome of an animal because of the inherent changes in the gut barrier and gastrointestinal health with age, this status can be considered in terms of the age status of the microbiome.

Blake C. Guard et al, "Characterization of the fecal microbiome during neonatal and early pediatric development in puppies", PLOS ONE, vol. 12, no. 4, (2017), page e0175718, DOI: 10.1371/journal.pone.0175718 characterises the fecal microbiota from 2 to 56 days in growing puppies using 454-pyrosequencing.

Asa Vilson et al, "Disentangling factors that shape the gut microbiota in German Shepherd dogs" PLOS ONE, vol. 13, no. 3, (2018), page e0193507, DOI: 10.1371/journal.pone.0193507 studies the development of the gut microbiota in 168 German Shepherd dogs from 7 weeks to 18 months of age to study the effect of relatedness, maternal microbiota composition and living environment in a large and well-defined population of dogs.

### SUMMARY OF THE INVENTION

The present invention is defined by the appended claims.

Disclosed herein are diagnostic methods, which allow the determination of a canid's microbiome biological age status, wherein the method further comprises comparing the microbiome biological age status to the chronological age of the canid. The methods of the present disclosure can achieve this with high accuracy, as shown in the examples.

In a first aspect, the present disclosure provides a method of determining the microbiome age status of a canid, comprising (a) detecting one or more bacterial taxa in a sample obtained from the canid; (b) correlating the one or more bacterial taxa in the sample to a control data set; and (c) determining the microbiome age, wherein the method further comprises comparing the microbiome biological age status of the canid to the chronological age of the canid. These methods are particularly useful for assessing a canid's health as a discrepancy between the microbiome age and the canid's actual age can be indicative of its health status. For example, in some embodiments, it would be undesirable for an adult dog to have a juvenile microbiome as this is associated with instability or low diversity and result in associated health conditions such as the susceptibility to diarrhoea.

In another aspect, provided is a method of monitoring a canid, comprising a step of determining the microbiome age of the canid by a method of the first aspect on at least two time points. This is particularly useful where a canid is receiving treatment to shift the microbiome as it can monitor the progress of the therapy. In some embodiments, it is also useful for monitoring health of the canid as a rapid shift from, for example, an adult microbiome to a senior microbiome, can be indicative of disease. This aspect of the present disclosure can also be used to assess whether the canid's microbiome progresses as the animal gets older.

Also disclosed herein, is a dietary change, through consumption of a functional food, administration of a nutraceutical or pharmaceutical composition or of a preparation comprising bacteria for use in the method of changing the composition of the microbiome of a canid, wherein the method comprises determining the microbiome age status of a canid, comprising (a) detecting one or more bacterial taxa in a sample obtained from the canid; (b) correlating the one or more bacterial taxa in the sample to a control data set; and (c) determining the microbiome age, wherein the method further comprises comparing the microbiome biological age status of the canid to the chronological age of the canid, and changing the composition of the microbiome through a dietary change, consumption of a functional food, or administration of a nutraceutical or pharmaceutical composition or of a preparation comprising bacteria. This is particularly useful where the methods of the present disclosure have identified a microbiome biological age status that is not consistent with the canid's actual age and where the canid can therefore be healthier or less healthy in the context of the animal's actual age.

In another aspect, also provided is a method of monitoring the microbiome age in a canid who has undergone a dietary change or who has received a functional food, nutraceutical or pharmaceutical composition that is able to change the microbiome composition, comprising determining the microbiome biological age status by a method according to the first aspect. Such methods allow a skilled person to determine the success of the treatment. In particular embodiments, these methods comprise determining the microbiome age before and after treatment as this helps to evaluate the success of the treatment.

As noted above, in a particular embodiment, the disclosed subject matter provides a method of determining the microbiome biological age status of a canid, comprising (a) detecting one or more bacterial taxa in a sample obtained from the canid; (b) correlating the one or more bacterial taxa in the sample to a control data set; and (c) determining the microbiome biological age status, wherein the method further comprises comparing the microbiome biological age status of the canid to the chronological age of the canid. In a particular embodiment of the claimed method, the control data set comprises microbiome data of canids at different times or life stages. In another embodiment of the claimed method, the control data set comprises microbiome data from at least two lifestages of a canid selected from the list consisting of a puppy, an adult canid, a senior canid and a geriatric canid. In a particular embodiment, the control data set comprises microbiome data from all of puppies, adults, seniors and geriatrics.

In certain embodiments of the claimed methods, the method comprises quantitating the one or more bacterial taxa.

In certain embodiments of the claimed methods, the bacterial taxa are bacterial species from genera selected from the group consisting of: *Lactobacillus, Terrisporobacter, Turicibacter, Bacteroides, Bifidobacterium, Allobaculum, Blautia, Cetobacterium, Clostridium, Dubosiella, Escherichia, Phascolarctobacterium, Streptococcus, Megamonas, Ruminococcus, Fusobacterium, Enterococcus, Lachnoclostridium, Epulopiscium, Erysipelotrichaceae, Dorea, Adlercreutzia, Anaerobiospirillum, Romboutsia, Peptostreptococcaceae,* and *Prevotella,* or bacterial species from the families *Erysipelotrichaceae, Peptostreptococcaceae* or *Lachnospiraceae.*

In certain embodiments of the claimed methods, the bacterial taxa are bacterial species selected from the group consisting of *Adlercreutzia equolifaciens, Anaerobiospirillum sp., Bacteroides sp., Blautia sp., Blautia [Ruminococcus] gnavus, Blautia [Ruminococcus] torques, Blautia obeum, Blautia producta, Cetobacterium somerae, Clostridium, Clostridium celatum, Clostridium dakarense, Clostridium hiranonsis, Clostridium perfringens, Clostridium sp., Clostridium sp.K39, Dorea sp., Dubosiella newyorkensis, Enterococcus asini, Enterococcus casseliflavus, Enterococcus haemoperoxidus, Enterococcus sulfureus, Erysipelotrichaceae sp., Escherichia coli*/*Shigella flexneri, Fusobacterium mortiferum, Lachnoclostridium sp., Lachnospiraceae sp., Lactobacillus animalis, Lactobacillus apodemi, Lactobacillus gasseri, Lactobacillus hamsteri, Lactobacillus helveticus, Lactobacillus johnsonii, Lactobacillus murinus, Lactobacillus reuteri, Lactobacillus taiwanensis, Megamonas sp., Niameybacter massiliensis, Peptostreptococcaceae TM5, Phascolarctobacterium sp., Prevotella sp., Romboutsia sp., Ruminococcus gauvreauii group sp., Streptococcus alactolyticus, Streptococcus luteciae, Terrisporobacter sp.,* and *Turicibacter sp.*

In certain embodiments of the claimed methods, the bacterial species has a 16S rDNA with at least about 95%, at least about 96%, at least about 97%, at least about 98%, at least about 99% or 100% identity to the sequence of any one of SEQ ID NOs: 32-68.

In certain embodiments of the claimed methods, the method comprises the detection of one or more bacterial species from genera selected from the group consisting of *Phascolarctobacterium, Bacteroides* and *Escherichia* /*Shigella.*

In certain embodiments of the claimed methods, the method comprises the detection of one or more bacterial species selected from the group consisting of *Phascolarctobacterium sp., Bacteroides sp.* and *Escherichia coli* /*Shigella flexneri.*

In certain embodiments of the claimed methods, the bacterial species have a 16S rDNA with at least about 95%, at least about 96%, at least about 97%, at least about 98%, at least about 99% or 100% identity to the sequence of any one of SEQ ID NOs: 32-36.

In certain embodiments of the claimed methods, the methods further comprise the detection of *Lactobacillus helveticus, Lactobacillus hamsteri, Terrisporobacter sp., Turicibacter sp., Blautia obeum* and *Blautia sp.*

In certain embodiments of the claimed methods, the bacterial species have a 16S rDNA with at least about 95%, at least about 96%, at least about 97%, at least about 98%, at least about 99% or 100% identity to the sequence of any one of SEQ ID NOs: 37-41.

In certain embodiments of the claimed methods, the methods further comprise the detection of *Clostridium celatum, Clostridium sp., Clostridium disporicum, Clostridium perfringens, Cetobacterium somerae, Lactobacillus johnsonii, Blautia torques, Dubosiella newyorkensis, Streptococcus alactolyticus, Megamonas sp.,* and *Turicibacter sp..*

In certain embodiments of the claimed methods, the bacterial species have a 16S rDNA with at least about 95%, at least about 96%, at least about 97%, at least about 98%, at least about 99% or 100% identity to the sequence of any one of SEQ ID NOs: 32 and 42-50.

In certain embodiments of the claimed methods, the method comprises the detection of all of SEQ ID NOs: 32, and 42 50.

In certain embodiments of the claimed methods, the bacterial species are from bacterial genera selected from the group consisting of *Clostridium, Collinsella, Enterococcus, Eubacterium, Escherichia-Shigella, Flavonifractor, Klebsiella, Lachnoclostridium, Lachnospiraceae, Lactobacillus, Paeniclostridium, Pediococcus, Peptoclostridium, Romboutsia, Staphylococcus,* and *Tyzzerella.*

In certain embodiments of the claimed methods, the bacterial species are selected from the group consisting of *Clostridium sp, Collinsella sp., Enterococcus sp., Escherichia-Shigella sp., Flavonifractor sp., Shigella dysenteriae, Lachnoclostridium sp., Lachnospiraceae sp., Lactobacillus sp., Eubacterium sp., Pediococcus sp., Peptoclostridium sp., Romboutsia sp., Staphylococcus sp.,* and *Tyzzerella sp.*

In certain embodiments of the claimed methods, the bacterial species have a 16S rDNA with at least about 95%, at least about 96%, at least about 97%, at least about 98%, at least about 99% or 100% identity to the sequence of any one of SEQ ID NOs: 3-31.

In certain embodiments of the claimed methods, the bacterium is of the species *Blautia [Ruminococcus] gnavus.*

In certain embodiments of the claimed methods, the bacterium has a 16S rDNA with at least about 95%, at least about 96%, at least about 97%, at least about 98%, at least about 99% or 100% identity with the sequence of SEQ ID NO: 20.

In certain embodiments of the claimed methods, the at least 5, at least 10, at least 15, at least 20, at least 25 or at least 28 different taxa are detected and/or quantitated.

In certain embodiments of the claimed methods, the methods comprise detecting or quantitating *Ruminococcus gnavus, Escherichia sp., Staphylococcus,* and *Flavonifractor.*

In certain embodiments of the claimed methods, the bacterial species have a 16S rDNA with at least about 95%, at least about 96%, at least about 97%, at least about 98%, at least about 99% or 100% identity to the sequence of any one of SEQ ID NOs: 13, 16, 17, 20, and 29.

In certain embodiments of the claimed methods, the method further comprises detecting or quantitating *Romboutsia sp., Peptoclostridium sp., Bacteroides sp., Megamonas sp.* and *Collinsella sp.*

In certain embodiments of the claimed methods, the bacterial species have a 16S rDNA with at least about 95%, at least about 96%, at least about 97%, at least about 98%, at least about 99% or 100% identity to the sequence of any one of SEQ ID NOs: 10, 15, 22, 26, and 27.

In certain embodiments of the claimed methods, the method further comprises detecting or quantitating Clostridium sp., Megamonas sp., Paeniclostridium sp, Tyzzerella sp., Romboutsia sp., and Blautia sp.

In certain embodiments of the claimed methods, the bacterial species have a 16S rDNA with at least about 95%, at least about 96%, at least about 97%, at least about 98%, at least about 99% or 100% identity to the sequence of any one of SEQ ID NOs: 3, 4, 8, 9, 14, 18, 19, 21, 23, and 24.

In certain embodiments of the claimed methods, the bacterial species is detected by means of DNA sequencing, RNA sequencing, protein sequence homology or other biological marker indicative of the bacterial species.

The present disclosure provides a method of determining the microbiome age status of a canid, comprising (a) detecting one or more bacterial taxa in a sample obtained from the canid; (b) correlating the one or more bacterial taxa in the sample to a control data set; and (c) determining the microbiome age, wherein the method further comprises comparing the microbiome biological age status of the canid to the chronological age of the canid. In certain embodiments of the claimed methods, the canid's microbiome biological age status is compared to its biological age and the canid's owner is notified when there is a discrepancy.

In an alternative embodiment, the disclosed subject matter provides a method of monitoring a canid, comprising a step of determining the microbiome biological age status of the animal by the method of the first aspect on at least two time points. In certain embodiments, the two time points are 6 months apart.

In certain embodiments of the claimed methods, the control data set is representative of the microbiome of puppies, a microbiome from an adult canid, a microbiome from a senior canid or a microbiome from a geriatric canid.

In certain embodiments of the claimed methods, the sample is a faecal sample, or a sample from the gastrointestinal tract. In certain embodiments of the claimed methods, the sample is a ileal, jejunal, duodenal or colonic sample.

Also disclosed herein, but not part of the claimed invention, the method further comprises changing the composition of the microbiome administration of a dietary change, a supplement, a functional food, a nutraceutical or a pharmaceutical composition or an exercise/physical activity regimen.

In an alternative embodiment, the disclosed subject matter provides a method of monitoring the microbiome biological age status in a canid that has received a dietary change, a supplement, a functional food, a nutraceutical or a pharmaceutical composition or an exercise/physical activity regimen, which is able to change the microbiome composition, comprising determining the microbiome biological age status by the method of any preceding claim. In certain embodiments, the microbiome age is determined before and after administration of the composition.

In certain embodiments of the claimed methods, the composition comprises bacteria.

In certain embodiments of the claimed methods, the canid is a dog.

### BRIEF DESCRIPTION OF DRAWINGS

**Figure 1****:** Bacterial taxa (OTUs) ranked by importance scores describing consistency in assignment of samples to the appropriate study group (early life, through weaning or later (rapid growth phase of) puppyhood.) Roise = Rares and noise.
**Figure 2****:** Cumulative sensitivity and positive predicted values for successively increasing numbers of OTUs used in the model with OTUs ranked by importance order for young animals (<2 years of age).
**Figures 3A** **and** **3B****:** Figures 3A and 3B each show Bacterial taxa (OTUs) ranked by importance scores describing consistency in assignment of samples to the appropriate canine study group (adult, senior and geriatric dogs).
**Figure 4****:** Cumulative sensitivity and positive predicted values for successively increasing numbers of OTUs ranked on order of importance in predictive model for adult life stages.

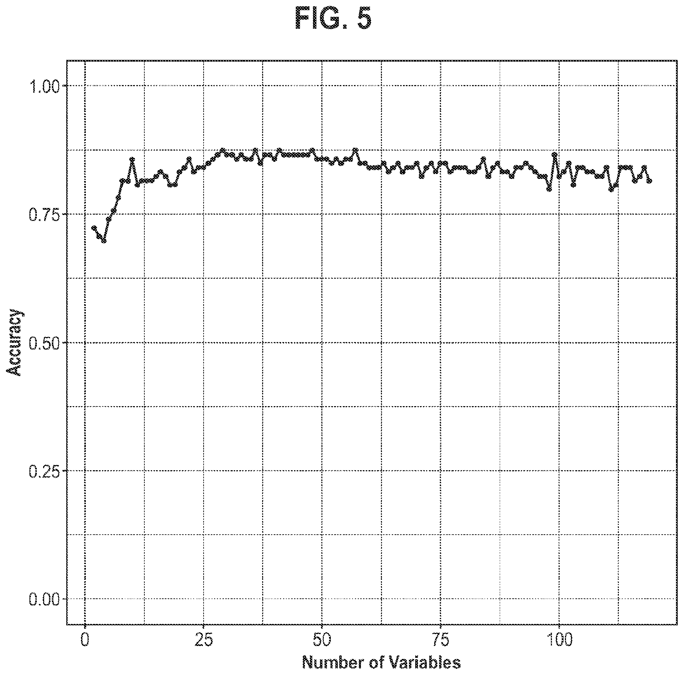

**Figure 5****:** Accuracy scores for successively increasing numbers of OTUs ranked in importance order.

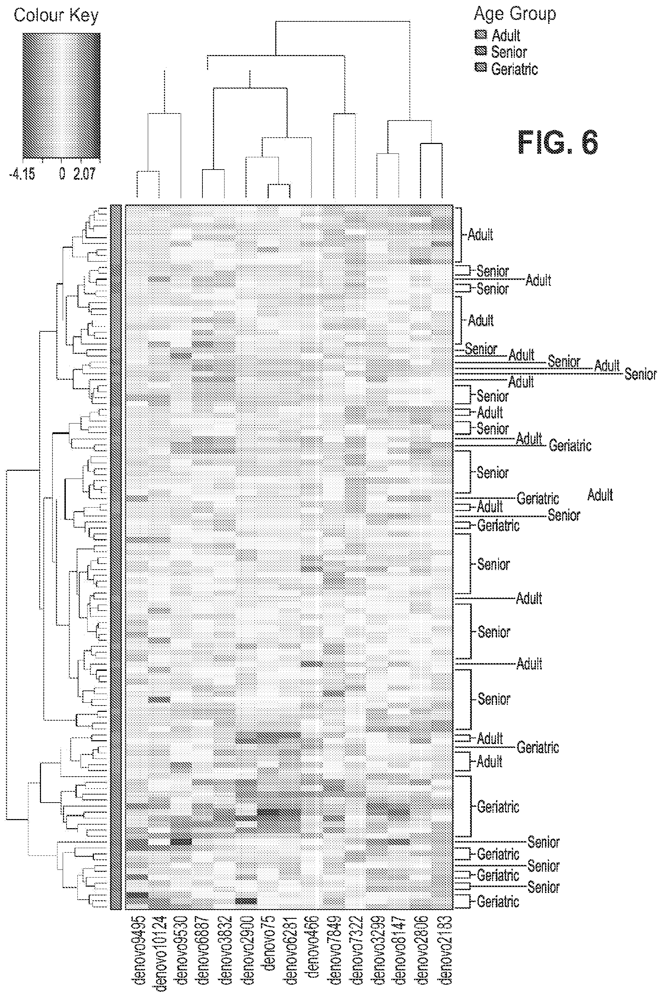

**Figure 6****:** PLSDA correlation plot for clustering of the relative abundance data of a control set of dogs and the test samples using the 15 taxa most influential in detecting biological age (samples represented on the y axis by group with red assigned to adult samples; green to senior and blue indicating samples from geriatric dogs). Faeces samples are represented in individual horizontal rows and are clustered according to likeness while bacterial OTUs are represented by individual columns within the heat plot. Colour coding within the correlation plot is indicative of the degree and direction of correlation.
**Figures 7A** **and** **7B****:** Figures 7A and 7B each show Phylum level summary data, showing change in phylum proportion across time for two independent studies.
**Figures 8A-8H:** Figures 8A through 8H provide stacked bar plots from eight representative dogs within the cohort demonstrating the distribution in the abundant taxonomic groups at each sampling point. Each of Figures 8A - 8H represent a different set of data for an individual dog.
**Figure 9****:** Partial least Square discriminate analysis (PLS-DA) correlation plot based on likeness in bacterial abundance data for the 25 OTUs displaying the greatest influence on clustering of the samples (variable importance in projection scores >1).
**Figure 10** corresponds to Table 1.3, which provides the cumulative prediction scores for the assignment of microbiome to age group based on relative abundance of OTUs ranked by importance. P = position.
**Figure 11** corresponds to Table 2.3, which provides the bacterial taxa useful for determining the microbiome age of a canid.

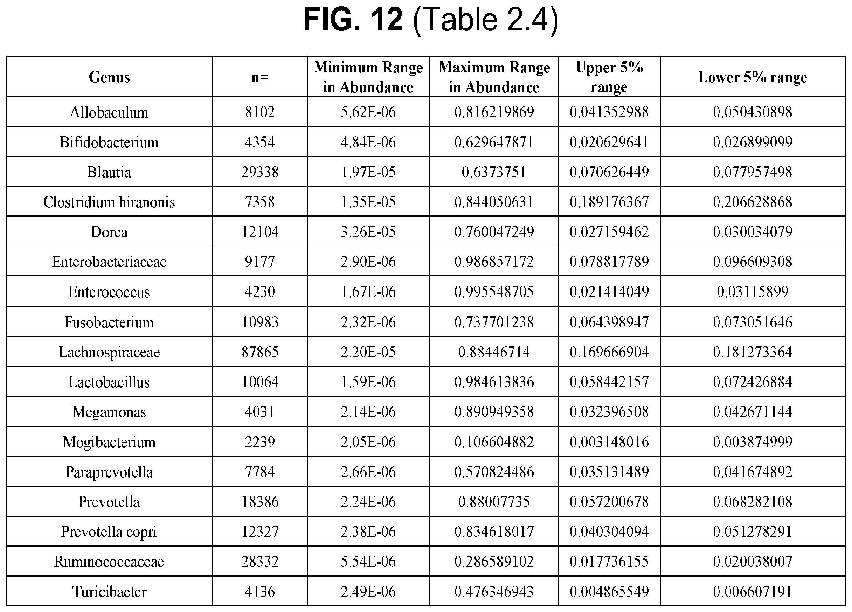

**Figure 12** corresponds to Table 2.4, which provides the range of abundance for various bacterial species indicative of a healthy microbiome in canids.
**Figure 13** corresponds to Table 4, which provides the bacterial taxa that are detected in the gut following treatment with antibiotics.

### DETAILED DESCRIPTION

### The microbiome biological age status

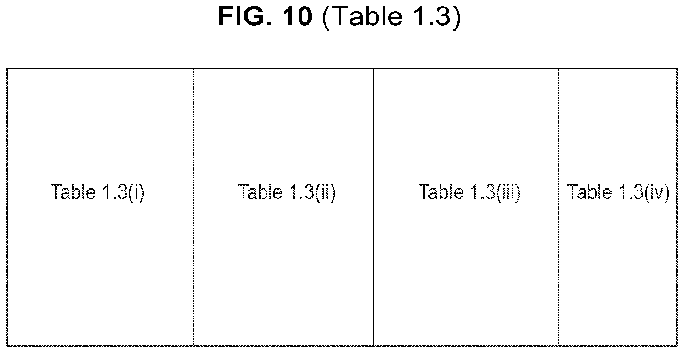

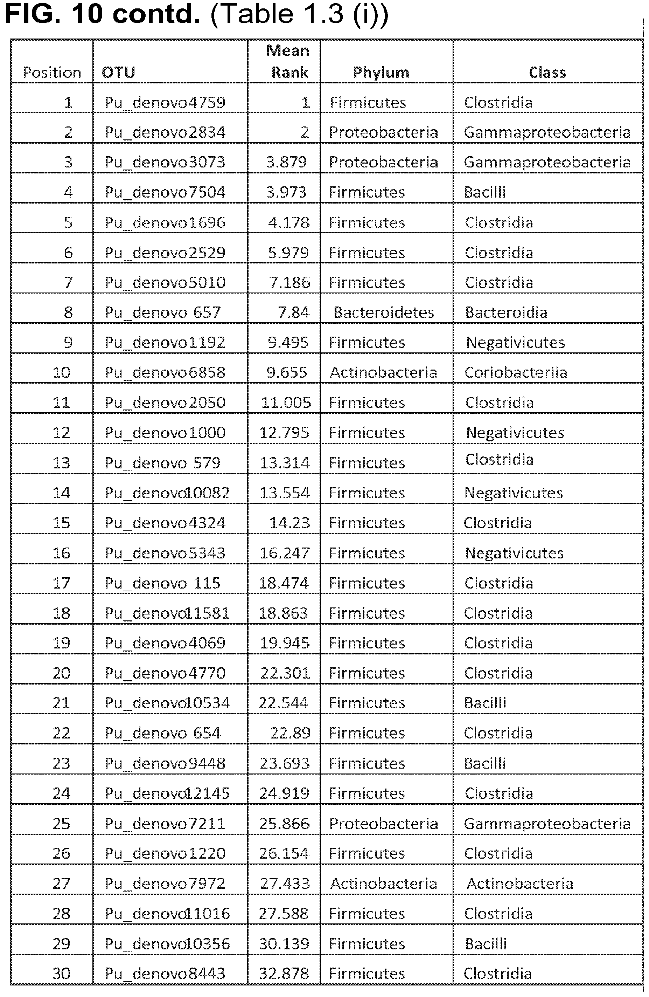

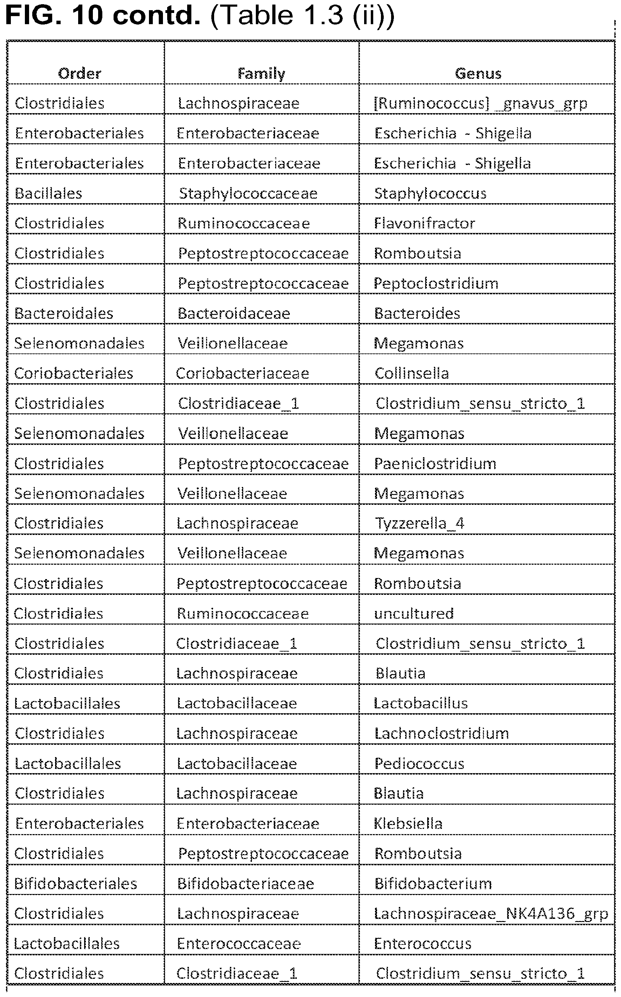

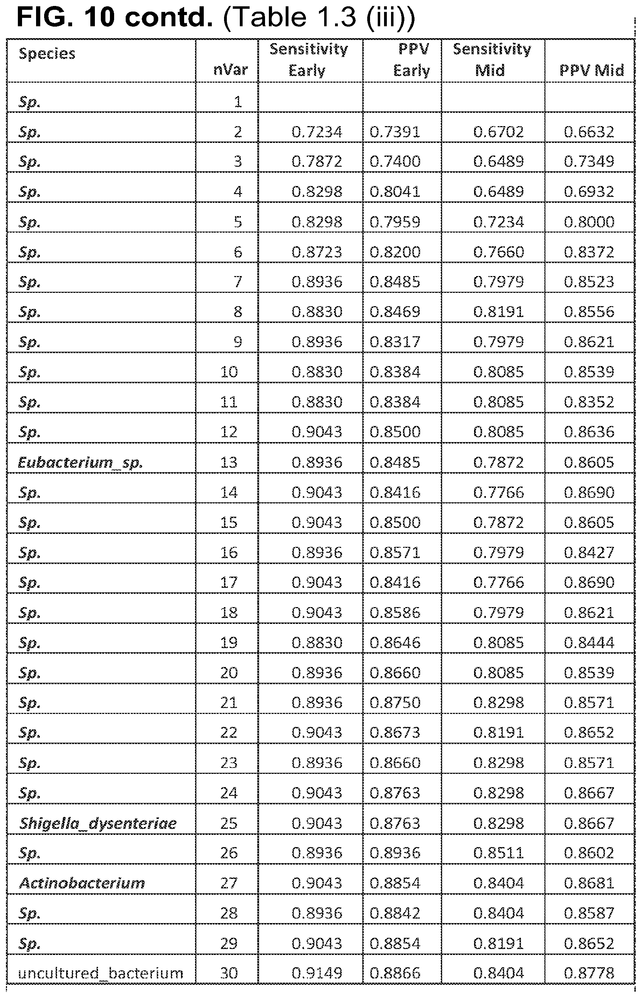

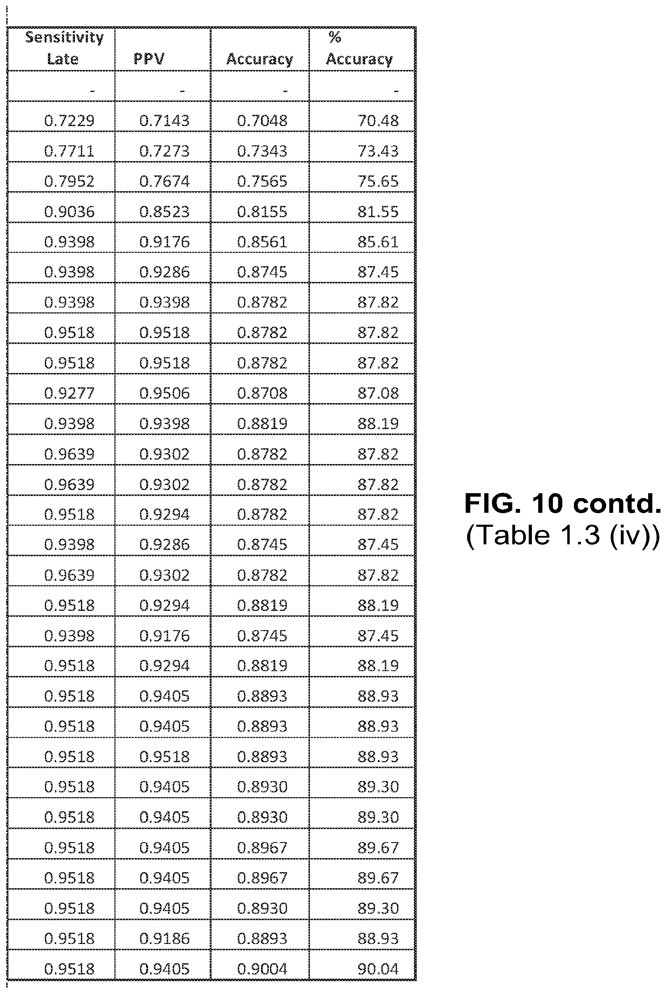

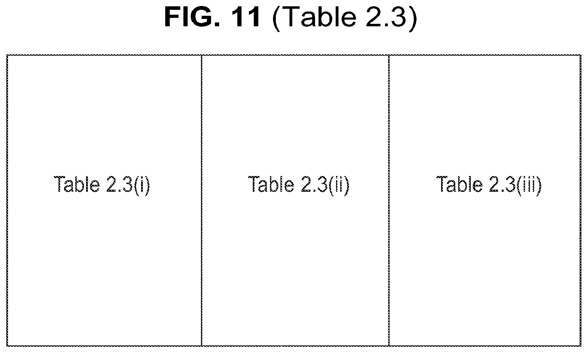

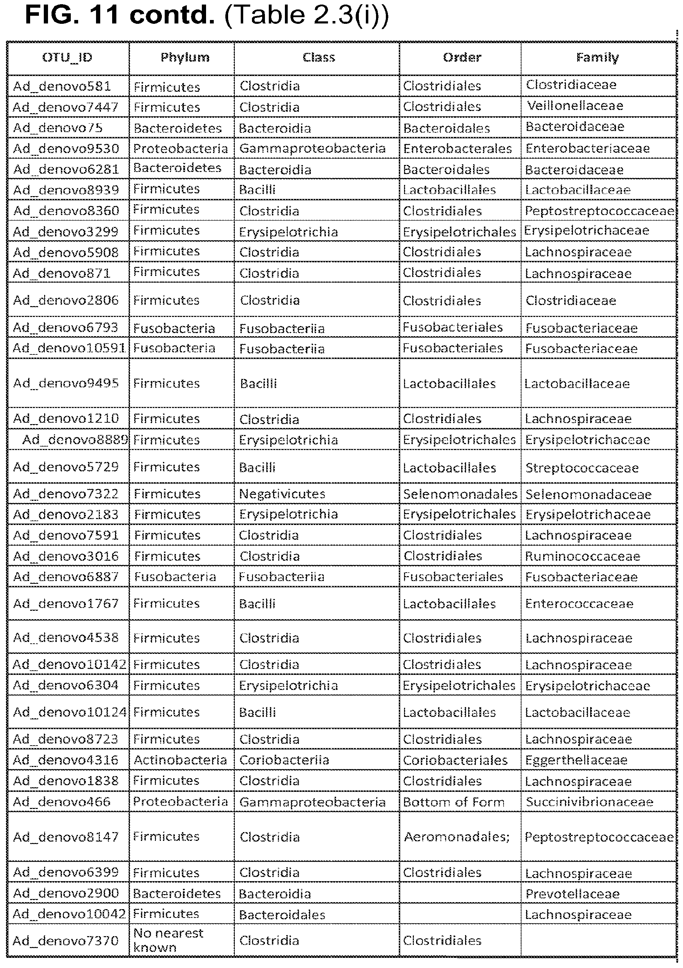

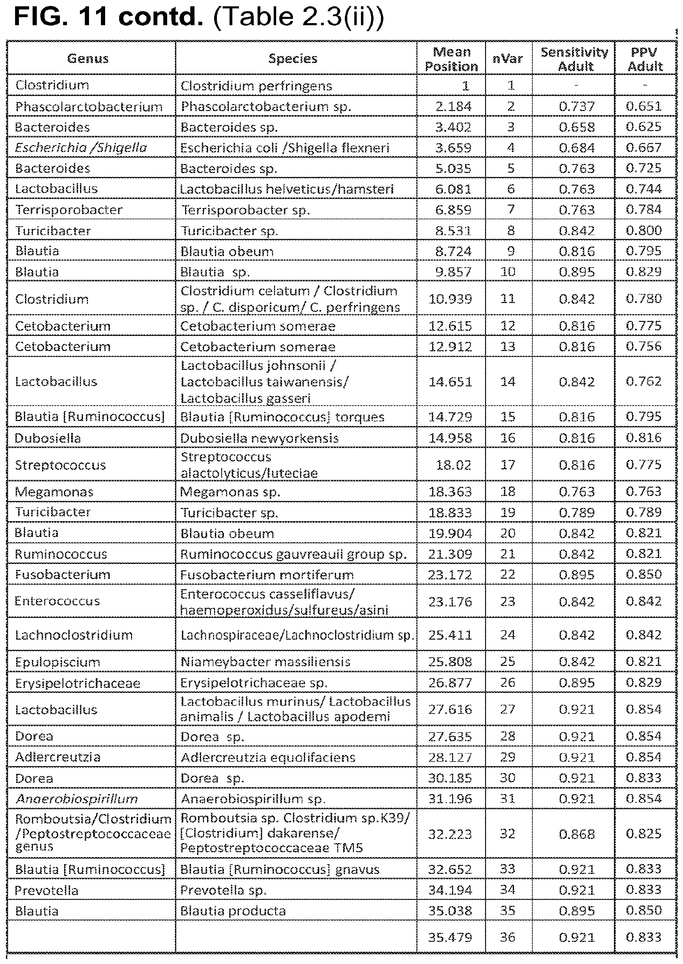

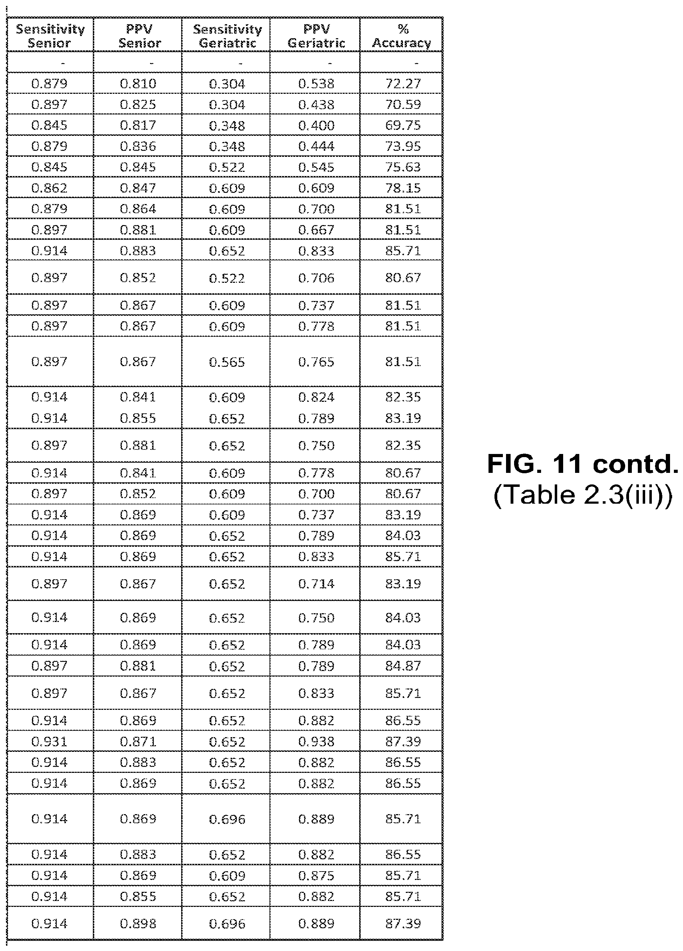

The methods of the present disclosure are used to identify the biological age status of the microbiome from a canid, wherein the method further comprises comparing the microbiome biological age status of the canid to the chronological age of the canid. The microbiome can be assigned to a particular "age" or "lifestage" by comparing the bacterial taxa in the sample and optionally their relative abundance to a control data set. Generally, the control data set will represent the microbiome of a canid with known microbiome composition and diversity ranges for the canid's age. For example, the microbiome composition of a number of canids from a specific age group (puppy, adult, senior, geriatric) can be analysed. In addition, or alternatively, the microbiome composition can be analysed from the same canid at intervals within a life stage or in different life stages. Bacterial taxa which show a high specificity for the different age groups across all tested individuals can then be used as a control data set. The control data set can also be based on the analysis of a single individual. The control data set can also be based on the analysis of the same individual at different life stages. In a particular embodiment, the data set shown in Table 1.1, Table 1.2, Figure 10 (Table 1.3), and Table 2.1, Table 2.2, and Figure 11 (Table 2.3) serve as a control data set in the methods of the present disclosure.

In some embodiments, the microbiome biological age status is determined by comparing the microbiome in the sample to the microbiome composition with a known microbiome age (control microbiome) and determining the microbiome biological age status based on the similarity to the control. Thus, in some embodiments, the control data set can comprise typical microbiomes of canids at different life stages. In particular embodiments, the control data set comprises microbiome data from a puppy, an adult canid, a senior canid and a geriatric canid. The microbiome biological age status can be determined by identifying the control microbiome to which the microbiome of the canid has the most similarity and assigning the corresponding life stage to the canid. These microbiome data can be obtained by the methods discussed above.

In some embodiments, one or a few bacterial taxa (*e.g.,* less than 3, 4, 5, 8, 10, 15, 20, 25 or 30) are detected and/or quantitated. In a particular embodiment, a minimum of two bacterial taxa are analysed as the presently disclosed subject matter has shown that such methods have good sensitivity. These will generally be bacteria which allow an unambiguous allocation to one (or possibly two) of the different life stages. In these embodiments, the one or more bacterial taxa that are assessed are based on a data set which shows that these are indicative of a certain life stage. Thus, in these embodiments, the detection of the taxa of interest and the subsequent assignment of the microbiome biological age status based on these data constitutes correlating the taxa in the sample to a control data set.

In the presently disclosed subject matter, it has been discovered that certain bacterial taxa are highly indicative of the biological age status of the canid's microbiome. The bacterial taxa shown in Table 1.1 have high consistency in correctly assigning samples to dogs at different stages of puppyhood. In some embodiments, the methods of the present disclosure thus allow the determination of the microbiome biological age status of a canid puppy or young dog. In particular, the methods of the present disclosure can be used to determine whether a canid's microbiome is equivalent to that seen in early postpartum puppyhood (between birth and around 12 days), mid puppyhood (between about 17 and about 31 days) or later (rapid growth phase) puppyhood such as the period between about 38 and about 52 days. The results are then assessed to determine whether the microbiome biological age status concurs with the canid's actual age. If not, steps to shift the microbiome biological age status can be taken, as discussed below.

In some embodiments, the bacterial taxa which are analysed in the methods of the present disclosure are quantitated. The presently disclosed subject matter has discovered that the relative abundance of different bacterial taxa within the gut and the faecal microbiome shifts as the dog ages, as discussed in detail in the Examples below. The combination and abundance of the bacterial taxa detected within the microbiome can therefore allow the determination of the biological age status of the microbiome in a dog, as discussed in detail in the Examples below.

The methods of the present disclosure generally detect and/or quantitate one or more different bacterial taxa, for example one or more bacterial genera and/or one or more bacterial species through the detection of gene sequences or other biomarkers.

In addition to those described herein, techniques which allow a skilled person to detect and quantitate bacterial taxa are well known in the art. These include, for example, polymerase chain reaction (PCR), quantitative PCR, 16S rDNA amplicon sequencing, shotgun sequencing, metagenome sequencing, Illumina sequencing, and nanopore DNA sequencing techniques. In a particular embodiment, the bacterial taxa are determined by sequencing the 16S rDNA sequence. Other methods would include shotgun sequencing to determine characteristic non-16SrDNA gene sequences or other metabolites and biomarkers for identification of the species.

In some embodiments, the bacterial taxa are determined by sequencing the V4-V6 region, for example using Illumina DNA sequencing techniques. These methods can use the primers 319F: CAAGCAGAAGACGGCATACGAGATGTGACTGGAGTTCAGACGTGTGCTCTTCCGATCT (SEQ ID NO: 1) and 806R: AATGATACGGCGACCACCGAGATCTACACTCTTTCCCTACACG ACGCTCTTCCGATCT (SEQ ID NO: 2).

In further embodiments, the bacterial species can be detected by other means known in the art such as, for example, RNA sequencing, protein sequence homology or other biological marker indicative of the bacterial species.

The sequencing data can then be used to determine the presence or absence of different bacterial taxa in the sample. For example, the sequences can be clustered at about 98%, about 99% or 100% identity and bacterial taxa (e.g., abundant taxa representing more than 0.01% or 0.05% of the total sequences) can then be assessed for their relative proportions. Suitable techniques for determining the descriptive nature of bacterial taxa and biomarkers or combinations of bacteria or biomarkers for their ability to assign a sample to a particular study group such as an age range are known in the art and include, for example, logistic regression analysis; partial least squares discriminate analysis (PLSDA) or random forest analysis and other multivariate methods. The bacterial taxa or biomarkers are then ranked based on their specificity for a particular microbiome age.

A skilled person will understand that the control data set does not need to be prepared every time a method according to the present disclosure is performed. Instead, a skilled person can analyse a sample as discussed below and compare the results with a database showing the microbial composition of canids with a known healthy microbiome for the age of the animal, or with an average composition and diversity for the life stage or age of the animal.

### Bacterial taxa

The presently disclosed subject matter has discovered that certain bacteria are particularly useful for determining the microbiome age of a canid in the methods of the present disclosure. These include bacteria of the genera *Phascolarctobacterium, Bacteroides, Clostridium* (in particular the species *Clostridium perfringens*) and *Escherichia* /*Shigella.* Particularly useful species within these genera include *Phascolarctobacterium sp., Bacteroides sp.* and *Escherichia coli* /*Shigella flexneri.* In a particular embodiment, the sequence(s) that is detected has at least about 95%, at least about 96%, at least about 97%, at least about 98%, at least about 99% or has/have 100% identity to the sequence of SEQ ID NOs: 32, 33, 34, 35 and/or 36.

The bacterial taxa discussed in the preceding paragraphs show the highest accuracy in determining a canid's microbiome biological age status, as shown in Figure 11 (Table 2.3). The presently disclosed subject matter has further shown that the accuracy increases when more taxa are detected and/or quantified. Thus, in some embodiments all of the taxa mentioned in the previous paragraphs are detected and/or quantified. For example, sequences having at least about 95%, at least about 96%, at least about 97%, at least about 98%, at least about 99% or 100% identity to the sequences of SEQ ID NOs: 33, 34, 35 and 36 are detected and/or quantitated. In addition, or alternatively, the methods of the present disclosure can analyse one or more of the bacterial taxa discussed below.

Further bacterial taxa which are useful for distinguishing between an adult, senior and geriatric microbiomes include species from genera selected from the group consisting of: *Lactobacillus, Terrisporobacter, Turicibacter, Bacteroides, Bifidobacterium, Allobaculum, Blautia, Cetobacterium, Clostridium, Dubosiella, Escherichia, Phascolarctobacterium, Streptococcus, Megamonas, Ruminococcus, Fusobacterium, Enterococcus, Lachnoclostridium, Epulopiscium, Erysipelotrichaceae, Dorea, Adlercreutzia, Anaerobiospirillum, Romboutsia, Peptostreptococcaceae, and Prevotella.* In some embodiments, particular bacterial species for use in the methods of the present disclosure include *Adlercreutzia equolifaciens, Anaerobiospirillum sp., Bacteroides sp., Blautia sp., Blautia [Ruminococcus] gnavus, Blautia [Ruminococcus] torques, Blautia obeum, Blautia producta, Cetobacterium somerae, Clostridium, Clostridium celatum, Clostridium dakarense, Clostridium hiranonsis, Clostridium perfringens, Clostridium sp., Clostridium sp.K39, Dorea sp., Dubosiella newyorkensis, Enterococcus asini, Enterococcus casseliflavus, Enterococcus haemoperoxidus, Enterococcus sulfureus, Erysipelotrichaceae sp., Escherichia coli*/*Shigella flexneri, Fusobacterium mortiferum, Lachnoclostridium sp., Lachnospiraceae sp., Lactobacillus animalis, Lactobacillus apodemi, Lactobacillus gasseri, Lactobacillus hamsteri, Lactobacillus helveticus, Lactobacillus johnsonii, Lactobacillus murinus, Lactobacillus reuteri, Lactobacillus taiwanensis, Megamonas sp., Niameybacter massiliensis, Peptostreptococcaceae TM5, Phascolarctobacterium sp., Prevotella sp., Romboutsia sp., Ruminococcus gauvreauii group sp., Streptococcus alactolyticus, Streptococcus luteciae, Terrisporobacter sp.,* and *Turicibacter sp.*

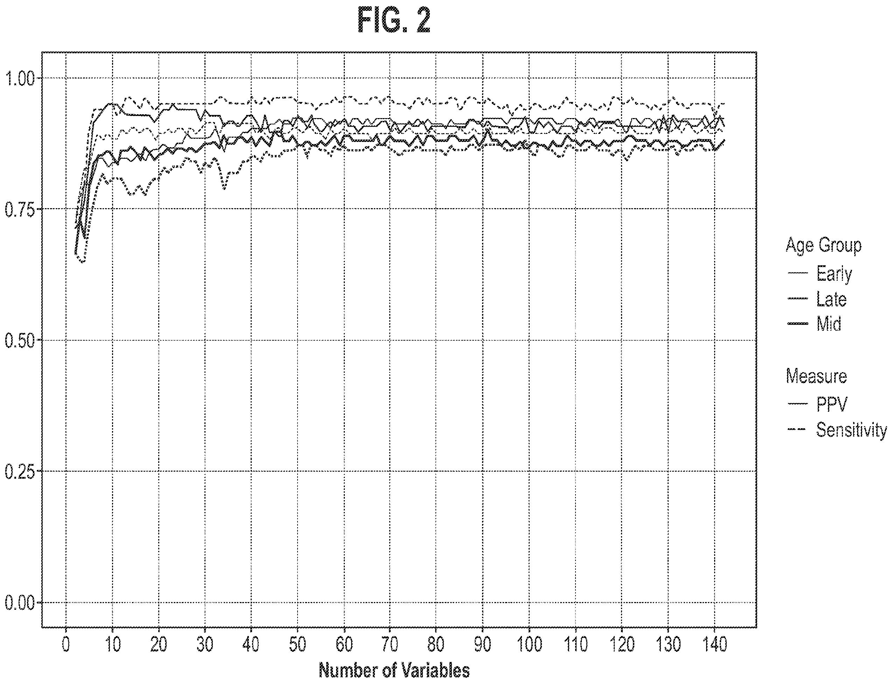

As discussed above and as shown in Figure 11 (Table 2.3), the accuracy of the methods of the present disclosure increases when more bacterial taxa are detected and/or quantified. Thus, in some embodiments, more than 5, more than 8, more than 10, more than 15, more than 20, more than 25 or, more than 30 of the bacterial taxa shown in Figure 2.3 (Table 2.3) are detected and/or quantified. The bacterial taxa can be those discussed in the previous paragraphs. For example, in some embodiments, the methods detect and/or quantitate sequences having at least about 95%, at least about 96%, at least about 97%, at least about 98%, at least about 99% or 100% identity to the sequences of SEQ ID NOs: 32-36. They can further comprise detecting and/or quantitating sequences having at least about 95%, at least about 96%, at least about 97%, at least about 98%, at least about 99% or 100% identity to the sequences of SEQ ID NOs: 37-41. The methods of the present disclosure in some embodiments can further comprise detecting and/or quantitating sequences having at least about 95%, at least about 96%, at least about 97%, at least about 98%, at least about 99% or 100% identity to the sequences of SEQ ID NOs 42-46, SEQ ID NOs 47-51, SEQ ID NOs 52-56, SEQ ID NOs 57-61, SEQ ID NOs 62-66 and/or SEQ ID NOs 67-68. In certain embodiments, the methods of the present disclosure detect and/or quantitate sequences having at least about 95%, at least about 96%, at least about 97%, at least about 98%, at least about 99% or 100% identity to the sequences of SEQ ID Nos: 32-68.

The presently disclosed subject matter has further discovered that bacteria of the genus *Blautia* [*Ruminococcus*]*,* and in particular of the species *Blautia* [*Ruminococcus*] *gnavus,* are particularly useful for determining the microbiome age of a young canid at the various stages of early life (pre-weaning, weaning, and post-weaning). Therefore, in certain embodiments, the method comprises detecting and/or quantitating a sequence having at least about 95%, at least about 96%, at least about 97%, at least about 98%, at least about 99% or having 100% identity to the sequence of SEQ ID NO: 20.

The presently disclosed subject has further discovered that bacteria of the genus *Escherichia* and/or the genus *Shigella,* are particularly useful for detection in the present disclosure. These bacteria also show specificity for the early life stages during puppyhood, as defined above, and thus allow a differentiation between the different stages of microbiome development in young dogs. Thus, in some embodiments, the method disclosed herein comprises detecting and/or quantitating a sequence having at least about 95%, at least about 96%, at least about 97%, at least about 98%, at least about 99% or having 100% identity to the sequences of SEQ ID NO: 16 and/or 17.

Further useful bacterial genera and species include *Staphylococcus* (species *Staphylococcus* sp.), *Flavonifractor* (*Flavonifractor sp.*)*, Romboutsia* (*Romboutsia sp*.), *Peptoclostridium* (*Peptoclostridium sp.*)*, Bacteroides* (*Bacteroides sp*.)*, Megamonas* (*Megamonas sp.*)*,* and *Collinsella* (*Collinsella sp.*)*.* In some instances, the method also comprises detecting and/or quantitating a sequence having at least about 95%, at least about 96%, at least about 97%, at least about 98%, at least about 99% or having 100% identity to the sequences of SEQ ID NOs: 29, and/or 13, and/or 15, and/or 22, and/or 26, and/or 10 and/or 27.

The bacterial taxa discussed in the preceding paragraphs showed the highest specificity for the various stages of puppyhood, as defined above, and are thus can be advantageous for use in the methods of the present disclosure. Thus, in some embodiments, the methods of the present disclosure comprise detecting and/or quantitating all of the species of the genera *Blautia* [*Ruminococcus*] *(Blautia* [*Ruminococcus] gnavus), Escherichia*/*Shigella* (*Escherichia spp.*/*Shigella spp.*), *Flavonifractor* (*Flavonifractor spp*.)*, Romboutsia* (*Romboutsia spp*.)*.* They can further comprise detecting and/or quantitating *Peptoclostridium* (*Peptoclostridium spp.*)*, Bacteroides* (*Bacteroides spp.*)*, Megamonas* (*Megamonas spp.*)*,* and *Collinsella* (*Collinsella spp.*)*.* These embodiments can comprise detecting and/or quantitating all of the sequences having at least about 95%, at least about 96%, at least about 97%, at least about 98%, at least about 99% or having 100% identity to the sequences of SEQ ID Nos: 20, 16, 17, 29 and 13. In further embodiments, the method further comprises detecting and/or quantitating all of sequences having at least about 95%, at least about 96%, at least about 97%, at least about 98%, at least about 99% or having 100% identity to the sequences of SEQ ID NOs: 15, 22, 26, 10 and 27.

The presently disclosed subject matter has further identified that an increase in *Megamonas sp., Blautia* and *Clostridium hiranonsis* are associated with a healthy microbiome in puppies. Furthermore, the presently disclosed subject matter has found that an increase of the taxa *Allobaculum, Peptostreptococcus* and *Bifidobacterium* are associated with the adult microbiome.

As shown in Figure 10 (Table 1.3), the presently disclosed subject matter has found that the specificity increases if more than one bacterial taxa is detected and/or quantified. Thus, in some embodiments, two or more bacterial taxa (e.g. bacterial genera and/or bacterial species) are detected and/or quantified. The specificity of the method increases as more bacterial taxa are detected and/or quantified. Thus, the methods of the present disclosure can comprise in some embodiments detection of 5 or more, 15 or more, 20 or more, or 25 or more of the bacterial taxa described in Figure 10 (Table 1.3). In some embodiments, the method comprises detecting all of the bacterial taxa, or all of the bacterial genera or bacterial species identified in Figure 10 (Table 1.3). For example, in some embodiments, the methods comprise detecting sequences having at least about 95%, at least about 96%, at least about 97%, at least about 98%, at least about 99% or having 100% identity to the sequences of SEQ ID NOs: 3-31.

Where a sequence is detected which has at least about 95%, at least about 96%, at least about 97%, at least about 98%, or at least about 99% identity to the 16S rDNA sequences identified herein, the sequence can stem from a bacterium which is either of the same species or a closely related species. For example, where a sequence has about 95% identity to the sequence of SEQ ID NO: x, the sequence will preferably stem from a bacterium in the same genus as the bacterium from which SEQ ID NO: x was obtained or preferably be from the same species. For example, in the case of SEQ ID NO: 32, the bacterium would preferably be of the genus *Clostridium* and most preferably of the species *Clostridium perfringens.*

### The canid

The methods of the present disclosure are used to determine the microbiome age of a canid, wherein the method further comprises comparing the microbiome biological age status of the canid to the chronological age of the canid. This genus comprises domestic dogs (*Canis lupus familiaris*), wolves, coyotes, foxes, jackals, dingoes and the present disclosure can be used for all these animals. In a particular embodiment, the subject is a domestic dog herein referred to simply as a dog.

Furthermore, in some embodiments, the canid is healthy. "Healthy," as used herein, refers to a canid who has not been diagnosed with a disease that is known to be related to the microbiome. Examples of such diseases include, but are not limited to, chronic and acute diarrhoea irritable bowel syndrome, ulcerative colitis, Crohn's disease and inflammatory bowel disease. In a particular embodiment, the canid does not suffer from dysbiosis.

One advantage of the methods of the present disclosure is that they allow a skilled person to determine whether a canid's microbiome biological age status corresponds to its physiological or chronological age. For example, a skilled person can determine whether an adult dog has a microbiome representative of healthy dogs in the adult lifestage and take appropriate steps if the microbiome age or lifestage and the actual age do not concur.

There are numerous different breeds of domestic dogs which show a diverse habitus. Different breeds also have different life expectancies with smaller dog breeds generally being expected to live longer than larger breeds. Accordingly, different breeds are considered to be junior, adult, senior or geriatric at different time points in their life. A summary of the different life stages is provided in Table 1.0 below.

**Table 1.0**

| | Youth | Adult | Senior | Geriatric |
|---|---|---|---|---|
| Toy | Up to 7 years | 8-11 years | 12-13 years | 14+ years |
| Small | Up to 7 years | 8-11 years | 12-13 years | 14+ years |
| Medium | Up to 5 years | 6-9 years | 10-13 years | 14+ years |
| Large | Up to 5 years | 6-9 years | 10-11 years | 12+ years |

The distinction between toy, small, medium and large breeds is known in the art. In particular, toy breeds comprise distinct breeds including but not limited to Affenpinscher, Australian Silky Terrier, Bichon Frise, Bolognese, Cavalier King Charles Spaniel, Chihuahua, Chinese Crested, Coton De Tulear, English Toy Terrier, Griffon Bruxellois, Havanese, Italian Greyhound, Japanese Chin, King Charles Spaniel, Lowchen (Little Lion Dog), Maltese, Miniature Pinscher, Papillon, Pekingese, Pomeranian, Pug, Russian Toy and Yorkshire Terrier.

Small breeds are larger on average than toy breeds with an average body weight of up to about 10 kg. Non-limiting exemplary breeds include French Bulldog, Beagle, Dachshund, Pembroke Welsh Corgi, Miniature Schnauzer, Cavalier King Charles Spaniel, Shih Tzu, and Boston Terrier.

Medium dog breeds have an average weight of about 11 to about 26 kg. These dog breeds include, but are not limited to, Bulldog, Cocker Spaniel, Shetland Sheepdog, Border Collie, Basset Hound, Siberian Husky and Dalmatian.

Large breed are those with an average body weight of at least about 27 kg. Non-limiting examples include Great Dane, Neapolitan mastiff, Scottish Deerhound, Dogue de Bordeaux, Newfoundland, English mastiff, Saint Bernard, Leonberger and Irish Wolfhound.

Cross-breeds can generally be categorised into toy, small, medium and large dogs depending on their body weight.

### The sample

The methods of the present disclosure generally use a fecal sample or a sample from the gastrointestinal lumen of the canid. Fecal samples are convenient because their collection is non-invasive and it also allows for easy repeated sampling of individuals over a period of time. However, other samples can also be used with the methods disclosed herein, including, but not limited to, ileal, jejunal, duodenal samples and colonic samples.

In some embodiments, the sample is a fresh sample. In further embodiments, the sample is frozen or is stabilised by other means, such as addition to preservation buffers, or by dehydration using methods such as freeze drying, before use in the methods of the present disclosure.

Before use in the methods of the present disclosure, in some embodiments, the sample is processed to extract DNA. Methods for isolating DNA are well known in the art, as reviewed in reference [8], for example. Suitable methods include, for instance, the QIAamp Power Faecal DNA kit (Qiagen).

### Changing the microbiome

Also disclosed herein, but not part of the claimed invention, the methods of the present disclosure can comprise a further step of changing the composition of the microbiome. The composition of the microbiome can be changed by administering to the canid a dietary change, a functional food, a supplement, a nutraceutical, or a pharmaceutical composition that is capable of changing the composition of the microbiome.
Also disclosed herein is a dietary change, a functional food, a supplement, a nutraceutical, or a pharmaceutical composition for use in a method of changing the composition of a microbiome of a canid, wherein the method first comprises determining the microbiome age status of a canid, comprising (a) detecting one or more bacterial taxa in a sample obtained from the canid; (b) correlating the one or more bacterial taxa in the sample to a control data set; and (c) determining the microbiome age, wherein the method further comprises comparing the microbiome biological age status of the canid to the chronological age of the canid, before the step of changing the composition of the microbiome by administering to the canid a dietary change, a functional food, a supplement, a nutraceutical or a pharmaceutical composition.
Such functional foods, nutraceuticals, live biotherapeutic products (LBPs) and pharmaceutical compositions are well known in the art and comprise bacteria [9]. They can comprise single bacterial species selected from the group consisting of *Bifidobacterium* sp., such as *B. animalis (e.g., B. animalis subsp. animalis or B. animalis subsp. lactis), B. bifidum, B. breve, B. longum (e.g., B. longum subsp. infantis or B. longum subsp. longum), B. pseudolongum, B.adolescentis, B. catenulatum,or B. pseudocatanulatum;* single bacterial species of *Lactobacillus,* such as *L. acidophilus, L. antri, L. brevis, L. casei, L. coleohominis, L. crispatus, L. curvatus, L. fermentum, L. gasseri, L. johnsonii, L. mucosae, L. pentosus, L. plantarum, L. reuteri, L. rhamnosus, L. sakei, L. salivarius, L. paracasei, L. kisonensis., L. paralimentarius, L. perolens, L. apis, L. ghanensis, L. dextrinicus, L. shenzenensis, L. harbinensis;* or single bacterial species of *Pediococcus,* such as *P. parvulus, P. lolii, P. acidilactici, P. argentinicus, P. claussenii, P. pentosaceus,* or *P. stilesii* or similarly species of *Enterococcus* such as *E. faecium* or *Bacillus* species such as *Bacillus subtilis, B. coagulans, B. indicus, or B. clausii.* Additionally, combinations of these and other bacterial species can be used. The amount of the dietary change, the functional food, the supplement, the nutraceutical composition, or the pharmaceutical composition that is administered to the canid can be an amount that is effective to effect a change in the composition of the microbiome.

The further step of changing the composition of the microbiome can be performed in instances where a canid's microbiome age does not positively concur with its actual age. For example, the methods of the present disclosure can reveal that an adult dog has a microbiome composition and diversity representative of a senior or geriatric dog. As discussed above, characteristics associated with the adult microbiome are considered the healthiest microbiome characteristics and so in these circumstances it would be highly desirable in the older dog to make a dietary change and/or to administer a functional food, nutraceutical, LBP or pharmaceutical composition to shift the microbiome back to an adult microbiome composition/status.

Similarly, in other embodiments, it can be desirable to shift the microbiome so that the microbiome biological age status does not concur with the canid's actual age. For example, an older dog in the senior or geriatric life stage suffering from recurrent diarrhea can benefit from receiving a diet change, functional food, nutraceutical, LBP or pharmaceutical composition to shift the microbiome to one representative of an adult dog.

The methods of the present disclosure can also be used to assess the success of a treatment as described above. To this end, a canid can receive a change in diet, functional food, supplement, LBP, nutraceutical or pharmaceutical composition or an exercise/physical activity regimen, which is capable of changing the composition of the microbiome. Following administration of the treatment through nutrition or exercise (for example after about 1 day, 2 days, 5 days, 1 week, 2 weeks, 3 weeks, 1 month, 3 months, 6 months, etc.), the microbiome age can be assessed using any of the methods of the present disclosure. In a particular embodiment, the microbiome biological age status is determined before and after administration of the dietary change, functional food, supplement, LBP, nutraceutical or pharmaceutical composition or exercise/physical activity regimen.

### Monitoring

The present disclosure provides a method of determining the microbiome age status of a canid, comprising (a) detecting one or more bacterial taxa in a sample obtained from the canid; (b) correlating the one or more bacterial taxa in the sample to a control data set; and (c) determining the microbiome age, wherein the method further comprises comparing the microbiome biological age status of the canid to the chronological age of the canid. In some embodiments, the methods of the present disclosure are performed once to determine a canid's microbiome status and compare to chronological age. The methods of the present disclosure can also be performed more than once, for example two times, three times, four times, five times, six times, seven times or more than seven times. This allows the microbiome age to be monitored over time. This can be useful for example where a canid is receiving treatment to shift the microbiome or is at an age representing a transition between life stages to track the need for an intervention (e.g., a treatment) in the future. The first time the method is performed the microbiome age is determined and, following an intervention (e.g., a treatment) such as a dietary change or administration of a supplement, functional food nutraceutical or pharmaceutical composition or exercise/physical activity regimen, the method is repeated to assess the influence of the composition or intervention (e.g., treatment) on the microbiome. The microbiome biological age status can also be determined for the first time after the canid has received treatment and the method repeated afterwards to assess whether there is a change in the microbiome biological age status.

The methods described herein can be repeated over several days, one week, two weeks, three weeks, one month, two months, three months, four months, five months, six months, 12 months, 18 months, 24 months, 30 months, 36 months, or more than 36 months apart.

### Treatment

In some embodiments, the methods of the present disclosure can also relate to a dietary change, a functional food, a supplement, a nutraceutical, or a pharmaceutical composition for use in methods for treating a canid. The canid can have a microbiome age that does not concur with its actual age, *e.g.,* an adult dog having a microbiome composition and diversity of a senior or geriatric dog. In other embodiments, the methods of the present disclosure can relate to a dietary change, a functional food, a supplement, a nutraceutical, or a pharmaceutical composition for use in methods for treating a canid to shift the microbiome age of the canid to be lower than the canid's actual age. The dietary change, functional food, supplement, nutraceutical, or pharmaceutical composition disclosed herein for use in methods of treatment include (i) identifying the canid as requiring treatment by determining the microbiome age according to any of the methods disclosed herein, and (ii) administering to the canid a dietary change, a functional food, a supplement, a nutraceutical, or a pharmaceutical composition as disclosed herein that is capable of changing the composition of the microbiome. The amount of the dietary change, the functional food, the supplement, the nutraceutical composition, or the pharmaceutical composition that is administered to the canid can be an amount that is effective to effect a change in the composition of the microbiome, or to improve any symptoms relating to the canid having an unhealthy microbiome status. Optionally, in some embodiments, the method further includes determining the microbiome age following the administration of the dietary change, the functional food, the supplement, the nutraceutical, or the pharmaceutical composition to evaluate the effectiveness of the treatment.

### General

The terms used in this specification generally have their ordinary meanings in the art, within the context of this invention and in the specific context where each term is used. Certain terms are discussed below, or elsewhere in the specification, to provide additional guidance to the practitioner in describing the methods and compositions of the invention and how to make and use them.

The practice of the present disclosure will employ, unless otherwise indicated, conventional methods of chemistry, biochemistry, molecular biology, immunology and pharmacology, within the skill of the art. Such techniques are explained fully in the literature. See, *e.g.,* references [10-17], *etc.*

References to a percentage sequence identity between two nucleotide sequences means that, when aligned, that percentage of nucleotides are the same in comparing the two sequences. This alignment and the percent homology or sequence identity can be determined using software programs known in the art, for example those described in section 7.7.18 of ref. [18]. A preferred alignment is determined using the BLAST (basic local alignment search tool) algorithm or the Smith-Waterman homology search algorithm using an affine gap search with a gap open penalty of 12 and a gap extension penalty of 2, BLOSUM matrix of 62. The Smith-Waterman homology search algorithm is disclosed in ref. [19]. The alignment can be over the entire reference sequence, *i.e.* it can be over 100% length of the sequences disclosed herein.

### Definitions

As used herein, the use of the word "a" or "an" when used in conjunction with the term "comprising" in the claims and/or the specification can mean "one," but it is also consistent with the meaning of "one or more," "at least one," and "one or more than one." Still further, the terms "having," "containing," and "comprising" are interchangeable, and one of skill in the art is cognizant that these terms are open ended terms. Further, the term "comprising" encompasses "including" as well as "consisting," e.g., a composition "comprising" X can consist exclusively of X or can include something additional, e.g., X + Y.

The term "about" or "approximately" means within an acceptable error range for the particular value as determined by one of ordinary skill in the art, which will depend in part on how the value is measured or determined, *i.e.,* the limitations of the measurement system. For example, "about" can mean within 3 or more than 3 standard deviations, per the practice in the art. Alternatively, "about" can mean a range of up to 20%, or alternatively up to 10%, or alternatively up to 5%, and alternatively still up to 1% of a given value. Alternatively, particularly with respect to biological systems or processes, the term can mean within an order of magnitude, preferably within 5-fold, and more preferably within 2-fold, of a value. In certain embodiments, the term "about" in relation to a numerical value x is optional and means, for example, *x*±10%.

The term "effective treatment" or "effective amount" of a substance means the treatment or the amount of a substance that is sufficient to effect beneficial or desired results, including clinical results, and, as such, an "effective treatment" or an "effective amount" depends upon the context in which it is being applied. In the context of administering a composition (e.g., a dietary change, a functional food, a supplement, a nutraceutical composition, or a pharmaceutical composition) to change the composition of a microbiome in a feline having an unhealthy microbiome, the effective amount is an amount sufficient to bring the health status of the microbiome back to a healthy state, which is determined according to one of the methods disclosed herein. In certain embodiments, an effective treatment, as described herein, can also include administering a treatment in an amount sufficient to decrease any symptoms associated with an unhealthy microbiome. The decrease can be an about 0.01%, about 0.1%, about 1%, about 5%, about 10%, about 20%, about 30%, about 40%, about 50%, about 60%, about 70%, about 80%, about 90%, about 95%, about 98% or about 99% decrease in severity of symptoms of an unhealthy microbiome. An effective amount can be administered in one or more administrations. A likelihood of an effective treatment described herein is a probability of a treatment being effective, *i.e.,* sufficient to alter the microbiome, or treat or ameliorate a digestive disorder and/or inflammation, as well as decrease the symptoms.

As used herein, and as well-understood in the art, "treatment" is an approach for obtaining beneficial or desired results, including clinical results. For purposes of this subject matter, beneficial or desired clinical results include, but are not limited to, alleviation or amelioration of one or more symptoms, diminishment of extent of a disorder, stabilized (*i.e.,* not worsening) state of a disorder, prevention of a disorder, delay or slowing of the progression of a disorder, and/or amelioration or palliation of a state of a disorder. In certain embodiments, the decrease can be an about 0.01%, about 0.1%, about 1%, about 5%, about 10%, about 20%, about 30%, about 40%, about 50%, about 60%, about 70%, about 80%, about 90%, about 95%, about 98% or about 99% decrease in severity of complications or symptoms. "Treatment" can also mean prolonging survival as compared to expected survival if not receiving treatment.

The word "substantially" does not exclude "completely" e.g. a composition which is "substantially free" from Y can be completely free from Y. Where necessary, the word "substantially" can be omitted from the definition of the present disclosure.

Unless specifically stated, a process or method comprising numerous steps can comprise additional steps at the beginning or end of the method, or can comprise additional intervening steps. Also, steps can be combined, omitted or performed in an alternative order, if appropriate.

Various embodiments of the methods of the present disclosure are described herein. It will be appreciated that the features specified in each embodiment can be combined with other specified features, to provide further embodiments. In particular, embodiments highlighted herein as being suitable, typical or preferred can be combined with each other (except when they are mutually exclusive).

### EXAMPLES

The presently disclosed subject matter will be better understood by reference to the following Example, which is provided as exemplary of the invention, and not by way of limitation.

### Example 1: A method of detecting the biological age of the faecal microbiome or the microbiota of young life stages in dogs (puppies)

Data on the faecal microbiota was derived by analysis of the microbiota in freshly produced faecal samples from 39 puppies with samples taken at 12 time points. These time points were grouped into, early postpartum puppyhood (2, 4, 6, 8, 10, 12 days); mid puppyhood (during-weaning) 17, 24 and 31 days and later (rapid growth phase of) puppyhood (post-weaning) late 38, 45 and 52 days.

### Method

Following Illumina sequencing of the V4-V6 region using primer sequences (319F: CAAGCAGAAGACGGCATACGAGATGTGACTGGAGTTCAGACGTGTGCTCTTCCGATCT (SEQ ID NO: 1) and 806R: AATGATACGGCGACCACCGAGATCTACACTCTTTCCCTACAC GACGCTCTTCCGATCT (SEQ ID NO: 2) the resulting DNA sequences were clustered at 98% identity and abundant taxa (representing >0.001 of the total sequences) were then assessed for their relative proportions. Importance scores were calculated using 2000 random forests each with 2000 trees using the default number of variables per tree (square root of the number of variables) and permutation importance score calculation. From these 1000 random forests the average ranks of each OTU were calculated, with 1 being the most important, alongside the standard deviation, and maximum and minimum rank.

Positive predictive value and specificity were also calculated by fitting random forests to data with successively more OTUs (taken in importance order). Analyses were performed using R version 3.5.1 and the ranger library.

The method involves the extraction of DNA from a freshly produced faecal sample by a means such as the QIAamp Power Faecal DNA kit (Qiagen) and subsequently the use of molecular biology techniques to assess the detection rate and abundance of the combination of the bacterial taxa described below (Tables 1.1 and 2.1). The combination and abundance of the organisms allow the detection of the biological age of the microbiome and assessment of whether the microbiome components observed in faeces of the animal is descriptive of an immature or older than expected microbiome relative to the actual age of the animal. After DNA extraction and sequencing of the DNA by techniques such as 16S rDNA amplicon, shotgun, metagenome, Illumina, nanopore or other DNA sequencing techniques is conducted on the faecal DNA and the assessment of relative abundance of the sequences descriptive of the organisms in Table 1.1 or DNA sequences within 95% identical to those in Table 1.2 is made. Briefly, sequence data obtained from the test sample is clustered into groups of sequences with 98% - 100% identity and a reference sequence from the clusters which represent >0.001% of the total sequences is then used to either 1) assign taxonomy, gene function through database homologues or to determine the nature of the biomarker through homology searches of DNA databases such as the Greengenes or Silva or the NCBI non-redundant nucleotide sequence database for comparison to known DNA sequences for species held within the databases or 2) compared to the DNA sequences given in Table 1.2 below.

The number and abundance of the organisms, sequences or biomarkers identified from within the combinations described in Table 1.2 are then used to describe the biological age of the sample in terms of the group with which they best fit.

### Results

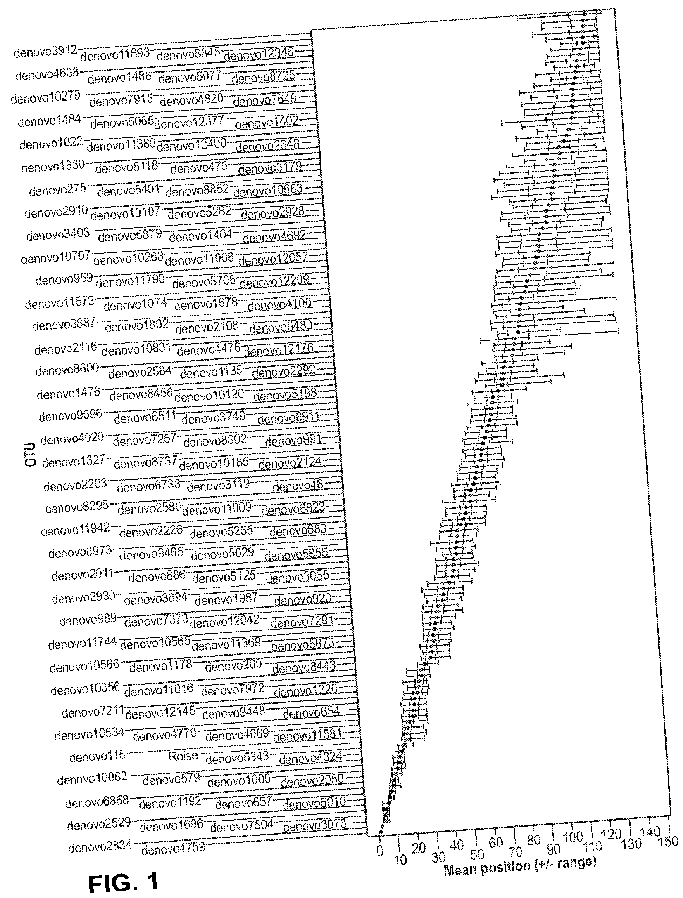

Importance scores were used to rank the bacterial taxa (OTUs) for their consistency in assigning samples to the correct study group defined as early life, through weaning or later (rapid growth phase of) puppyhood (Table 1.1 and Figure 1). The sensitivity and positive predicted values were also calculated for successively increasing numbers of OTUs used in the model in the importance order derived from the data analysis (Figure 2). A plateau in the sensitivity and positive predictive was reached at approximately 28 OTUs. Consequently the first 28 OTUs were determined to be most optimal for defining the biological age of the microbiota during puppyhood (Table 1.1).

**Table 1.1: Bacterial taxa (OTUs) ranked by importance scores descriptive of their consistency in assignment of samples to the appropriate study group (early life, through weaning or later (rapid growth phase of) puppyhood). P=position**

| **P** | **OTU** | **Taxonomy** |
|---|---|---|
| 1 | Pu_denovo4759 | Bacteria;Firmicutes;Clostridia;Clostridiales;Lachnospiraceae;Blauti *a* [Ruminococcus]_gnavus_group;uncultured_bacterium |
| 2 | Pu_denovo2834 | Bacteria;Proteobacteria; Gammaproteobacteria;Enterobacteriales;Ent erobacteriaceae;Escherichia-Shigella;uncultured bacterium |
| 3 | Pu_denovo3073 | Bacteria;Proteobacteria; Gammaproteobacteria;Enterobacteriales;Ent erobacteriaceae;Escherichia-Shigella;uncultured_bacterium |
| 4 | Pu_denovo7504 | Bacteria;Firmicutes;Bacilli;Bacillales; Staphylococcaceae; Staphyloc occus;uncultured_bacterium |
| 5 | Pu_denovo1696 | Bacteria;Firmicutes;Clostridia;Clostridiales;Ruminococcaceae;Flavo nifractor;uncultured_bacterium |
| 6 | Pu_denovo2529 | Bacteria;Firmicutes;Clostridia;Clostridiales;Peptostreptococcaceae; Romboutsia;uncultured_bacterium |
| 7 | Pu_denovo5010 | Bacteria;Firmicutes;Clostridia;Clostridiales;Peptostreptococcaceae; Peptoclostridium;uncultured _bacterium |
| 8 | Pu_denovo657 | Bacteria;Bacteroidetes;Bacteroidia;Bacteroidales;Bacteroidaceae;Ba cteroides;uncultured _bacterium |
| 9 | Pu_denovo1192 | Bacteria;Firmicutes;Negativicutes;Selenomonadales;Veillonellaceae ;Megamonas;uncultured_bacterium |
| 10 | Pu_denovo6858 | Bacteria;Actinobacteria;Coriobacteriia;Coriobacteriales;Coriobacter iaceae;Collinsella;uncultured _bacterium |
| 11 | Pu_denovo2050 | Bacteria;Firmicutes;Clostridia;Clostridiales;Clostridiaceae_1;Clostri dium_sensu_stricto_1;uncultured_bacterium |
| 12 | Pu_denovo1000 | Bacteria;Firmicutes;Negativicutes;Selenomonadales;Veillonellaceae ;Megamonas;uncultured_bacterium |
| 13 | Pu_denovo579 | Bacteria;Firmicutes;Clostridia;Clostridiales;Peptostreptococcaceae; Paeniclostridium;uncultured_Eubacterium_sp. |
| 14 | Pu_denovo10082 | Bacteria;Firmicutes;Negativicutes;Selenomonadales;Veillonellaceae ;Megamonas;uncultured_bacterium |
| 15 | Pu_denovo4324 | Bacteria;Firmicutes;Clostridia;Clostridiales;Lachnospiraceae;Tyzzer ella_4;uncultured_bacterium |
| 16 | Pu_denovo5343 | Bacteria;Firmicutes;Negativicutes;Selenomonadales;Veillonellaceae ;Megamonas;uncultured_bacterium |
| 17 | Pu_denovo115 | Bacteria;Firmicutes;Clostridia;Clostridiales;Peptostreptococcaceae; Romboutsia;uncultured _bacterium |
| 18 | Pu_denovo11581 | Bacteria;Firmicutes;Clostridia;Clostridiales;Ruminococcaceae;uncul tured;uncultured _bacterium |
| 19 | Pu_denovo4069 | Bacteria;Firmicutes;Clostridia;Clostridiales;Clostridiaceae_1;Clostri dium_sensu_stricto_1;uncultured_bacterium |
| 20 | Pu_denovo4770 | Bacteria;Firmicutes;Clostridia;Clostridiales;Lachnospiraceae;Blauti a;uncultured _bacterium |
| 21 | Pu_denovo10534 | Bacteria;Firmicutes;Bacilli;Lactobacillales;Lactobacillaceae;Lactob acillus;uncultured _bacterium |
| 22 | Pu_denovo654 | Bacteria;Firmicutes;Clostridia;Clostridiales;Lachnospiraceae;Lachn oclostridium;uncultured _bacterium |
| 23 | Pu_denovo9448 | Bacteria;Firmicutes;Bacilli;Lactobacillales;Lactobacillaceae;Pedioc occus;uncultured_organism |
| 24 | Pu_denovo12145 | Bacteria;Firmicutes;Clostridia;Clostridiales;Lachnospiraceae;Blauti a;uncultured _bacterium |
| 25 | Pu_denovo7211 | Bacteria;Proteobacteria; Gammaproteobacteria;Enterobacteriales;Ent erobacteriaceae;Klebsiella; Shigella_dysenteriae |
| 26 | Pu_denovo1220 | Bacteria;Firmicutes;Clostridia;Clostridiales;Peptostreptococcaceae; Romboutsia;uncultured _bacterium |
| 27 | Pu_denovo7972 | Bacteria;Actinobacteria;Actinobacteria;Bifidobacteriales;Bifidobact eriaceae;Bifidobacterium;uncultured_actinobacterium |
| 28 | Pu_denovol1016 | Bacteria;Firmicutes;Clostridia;Clostridiales;Lachnospiraceae;Lachn ospiraceae_NK4A136_group;uncultured_bacterium |
| 29 | Pu_denovo10356 | Bacteria;Firmicutes;Bacilli;Lactobacillales;Enterococcaceae;Enteroc occus;uncultured_bacterium |

The number and abundance of the organisms, sequences or biomarkers identified from within the combinations described in Table 1.2 are then used to describe the biological age of the sample in terms of the group with which they best fit.

**Table 1.2: DNA sequences ranked by importance scores descriptive of their consistency in assignment of samples to the appropriate study group (early life, through weaning or later (rapid growth phase of) puppyhood.**

| SEQ ID NO: | OTU ID | Reference 16S rDNA Sequence |
|---|---|---|
| 3 | Pu_denov o4759 | CCTGTTTGCTCCCCACGCTTTCGAGCCTCAACGTCAGTCATCGTCCAG AAAGCCGCCTTCGCCACTGGTGTTCCTCCTAATATCTACGCATTTCAC CGCTACACTAGGAATTCCGCTTTCCTCTCCGACACTCTAGCCTGACAG TTCCAAATGCAGTCCCGGGGTTGAGCCCCGGGCTTTCACATCTGGCTT GCCATGCCGTCTACGCTCCCTTTACACCCAGTAAATCCGGATAACGCT TGCCCCCTACGTATTACCGCGGCTGCTGGCACGTAGTTAGCCGGGGC TTCTTAGTCAGGTACCGTCATTTCTTCCCTGCTGATAGAGCTTTACAT ACCGAAATACTTCATCGCTCACGCGGCGTCGCTGCATCAGGGTTTCCC CCATTGTGCAATATTCCCCA |
| 4 | Pu_denov o2834 | CCTGTTTGCTCCCCACGCTTTCGCACCTGAGCGTCAGTCTTCGTCCAG GGGGCCGCCTTCGCCACCGGTATTCCTCCAGATCTCTACGCATTTCAC CGCTACACCTGGAATTCTACCCCCCTCTACGAGACTCAAGCTTGCCAG TATCAGATGCAGTTCCCAGGTTGAGCCCGGGGATTTCACATCTGACTT AACAAACCGCCTGCGTGCGCTTTACGCCCAGTAATTCCGATTAACGC TTGCACCCTCCGTATTACCGCGGCTGCTGGCACGGAGTTAGCCGGTG CTTCTTCTGCGGGTAACGTCAATGAGCAAAGGTATTAACTTTACTCCC TTCCTCCCCGCTGAAAGTACTTTACAACCCGAAGGCCTTCTTCATACA CGCGGCATGGCTGCATCAGGCTTGCGCCCATTGTGCAATATTCCCCA |
| 5 | Pu_denov o3073 | CCTGTTTGCTCCCCACGCTTTCGCACCTGAGCGTCAGTCTTCGTCCAG GGGGCCGCCTTCGCCACCGGTATTCCTCCAGATCTCTACGCATTTCAC CGCTACACCTGGAATTCTACCCCCCTCTACGAGACTCAAGCTTGCCAG TATCAGATGCAGTTCCCAGGTTGAGCCCGGGGATTTCACATCTGACTT AACAAACCGCCTGCGTGCGCTTTACGCCCAGTAATTCCGATTAACGC TTGCACCCTCCGTATTACCGCGGCTGCTGGCACGGAGTTAGCCGGTG CTTCTTCTGCGGGTAACGTCAATGAGCAAAGGTATTAACTTTACTCCC |
| | | TTCCTCCCCGCTGAAAGTACTTTACAACCCGAAGGCCGTCATCACTCA CGCGGCGTTGCTGCATCAGGGTTTCCCCCATTGTGCAATATTCCCCA |
| 6 | Pu_denov o7504 | CCTGTTTGATCCCCACGCTTTCGCACATCAGCGTCAGTTGCAGACCAG AAAGCCGCCTTCGCCACTGGTGTTCCTCCATATCTCTGCGCATTTCAC CGCTACACATGGAATTCCACTTTCCTCTTCTGCACTCAAGTTTTCCAG TTTCCAATGACCCTCCACGGTTGAGCCGTGGGCTTTCACATCAGACTT AAAAAACCGCCTACGCGCGCTTTACGCCCAATAATTCCGGATAACGC TTGCCACCTACGTATTACCGCGGCTGCTGGCACGTAGTTAGCCGTGGC TTTCTGGTTAGGTACCGTCAAGATGTGCACAGTTACTTACACATTTGT TCTTCCCTAACAACAGAGCTTTACGATCCGAAGACCTTCATCACTCAC GCGGCGTTGCTCCGTCAGGCTTTCGCCCATTGCGGAAGATTCCCTA |
| 7 | Pu_denov o1696 | CCTGTTTGCTCCCCACGCTTTCGCGCCTCAGCGTCAGTTACTGTCCAG CAATCCGCCTTCGCCACTGGTGTTCCTCCGTATATCTACGCATTTCAC CGCTACACACGGAATTCCGATTGCCTCTCCAGCACTCAAGAACTACA GTTTCAAATGCAGGCTGGAGGTTGAGCCCCCAGTTTTCACATCTGACT TGCAATCCCGCCTACACGCCCTTTACACCCAGTAAATCCGGATAACG CTTGCCACCTACGTATTACCGCGGCTGCTGGCACGTAGTTAGCCGTGG CTTATTCGTCAGGTACCGTCATTTGTTTCGTCCCCGACAAAAGAAGTT TACAACCCGAAAGCCTTCTTCCTTCACGCGGCGTTGCTGGGTCAGGCT TGCGCCCATTGCCCAATATTCCCCA |
| 8 | Pu_denov o2529 | CCTGTTTGCTCCCCACGCTTTCGAGCCTCAACGTCAGTTACCGTCCAG TAAGCCGCCTTCGCCACTGGTGTTCCTCCTAATATCTACGCATTTCAC CGCTACACTAGGAATTCCACTCTCCTCTCCTGCACTCAAGTCTTACAG TTTCAAAAGCTTACTACGGTTGAGCCGTAGCCTTTCACTTCTGACTTG AAAGACCGCCTACGCACCCTTTACGCCCAGTAATTCCGGATAACGCT AGCCCCCTACGTATTACCGCGGCTGCTGGCACGTAGTTAGCCGGGGC TTCCTCCTCAAGTACCGTCATTATCTTCCTTGAGGACAGAGCTTTACG ACCCGAAGGCCTTCATCGCTCACGCGGCGTTGCTGCATCAGGCTTTCG CCCATTGTGCAATATTCCCCA |
| 9 | Pu_denov o5010 | CCTGTTTGCTCCCCACGCTTTCGTGCCTCAGCGTCAGTTACAGTCCAG AGAGCCGCCTTCGCAACTGGTGTTCCTCCTAATATCTACGCATTTCAC CGCTACACTAGGAATTCCACTCTCCTCTCCTGCACTCAAGTCTCCCAG TTTCAAGAGCTTACTACGGTTGAGCCGTAGCCTTTCACTCCTGACTTG AAAGACCGCCTACGCACCCTTTACGCCCAGTAAATCCGGATAACGCT AGCCCCCTACGTATTACCGCGGCTGCTGGCACGTAGTTAGCCGGGGC |
| | | TTCCTCCTCAAGTACCGTCATTATCTTCCTTGAGGACAGAGTTTTACG ACCCGAAGGCCTTCATCACTCACGCGGCGTTGCTGCATCAGGCTTTCG CCCATTGTGCAATATTCCCCA |
| 10 | Pu_denov o657 | CCTGTTTGATACCCACACTTTCGAGCATCAGTGTCAGTTGCAGTCCAG TGAGCTGCCTTCGCAATCGGAGTTCTTCGTGATATCTAAGCATTTCAC CGCTACACCACGAATTCCGCCCACCTCTACTGTACTCAAGACTGACA GTATCAACTGCAATTTTACGGTTGAGCCGCAAACTTTCACAACTGACT TACCAGTCCACCTACGCTCCCTTTAAACCCAATAAATCCGGATAACG CTCGGATCCTCCGTATTACCGCGGCTGCTGGCACGGAGTTAGCCGAT CCTTATTCATATAATACATACAAAACAGTATACATACTGCACTTTATT CTTATATAAAAGAAGTTTACGACCCATAGAGCCTTCATCCTTCACGCT ACTTGGCTGGTTCAGGCTAACGCCCATTGACCAATATTCCTCA |
| 11 | Pu_denov o1192 | CCTGTTTGCTCCCCACGCTTTCGAGCCTCAACGTCAGTTACCGTCCAG TAAGCCGCCTTCGCCACTGGTGTTCCTCCTAATATCTACGCATTTCAC CGCTACACTGGGAATTCCGCTTTCCTCTCCTGCACTCAAGAAAACCAG TTCGGACCCCATCACGGGGTTGAGCCCCGCACTTTTAAGATCCGCTTA GTTTCCCGCCTGCGCTCCCTTTACGCCCAATAATTCCGGACAACGCTT GCCGCCTACGTATTACCGCGGCTGCTGGCACGTAGTTAGCCGTGGCTT CCTCGTTAGGTACCGTCAAATAAAAACCTTATTATGATTCTTACCCTT CGTCCCTAACAACAGAACTTTACGATCCTAAGACCTTCATCGTTCACG CGGCGTTGCTCCGTCAGGCTTTCGCCCATTGCGGAAGATTCCCCA |
| 12 | Pu_denov o6858 | CCTGTTCGCTCCCCCAGCTTTCGCGCCTCAGCGTCGGTCTCGGCCCAG AGGGCCGCCTTCGCCACCGGTGTTCCACCCGATATCTGCGCATTCCAC CGCTACACCGGGTGTTCCACCCTCCCCTACCGGACCCGAGCCCGGCG GTTCAGGGGGCGGACGGGGTTGAGCCCCGCCATTTGACCCCCTGCC TGCCGGGCCGCCTACGCGCTTTACGCCCAATGAATCCGGATAACG CTCGCCCCCTACGTATTACCGCGGCTGCTGGCACGTAGTTAGCCGGG GCTTCTTCTGCAGGTACCGTCTTGTCTCATCCCTGCTGAAAGCGGTTT ACGACCCGAGGGCCTTCGTCCCGCACGCGGCGTCGCTGCGTCAGGGT TCCCCCCATTGCGCAAGATTCCCCA |
| 13 | Pu_denov o2050 | CCTGTTTGCTCCCCACGCTTTCGAGCCTCAGCGTCAGTTACAGTCCAG AGAGTCGCCTTCGCCACTGGTGTTCTTCCTAATCTCTACGCATTTCAC CGCTACACTAGGAATTCCACTCTCCTCTCCTGCACTCTAGATAATCAG TTTGGAATGCAGCCCCCAGGTTGAGCCTGAGTATTTCACATCCCACTT AATTATCCGCCTACGCTCCCTTTACGCCCAATAATTCCGGATAACGCT |
| | | CGCCACCTACGTATTACCGCGGCTGCTGGCACGTAGTTAGCCGTGGC TTCCTCCTTGGGTACCGTCATTATCGTCCCCAAAGACAGAGCTTTACA ATCCGAAGACCGTCATCACTCACGCGGCGTTGCTGCATCAGGGTTTC CCCCATTGTGCAATATTCCCCA |
| 14 | Pu_denov o1000 | CCCGTTCGCTACCCTAGCTTTCGAGCCTCAGCGTCAGTTACAGTCCAG AAAGCCGCCTTCGCCACTGGTGTTCTTCCCAATATCTACGCATTTCAC CGCTACACTGGGAATTCCGCTTTCCTCTCCTGCACTCAAGAAAACCAG TTCGGACCCCATCACGGGGTTGAGCCCCGCACTTTTAAGATCCGCTTA GTTTCCCGCCTGCGCTCCCTTTACGCCCAATAATTCCGGACAACGCTT GCCGCCTACGTATTACCGCGGCTGCTGGCACGTAGTTAGCCGTGGCTT CCTCGTTAGGTACCGTCAAATAAAAACCTTATTATGATTCTTACCCTT CGTCCCTAACAACAGAACTTTACGATCCTAAGACCTTCATCGTTCACG CGGCGTTGCTCCGTCAGGCTTTCGCCCATTGCGGAAGATTCCCCA |
| 15 | Pu_denov o579 | CCTGTTTGCTCCCCACGCTTTCGTGCCTCAGCGTCAGTTACAGTCCAG AGAGCCGCCTTCGCTACTGGTGTTCCTCCTAATATCTACGCATTTCAC CGCTACACTAGGAATTCCACTCTCCTCTCCTGCACTCAAGTCCTACAG TTCCAAAAGCTTACTACGGTTGAGCCGTAGCCTTTCACTTCTGGCTTG AAAGACCGCCTACGCACCCTTTACGCCCAGTAATTCCGGATAACGCT AGCCCCCTACGTATTACCGCGGCTGCTGGCACGTAGTTAGCCGGGGC TTCCTCCTCAAGTACCGTCATTATCTTCCTTGAGGACAGAGCTTTACG ACCCGAAGGCCTTCATCGCTCACGCGGCGTTGCTGCATCAGGCTTTCG CCCATTGTGCAATATTCCCCA |
| 16 | Pu_denov o10082 | CCTGTTTGCTCCCCACGCTTTCGAGCCTCAGCGTCAGTTACAGTCCAG AGAGTCGCCTTCGCCACTGGTGTTCTTCCTAATCTCTACGCATTTCAC CGCTACACTAGGAATTCCACTCTCCTCTCCTGCACTCAAGAAAACCA GTTCGGACCCCATCACGGGGTTGAGCCCCGCACTTTTAAGATCCGCTT AGTTTCCCGCCTGCGCTCCCTTTACGCCCAATAATTCCGGACAACGCT TGCCGCCTACGTATTACCGCGGCTGCTGGCACGTAGTTAGCCGTGGCT TCCTCGTTAGGTACCGTCAAATAAAAACCTTATTATGATTCTTACCCT TCGTCCCTAACAACAGAACTTTACGATCCTAAGACCTTCATCGTTCAC GCGGCGTTGCTCCGTCAGGCTTTCGCCCATTGCGGAAGATTCCCCA |
| 17 | Pu_denov o4324 | CCTGTTTGCTCCCCACGCTTTCGAGCCTCAACGTCAGTGATCGTCCAG TAAGCCGCCTTCGCCACTGGTGTTCCTCCTAATATCTACGCATTTCAC CGCTACACTAGGAATTCCGCTTACCTCTCCGACACTCTAGTTACATAG TTTCCAATGCAGTCCCGGGGTTGAGCCCCGGGCTTTCACATCAGACTT |
| | | ACACAACCGTCTACGCTCCCTTTACACCCAGTAAATCCGGATAACGC TTGCACCATACGTATTACCGCGGCTGCTGGCACGTATTTAGCCGGTGC TTCTTAGTCAGGTACCGTCCTATTTCTTCCCTGCTGATAGAGCTTTAC ATACCGAAATACTTCTTCGCTCACGCGGCGTCGCTGCATCAGGGTTTC CCCCATTGTGCAATATTCCCCA |
| 18 | Pu_denov o5343 | CCTGTTTGCTCCCCACGCTTTCGAGCCTCAGCGTCAGTTACAGACCAG AGAGCCGCTTTCGCCACCGGTGTTCCTCCATATATCTACGCATTTCAC CGCTACACTGGGAATTCCGCTTTCCTCTCCTGCACTCAAGAAAACCAG TTCGGACCCCATCACGGGGTTGAGCCCCGCACTTTTAAGATCCGCTTA GTTTCCCGCCTGCGCTCCCTTTACGCCCAATAATTCCGGACAACGCTT GCCGCCTACGTATTACCGCGGCTGCTGGCACGTAGTTAGCCGTGGCTT CCTCGTTAGGTACCGTCAAATAAAAACCTTATTATGATTCTTACCCTT CGTCCCTAACAACAGAACTTTACGATCCTAAGACCTTCATCGTTCACG CGGCGTTGCTCCGTCAGGCTTTCGCCCATTGCGGAAGATTCCCCA |
| 19 | Pu_denov o115 | CCTGTTTGCTCCCCACGCTTTCGTGCCTCAGCGTCAGTTACAGTCCAG AGAGCCGCCTTCGCAACTGGTGTTCCTCCTAATATCTACGCATTTCAC CGCTACACTAGGAATTCCACTCTCCTCTCCTGCACTCAAGTCTTACAG TTTCAAAAGCTTACTACGGTTGAGCCGTAGCCTTTCACTTCTGACTTG AAAGACCGCCTACGCACCCTTTACGCCCAGTAATTCCGGATAACGCT AGCCCCCTACGTATTACCGCGGCTGCTGGCACGTAGTTAGCCGGGGC TTCCTCCTCAAGTACCGTCATTATCTTCCTTGAGGACAGAGCTTTACG ACCCGAAAACCTTCATCACTCACGCGGCGTTGCTGCATCAGGGTTTCC CCCATTGTGCAATATTCCCCA |
| 20 | Pu_denov o11581 | CCTGTTTGCTCCCCACGCTTTCGCGCCTCAGCGTCAGTTAATGTCCAG CAGGCCGCCTTCGCCACTGGTGTTCCTCCCAATATCTACGCATTTCAC CGCTACACTGGGAATTCCGCCTGCCTCTCCATCACTCAAGATCCGCAG TTTTGAAAGCAGTTTGGGGGTTGAGCCCCCAGATTTCACTCCCAACTT ACAGACCCGCCTACACGCCCTTTACACCCAGTAAATCCGGATAACGC TTGCTCCCTACGTATTACCGCGGCTGCTGGCACGTAGTTAGCCGGAGC TTATTCTTTAGGTACCGTCATTTTTTTCGTCCCTAATTAAAGATTTTTA CAATCCGAAGACCTTCTTCAATCACGCGGCGTTGCTGCGTCAGGGTT GCCCCCATTGCGCAATATTCCCCA |
| 21 | Pu_denov o4069 | CCTGTTTGCTCCCCACGCTTTCGAGCCTCAGCGTCAGTTACAGTCCAG AGAGTCGCCTTCGCCACTGGTGTTCTTCCTAATCTCTACGCATTTCAC CGCTACACTAGGAATTCCACTCTCCTCTCCTGCACTCTAGATAACCAG |
| | | TTTGGAATGCAGCACCCAAGTTGAGCCCGGGTATTTCACATCCCACTT AATCATCCGCCTACGCTCCCTTTACGCCCAGTAAATCCGGATAACGCT CGCCACCTACGTATTACCGCGGCTGCTGGCACGTAGTTAGCCGTGGC TTCCTCCTTGGGTACCGTCATTATCTTCCCCAAAGACAGAGCTTTACG ATCCGAAAACCTTCATCACTCACGCGGCGTTGCTGCATCAGGGTTTCC CCCATTGTGCAATATTCCCCA |
| 22 | Pu_denov o4770 | CCTGTTTGCTCCCCACGCTTTCGAGCCTCAACGTCAGTTACCGTCCAG TAAGCCGCCTTCGCCACTGGTGTTCCTCCTAATATCTACGCATTTCAC CGCTACACTAGGAATTCCGCTTACCTCTCCGGCACTCAAGATGGACA GTTTCCAATGCAGTCCCGGGGTTAAGCCCCGGGCTTTCACATCAGACT TGCCCGTCCGTCTACGCTCCCTTTACACCCAGTAAATCCGGATAACGC TTGCCCCCTACGTATTACCGCGGCTGCTGGCACGTAGTTAGCCGGGG CTTCTTAGTCAGGTACCGTCATTTCTTCCCTGCTGATAGAAGTTTAC ATACCGAGATACTTCTTCCTTCACGCGGCGTCGCTGCATCAGAGTTTC CTCCATTGTGCAATATTCCCCA |
| 23 | Pu_denov o10534 | CCTGTTTGCTACCCACGCTTTCGAACCTCAGCGTCAGTTACAGACCAG AGAGCCGCTTTCGCCACTGGTGTTCTTCCATATATCTACGCATTTCAC CGCTACACATGGAGTTCCACTCTCCTCTTCTGCACTCAAGTCTCCCAG TTTCCAATGCACTACTCCGGTTAAGCCGAAGGCTTTCACATCAGACTT AAAAGACCGCCTGCGTTCCCTTTACGCCCAATAAATCCGGATAACGC TTGCCACCTACGTATTACCGCGGCTGCTGGCACGTAGTTAGCCGTGGC TTTCTGGTTAGATACCGTCGAAACGTGAACAGTTACTCTCACGCACTT TCTTCTCTAACAACAGGGTTTACGATCCGAAGACCTTCTTCACCCAC GCGGCGTTGCTCCATCAGGCTTTCGCCCATTGTGGAAGATTCCCTA |
| 24 | Pu_denov o654 | CCTGTTTGCTCCCCACGCTTTCGAGCCTCAACGTCAGTTACTGTCCAG TAAGCCGCCTTCGCCACTGGTGTTCCTCCTAATATCTACGCATTTCAC CGCTACACTAGGAATTCCGCTTACCTCTCCAGCACTCCAGCTTAACAG TTTCCAAAGCAGTCCCGGGGTTGAGCCCCGGGCTTTCACTTCAGACTT GCTAAGCCGTCTACGCTCCCTTTACACCCAGTAAATCCGGATAACGCT TGCCCCCTACGTATTACCGCGGCTGCTGGCACGTAGTTAGCCGGGGC TTCTTAGTCAGGTACCGTCATTCTCTTCCCTGCTGATAGAGCTTTACA TACCGAAATACTTCTTCACTCACGCGGCGTCGCTGCATCAGGGTTTCC CCCATTGTGCAATATTCCCCA |
| 25 | Pu_denov o9448 | CCTGTTCGCTACCCATGCTTTCGAGCCTCAGCGTCAGTTACAGACCAG ACAGCCGCCTTCGCCACTGGTGTTCTTCCATATATCTACGCATTTCAC |
| | | CGCTACACATGGAGTTCCACTGTCCTCTTCTGCACTCAAGTCTCCCAG TTTCCAATGCACTTCTTCGGTTGAGCCGAAGGCTTTCACATTAGACTT AAAAGACCGCCTGCGCTCGCTTTACGCCCAATAAATCCGGATAACGC TTGCCACCTACGTATTACCGCGGCTGCTGGCACGTAGTTAGCCGTGGC TTTCTGGTTAAATACCGTCACTGGGTGAACAGTTACTCTCACCCACGT TCTTCTTTAACAACAGAGCTTTACGAGCCGAAACCCTTCTTCACTCAC GCGGCGTTGCTCCATCAGACTTGCGTCCATTGTGGAAGATTCCCTA |
| 26 | Pu_denov o12145 | CCTGTTTGCTCCCCACGCTTTCGAGCCTCAACGTCAGTTACCGTCCAG TAAGCCGCCTTCGCCACTGGTGTTCCTCCTAATATCTACGCATTTCAC CGCTACACTAGGAATTCCGCTTACCTCTCCGGCACTCTAGAAAAACA GTTTCCAATGCAGTCCTGGGGTTAAGCCCCAGCCTTTCACATCAGACT TGCTCTTCCGTCTACGCTCCCTTTACACCCAGTAAATCCGGATAACGC TTGCCCCCTACGTATTACCGCGGCTGCTGGCACGTAGTTAGCCGGGG CTTCTTAGTCAGGTACCGTCATTTCTTCCCTGCTGATAGAAGTTTAC ATACCGAGATACTTCTTCCTTCACGCGGCGTCGCTGCATCAGGGTTTC CCCCATTGTGCAATATTCCCCA |
| 27 | Pu_denov o7211 | CCTGTTTGCTCCCCACGCTTTCGCACCTGAGCGTCAGTCTTTGTCCAG GGGGCCGCCTTCGCCACCGGTATTCCTCCAGATCTCTACGCATTTCAC CGCTACACCTGGAATTCTACCCCCCTCTACAAGACTCTAGCCTGCCAG TTTCGAATGCAGTTCCCAGGTTGAGCCCGGGGATTTCACATCCGACTT GACAGACCGCCTGCGTGCGCTTTACGCCCAGTAATTCCGATTAACGC TTGCACCCTCCGTATTACCGCGGCTGCTGGCACGGAGTTAGCCGGTG CTTCTTCTGCGGGTAACGTCAATGAGCAAAGGTATTAACTTTACTCCC TTCCTCCCCGCTGAAAGTACTTTACAACCCGAAGGCCTTCTTCATACA CGCGGCATGGCTGCATCAGGCTTGCGCCCATTGTGCAATATTCCCCA |
| 28 | Pu_denov o1220 | CCTGTTTGCTCCCCACGCTTTCGTGCCTCAGCGTCCGTTACAGTCCAG AGAGTCGCCTTCGCACCTGGTGTTCCTCCTAATATCTACGCATTTCAC CGCTACACTAGGAATTCCACTCTCCTCTCCTGCACTCAAGTCTTACAG TTTCAAAAGCTTACTACGGTTGAGCCGTAGCCTTTCACTTCTGACTTG AAAGACCACCTACGCACCCTTTACGCCCAGTAATTCCGGATAACGCT AGCCCCCTACGTATTACCGCGGCTGCTGGCACGTAGTTAGCCGGGGC TTCCTCCTCAAGTACCGTCATTATCTTCCTTGAGGACAGAGCTTTACG ACCCGAAGGCCTTCATCGCCCACGCGGCGTTGCTGCATCAGGCTTTC GCCCATTGTGCAATATTCCCCA |
| 29 | Pu_denov o7972 | CCTGTTCGCTCCCCACGCTTTCGCTCCTCAGCGTCAGTAACGGCCCAG AGACCTGCCTTCGCCATTGGTGTTCTTCCCGATATCTACACATTCCAC CGTTACACCGGGAATTCCAGTCTCCCCTACCGCACTCAAGCCCGCCC GTACCCGGCGCGGATCCACCGTTAAGCGATGGACTTTCACACCGGAC GCGACGAACCGCCTACGAGCCCTTTACGCCCAATAATTCCGGATAAC GCTTGCACCCTACGTATTACCGCGGCTGCTGGCACGTAGTTAGCCGGT GCTTATTCAACGGGTAAACTCACTCTCGCTTGCTCCCCGATAAAAGA GGTTTACAACCCGAAGGCCTCCATCCCTCACGCGGCGTCGCTGCATC AGGCTTGCGCCCATTGTGCAATATTCCCCA |
| 30 | Pu_denov o11016 | CCTGTTTGCTCCCCACGCTTTCGAGCCTCAACGTCAGTTACCGTCCAG TAAGCCGCCTTCGCCACTGGTGTTCTTCCTAATATCTACGCATTTCAC CGCTACACTAGGAATTCCGCTTACCTCTCCGGCACTCTAGCTAAACAG TTTCCAAAGCAGTCCCGGCGTTGAGCACCGGGCTTTCACTTCAGACTT GCCTTGCCGTCTACACTCCCTTTACACCCAGTAAATCCGGATAACGCT TGCCCCCTACGTATTACCGCGGCTGCTGGCACGTAGTTAGCCGGGGC TTCTTATTCAGGTACCGTCACTTTCTTCCCTGCTGATAGAGCTTTACAT ACCGAAATACTTCTTCACTCACGCGGCGTCGCTGCATCAGGGTTTCCC CCATTGTGCAATATTCCCCA |
| 31 | Pu_denov o10356 | CCTGTTTGCTCCCCACGCTTTCGAGCCTCAGCGTCAGTTACAGACCAG AGAGCCGCCTTCGCCACTGGTGTTCCTCCATATATCTACGCATTTCAC CGCTACACATGGAATTCCACTCTCCTCTTCTGCACTCAAGTCTCCCAG TTTCCAATGACCCTCCCCGGTTGAGCCGGGGGCTTTCACATCAGACTT AAGAAACCGCCTGCGCTCGCTTTACGCCCAATAAATCCGGACAACGC TTGCCACCTACGTATTACCGCGGCTGCTGGCACGTAGTTAGCCGTGGC TTTCTGGTTAGATACCGTCAAGGATGAACAGTTACTCTCATCCTTGT TCTTCTCTAACAACAGAGTTTTACGATCCGAAAACCTTCTTCACTCAC GCGGCGTTGCTCGGTCAGACTTTCGTCCATTGCCGAAGATTCCCTA |

Figure 10 (Table 1.3) provides cumulative prediction scores for the assignment of microbiome to age group based on relative abundance of OTUs ranked by importance. P=position

### Example 2 - A method of detecting the biological age of the microbiome in adult and mature life stages

The faecal microbiota was analysed in a cohort of 41 adult Beagle dogs aged between 3.8 and 15.0 years. The study cohort included 13 animals assigned to the adult group (aged 3.8 to 6.2 years), 20 dogs assigned to the senior group (aged 8.2 to 12.9 years) and 8 dogs assigned to the geriatric group (aged 14.6 to 15.0 years).

### Methods

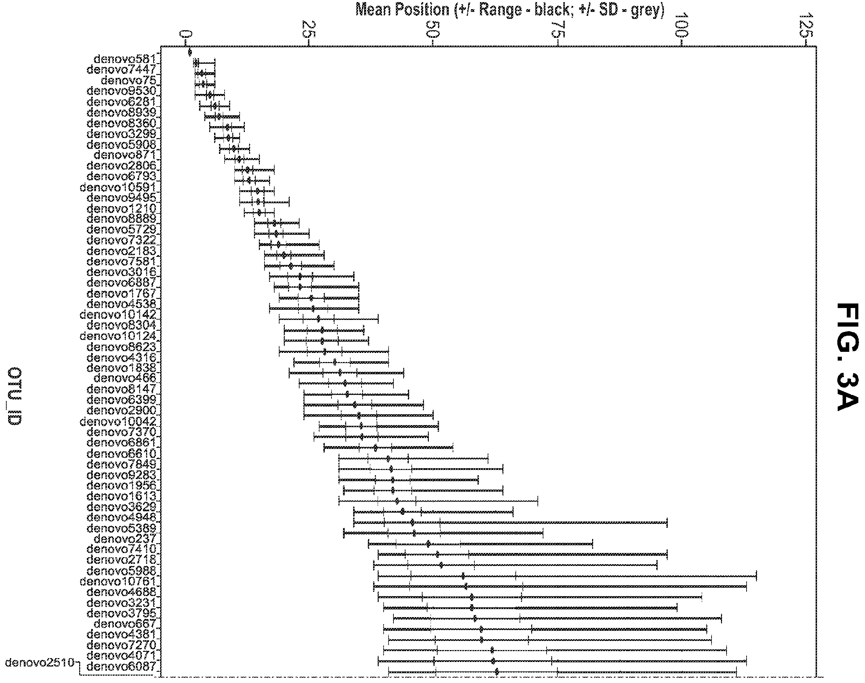

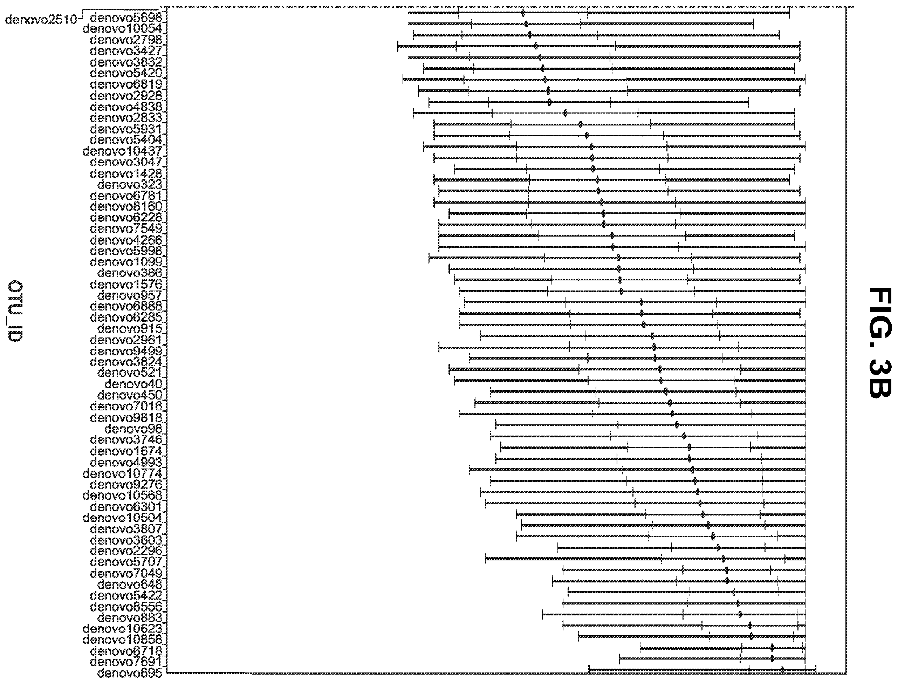

Following DNA extraction from the faecal sample and Illumina sequencing of the V4-V6 region using DNA oligonucleotide primers (sequence 319F: CAAGCAGAAG ACGGCATACG AGATGTGACT GGAGTTCAGA CGTGTGCTCT TCCGATCT and 806R: AATGATACGG CGACCACCGA GATCTACACT CTTTCCCTAC ACGACGCTCT TCCGATCT) the resulting DNA sequences were clustered at 98% identity and abundant taxa (representing >0.001 of the total sequences) were then assessed for their relative proportions. Importance scores were calculated using 2000 random forests each with 2000 trees using the default number of variables per tree (square root of the number of variables) and permutation importance score calculation. From these 2000 random forests the average ranks (position [P]) of each OTU were calculated, with 1 being the most important in determining sample fit to age (Figures 3A and 3B).

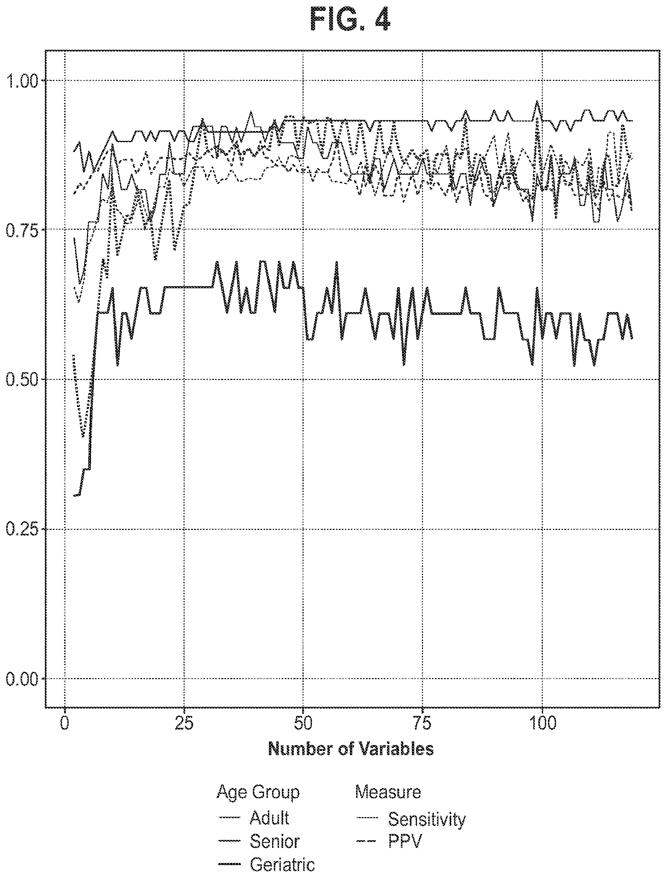

Positive predictive value, specificity and accuracy were also calculated by fitting random forests to data with successively more OTUs taken in importance order (Figure 4). Analyses were performed using R version 3.5.1 and the ranger library.

The method involves the extraction of DNA from a freshly produced faecal sample by a means such as the QIAamp Power Faecal DNA kit (Qiagen) and subsequently the use of molecular biology techniques to assess the detection rate and abundance of the combination of the bacterial taxa described below (Tables 2.1 and 2.2). The combination and abundance of the organisms detected allow the assessment of the biological age of the microbiome and assessment of whether the microbiome components observed in faeces of the animal is descriptive of an immature or older than expected microbiome relative to the actual age of the animal. After DNA extraction and sequencing of the DNA by techniques such as 16S rDNA amplicon, shotgun, metagenome, Illumina, nanopore or other DNA sequencing techniques is conducted on the faecal DNA and the assessment of relative abundance of the sequences descriptive of the organisms in table 1 or DNA sequences within 95% identical to those in Table 2.2 is made. Briefly, sequence data obtained from the test sample is clustered into groups of sequences with 98% - 100% identity and a reference sequence from the clusters which represent >0.001% of the total sequences is then used to either 1) assign taxonomy, gene function through database homologues or to determine the nature of the biomarker through homology searches of DNA databases such as the Greengenes or Silva or the NCBI non-redundant nucleotide sequence database for comparison to known DNA sequences for species held within the databases or 2) compared to the DNA sequences given in Table 2.2 below.

The number and abundance of the organisms, sequences or biomarkers identified from within the combinations described in Table 2.2 are then used to describe the biological age of the sample in terms of the group with which they best fit.

**Table 2.1: Bacterial taxa (OTUs) ranked by importance score descriptive of their consistency in assignment of samples to the appropriate age group adult (aged 3.8-6.2 years), 20 dogs assigned to the senior group (aged 8.2-12.9 years) and 8 dogs assigned to the geriatric group (aged 14.6-15.0 years). P=position**

| **P** | **OTU_I D** | **Phylu m** | **Class** | **Order** | **Family** | **Genus** | **Species** |
|---|---|---|---|---|---|---|---|
| P1 | Ad denovo 581 | Firmicutes | Clostridia | Clostridiales | Clostridiacea | *Clostridium* | *Clostridium perfringens* |
| P2 | Ad denovo 7447 | Firmicutes | Clostridia | Clostridiales | Veillonellaceae | *Phascolarctoba cterium* | *Phascolarctoba cterium sp.* |
| P3 | Ad Denovo 75 | Bacter - oidetes | Bacteroidia | Bacteroidales | Bacteroidaceae | *Bacteroides* | *Bacteroides sp.* |
| P4 | Ad_ denovo 9530 | Proteo - bacteri a | Gammaprot eobacteria | Enterobacteral es | Enterobacteriaceae | *Escherichia* /*Shigella* | *Escherichia coli* /*Shigella flexneri* |
| P5 | Ad Denovo 6281 | Bacter - oidetes | Bacteroidia | Bacteroidales | Bacteroidaceae | *Bacteroides* | *Bacteroides sp.* |
| P6 | Ad_ Denovo 8939 | Firmicutes | Bacilli | Lactobacillale s | Lactobacillaceae | *Lactobacillus* | *Lactobacillus helveticus*/*hamst eri* |
| P7 | Ad Denovo 8360 | Firmicutes | Clostridia | Clostridiales | Peptostreptococcaceae | *Terrisporobacte r* | *Terrisporobacte r sp*. |
| P8 | Ad Denovo 3299 | Firmicutes | Erysipelotri chia | Erysipelotrich ales | Erysipelotrichaceae | *Turicibacter* | *Turicibacter sp.* |
| P9 | Ad_ Denovo 5908 | Firmicutes | Clostridia | Clostridiales | Lachnospiraceae | *Blautia* | *Blautia obeum* |
| P10 | Ad Denovo 871 | Firmicutes | Clostridia | Clostridiales | Lachnospiraceae | *Blautia* | *Blautia sp.* |
| P11 | Ad_d Enovo 2806 | Firmicutes | Clostridia | Clostridiales | Clostridiacea e | *Clostridium* | *Clostridium celatum* / *Clostridium sp.* / *C. disporicum*/ *C. perfringens* |
| P12 | Ad Denovo 6793 | Fusobacteri a | Fusobacteri ia | Fusobacteriale s | Fusobacteriaceae | *Cetobacterium* | *Cetobacterium somerae* |
| P13 | Ad Denovo 10591 | Fusobacteri a | Fusobacteri ia | Fusobacteriale s | Fusobacteriaceae | *Cetobacterium* | *Cetobacterium somerae* |
| P14 | Ad Denovo 9495 | Firmicutes | Bacilli | Lactobacillale s | Lactobacillaceae | *Lactobacillus* | *Lactobacillus johnsonii* / *Lactobacillus taiwanensis*/ *Lactobacillus gasseri* |
| P15 | Ad Denovo 1210 | Firmicutes | Clostridia | Clostridiales | Lachnospiraceae | *Blautia [Ruminococcus]* | *Blautia [Ruminococcus] torques* |
| P16 | Ad_ Denovo 8889 | Firmicutes | Erysipelotri chia | Erysipelotrich ales | Erysipelₒtrichaceae | *Dubosiella* | *Dubosiella newvorkensis* |
| P17 | Ad_ Denovo 5729 | Firmicutes | Bacilli | Lactobacillale s | Streptococcaceae | *Streptococcus* | *Streptococcus alactolyticusllut eciae* |
| P18 | Ad_ Denovo 7322 | Firmicutes | Negativicut es | Selenomonada es | Selenononadaceae | *Megamonas* | *Megamonas sp.* |
| P19 | Ad Denovo 2183 | Firmicutes | Erysipelotri chia | Erysipelotrich ales | Erysipelotrichaceae | *Turicibacter* | *Turicibacter sp.* * |
| P20 | Ad Denovo 7591 | Firmicutes | Clostridia | Clostridiales | Lachnospiraceae | *Blautia* | *Blautia obeum* |
| P21 | Ad_ Denovo 3016 | Firmicutes | Clostridia | Clostridiales | Ruminococcaceae | *Ruminococcus* | *Ruminococcus gauvreauii group sp.* |
| P22 | Ad_ Denovo 6887 | Fusobacteri a | Fusobacteri ia | Fusobacteriale s | Fusobacteriaceae | *Fusobacterium* | *Fusobacterium mortiferum* |
| P23 | Ad Denovo 1767 | Firmicutes | Bacilli | Lactobacillale s | Enterococcaceae | *Enterococcus* | *Enterococcus casseliflavus*/*ha emoperoxidus*/*s ulfureus*/*asini* |
| P24 | Ad_ Denovo 4538 | Firmicutes | Clostridia | Clostridiales | Lachnospiraceae | *Lachnoclostridi um* | *Lachnospiracea e*/*Lachnoclostri dium sp.* |
| P25 | Ad Denovo 10142 | Firmicutes | Clostridia | Clostridiales | Lachnospiraceae | *Epulopiscium* | *Niameybacter massiliensis* |
| P26 | Ad denovo 6304 | Firmicutes | Erysipelotri chia | Erysipelotrichales | Erysipelotrichaceae | *Erysipelotrichac eae* | *Erysipelotrichac eae sp.* |
| P27 | Ad_ denovo 10124 | Firmicutes | Bacilli | Lactobacillales | Lactobacillaceae | *Lactobacillus* | *Lactobacillus murinus*/*Lactob acillus animalis* / *Lactobacillus apodemi* |
| P28 | Ad denovo 8723 | Firmicutes | Clostridia | Clostridiales | Lachnospiraceae | *Dorea* | *Dorea sp.* |
| P29 | Ad_ denovo 4316 | Actino - bacteri a | Coriobacter iia | Coriobacterial es | Eggerthellac eae | *Adlercreutzia* | *Adlercreutzia equolifaciens* |
| P30 | Ad denovo 1838 | Firmicutes | Clostridia | Clostridiales | Lachnospiraceae | *Dorea* | *Dorea sp.* |
| P31 | Ad_ denovo 466 | Proteo - bacteri a | Gammaprot eobacteria; | Aeromonadale s; | Succinivibrionaceae | *Anaerobiospirill um* | *Anaerobiospirill um sp.* |
| P32 | Ad_ denovo 8147 | Firmicutes | Clostridia | Clostridiales | Peptostreptococcaceae | *Romboutsia*/*Clo stridium* /*Peptostreptoco ccaceae genus* | *Romboutsia sp. Clostridium sp.K39* / *[Clostridium] dakarense*/ *Peptostreptococ caceae TM5* |
| P33 | Ad_ denovo 6399 | Firmicutes | Clostridia | Clostridiales | Lachnospiraceae | *Blautia [Ruminococcus]* | *Blautia [Ruminococcus] gnavus* |
| P34 | Ad_ denovo 2900 | Bacter - oidetes | Bacteroidia | Bacteroidales | Prevotellace ae | *Prevotella* | *Prevotella sp.* |
| P35 | Ad denovo 10042 | Firmicutes | Clostridia | Clostridiales | Lachnospiraceae | *Blautia* | *Blautia producta* |
| P36 | Ad_ denovo 7370 | No nearest known specie s | | | | | |
| P37 | Ad denovo 6861 | Firmicutes | Bacilli | Lactobacillale s | Lactobacillaceae | *Lactobacillus* | *Lactobacillus reuteri* |

**Table 2.2: DNA sequences ranked by importance scores descriptive of their consistency in assignment of samples to the appropriate life stage group in dogs (adult, senior and geriatric groups). S=SEQ ID NO.**

| **S** | **OTU_ID** | **16S rDNA partial DNA sequence** |
|---|---|---|
| 32 | Ad_denovo58 1 | CCTGTTTGCTCCCCACGCTTTCGAGCCTCAGCGTCAGTTACAGTCCAGA GAGTCGCCTTCGCCACTGGTGTTCTTCCTAATCTCTACGCATTTCACCG CTACACTAGGAATTCCACTCTCCTCTCCTGCACTCTAGATAACCAGTTT GGAATGCAGCACCCAAGTTGAGCCCGGGTATTTCACATCCCACTTAAT CATCCGCCTACGCTCCCTTTACGCCCAGTAAATCCGGATAACGCTCGCC ACCTACGTATTACCGCGGCTGCTGGCACGTAGTTAGCCGTGGCTTCCTC CTTGGGTACCGTCATTATCTTCCCCAAAGACAGAGCTTTACGATCCGA AAACCTTCATCACTCACGCGGCGTTGCTGCATCAGGGTTTCCCCCATTG TGCAATATTCCCCA |
| 33 | Ad_denovo74 47 | CCCGTTCGCTACCCTGGCTTTCGCATCTCAGCGTCAGACACAGTCCAGA AAGGCGCCTTCGCCACTGGTGTTCCTCCCAATATCTACGCATTTCACCG CTACACTGGGAATTCCCCTTTCCTCTCCTGCACTCAAGTCTTCCAGTAT CCAGAGCCATACGGGGTTGAGCCCCGCATTTTCACTCCAGACTTAAAA AACCGCCTACATGCTCTTTACGCCCAATAATTCCGGACAACGCTCGCC ACCTACGTATTACCGCGGCTGCTGGCACGTAGTTAGCCGTGGCTTCCTC GTCTACTACCGTCATTACACGTCATTGTTCACAACATGCACATTCGTCA TAGACAACAGAGCTTTACGGGACGAATCCCTTCATCACTCACGCGGCA TTGCTCCGTCAGGCTTTCGCCCATTGCGGAAGATTCCCCA |
| 34 | Ad_denovo75 | CCTGTTTGATACCCGCACTTTCGAGCATCAGCGTCAGTTACGGTCCAGC AAGCTGCCTTCGCAATCGGAGTTCTTCGTGATATCTAAGCATTTCACCG CTACACCACGAATTCCGCCTGCCTTTACCGCACTCAAGAACTCCAGTAT CAACTGCAATTTTACGGTTGAGCCGCAAACTTTCACAACTGACTTAAA ATTCCGCCTACGCTCCCTTTAAACCCAATAAATCCGGATAACGCTCGG ATCCTCCGTATTACCGCGGCTGCTGGCACGGAGTTAGCCGATCCTTATT CATACGGTACATACAAAAAGCCACACGTGGCTAACTTTATTCCCGTAT AAAAGAAGTTTACAACCCATAGGGCAGTCATCCTTCACGCTACTTGGC TGGTTCAGACTCTCGTCCATTGACCAATATTCCTCA |
| 35 | Ad_denovo95 30 | CCTGTTTGCTCCCCACGCTTTCGCACCTGAGCGTCAGTCTTCGTCCAGG GGGCCGCCTTCGCCACCGGTATTCCTCCAGATCTCTACGCATTTCACCG CTACACCTGGAATTCTACCCCCCTCTACGAGACTCAAGCTTGCCAGTAT CAGATGCAGTTCCCAGGTTGAGCCCGGGGATTTCACATCTGACTTAAC AAACCGCCTGCGTGCGCTTTACGCCCAGTAATTCCGATTAACGCTTGC ACCCTCCGTATTACCGCGGCTGCTGGCACGGAGTTAGCCGGTGCTTCTT CTGCGGGTAACGTCAATGAGCAAAGGTATTAACTTTACTCCCTTCC36T |
| | | CCCCGCTGAAAGTACTTTACAACCCGAAGGCCTTCTTCATACACGCGG CATGGCTGCATCAGGCTTGCGCCCATTGTGCAATATTCCCCA |
| 36 | Ad_denovo62 81 | CCTGTTTGATACCCGCACTTTCGAGCATCAGCGTCAGTTACGGTCCAGT AAGCTGCCTTCGCAATCGGAGTTCTTCGTGATATCTAAGCATTTCACCG CTACACCACGAATTCCGCCTACCTATACCGCACTCAAGAAATCCAGTA TCAACTGCAATTTTACGGTTGAGCCGCAAACTTTCACAACTGACTTAA ACTTCCGCCTACGCTCCCTTTAAACCCAATAAATCCGGATAACGCTCG GATCCTCCGTATTACCGCGGCTGCTGGCACGGAGTTAGCCGATCCTTAT TCATACGGTACATACAAAAAAGCACACGTGCTTCACTTTATTCCCGTAT AAAAGAAGTTTACAACCCATAGGGCAGTCATCCTTCACGCTACTTGGC TGGTTCAGACTCTCGTCCATTGACCAATATTCCTCA |
| 37 | Ad_denovo89 39 | CCTGTTCGCTACCCATGCTTTCGAGCCTCAGCGTCAGTTGCAGACCAGA GAGCCGCCTTCGCCACTGGTGTTCTTCCATATATCTACGCATTCCACCG CTACACATGGAGTTCCACTCTCCTCTTCTGCACTCAAGAAAAACAGTTT CCGATGCAGTTCCTCGGTTAAGCCGAGGGCTTTCACATCAGACTTATTC TTCCGCCTGCGCTCGCTTTACGCCCAATAAATCCGGACAACGCTTGCCA CCTACGTATTACCGCGGCTGCTGGCACGTAGTTAGCCGTGACTTTCTGG TTGATTACCGTCAAATAAAGGCCAGTTACTACCTCTATCCTTCTTCACC AACAACAGAGCTTTACGATCCGAAAACCTTCTTCACTCACGCGGCGTT GCTCCATCAGACTTTCGTCCATTGTGGAAGATTCCCTA |
| 38 | Ad_denovo83 60 | CCTGTTTGCTCCCCACGCTTTCGTGCCTCAGTGTCAGTTACAGTCCAGA AAGCCGCCTTCGCTACTGGTGTTCCTCCTAATATCTACGCATTTCACCG CTACACTAGGAATTCCACTTTCCTCTCCTGCACTCAAGTTTCCCAGTTT CAAGAGCTTACTACGGTTAAGCCGTAGCCTTTCACTCCTGACTTAAGA AACCACCTACGCACCCTTTACGCCCAGTAAATCCGGATAACGCTAGCC CCCTACGTATTACCGCGGCTGCTGGCACGTAGTTAGCCGGGGCTTCCTC CTCAAGTACCGTCATTATCTTCCTTGAGGACAGAGTTTACGACCCGAA GGCCTTCATCACTCACGCGGCGTTGCTGCATCAGGCTTTCGCCCATTGT GCAATATTCCCCA |
| 39 | Ad_denovo32 99 | CCTGTTTGCTCCCCACGCTTTCGCGCCTCAGTGTCAGTTGCAGACCAGG AAGCCGCCTTCGCCACTGGTGTTCCTCCATATCTCTACGCATTTCACCG CTACACATGGAATTCCACTTCCCTCTTCTGCACTCAAGTCAACCAGTTT CCAATGACCCTCCACGGTTAAGCCGTGGGCTTTCACATCAGACTTAATT AACCACCTGCGCGCTCTTTACGCCCAATAATTCCGGATAACGCTTGCC |
| | | ACCTACGTATTACCGCGGCTGCTGGCACGTAGTTAGCCGTGGCTTTCTC ATAAGGTACCGTCAATTGATAGTCATTTCCTCCTATCACCTTTCTTCCC TTATAACAGAATTTTACAACCCGAAGGCCTTCTTCATTCACGCGGCGTT GCTCGGTCAGGCTTTCGCCCATTGCCGAAGATTCCCTA |
| 40 | Ad_denovo59 08 | CCTGTTTGCTCCCCACGCTTTCGAGCCTCAACGTCAGTTACCGTCCAGT AAGCCGCCTTCGCCACTGGTGTTCCTCCTAATATCTACGCATTTCACCG CTACACTAGGAATTCCGCTTACCCCTCCGGTACTCAAGACTGACAGTTT CCAATGCAGTCCAGGGGTTGAGCCCCTGCCTTTCACATCAGACTTGCC ATTCCGTCTACGCTCCCTTTACACCCAGTAAATCCGGATAACGCTTGCC CCCTACGTATTACCGCGGCTGCTGGCACGTAGTTAGCCGGGGCTTCTTA GTCAGGTACCGTCATTTTCTTCCCTGCTGATAGAAGTTTACATACCGAG ATACTTCTTCCTTCACGCGGCGTCGCTGCATCAGGGTTTCCCCCATTGT GCAATATTCCCCA |
| 41 | Ad_denovo87 1 | CCTGTTTGCTCCCCACGCTTTCGAGCCTCAACGTCAGTTACCGTCCAGT AAGCCGCCTTCGCCACTGGTGTTCCTCCTAATATCTACGCATTTCACCG CTACACTAGGAATTCCGCTTACCCCTCCGGCACTCAAGCCTGGCAGTTT CCAATGCAGTCCAGGAGTTGAGCCCCTGCCTTTCACATCAGACTTGCC ATGCCGTCTACGCTCCCTTTACACCCAGTAAATCCGGATAACGCTTGCC CCCTACGTATTACCGCGGCTGCTGGCACGTAGTTAGCCGGGGCTTCTTA GTCAGGTACCGTCATTATCTTCCCTGCTGATAGAAGTTTACATACCGAA ATACTTCATCGCTCACGCGGCGTCGCTGCATCAGGGTTTCCCCCATTGT GCAATATTCCCCA |
| 42 | Ad_denovo28 06 | CCTGTTTGCTCCCCACGCTTTCGAGCCTCAGCGTCAGTTACAGTCCAGA GAATCGCCTTCGCCACTGGTGTTCTTCCTAATCTCTACGCATTTCACCG CTACACTAGGAATTCCATTCTCCTCTCCTGCACTCTAGACTTCCAGTTT GAAATGCAGCACCCAAGTTGAGCCCGGGTATTTCACATCTCACTTAAA AGTCCGCCTACGCTCCCTTTACGCCCAGTAAATCCGGACAACGCTCGC CACCTACGTATTACCGCGGCTGCTGGCACGTAGTTAGCCGTGGCTTCCT CCTCAGGTACCGTCATTATCGTCCCTGAAGACAGAGCTTTACAACCCG AAGGCCGTCATCACTCACGCGGCGTTGCTGCATCAGGGTTTCCCCCATT GTGCAATATTCCCCA |
| 43 | Ad_denovo67 93 | CCTGTTTGCTCCCCACGCTTTCGCGCTTCAGCGTCAGTATCTGTCCAGT GAGCTGACTTCTCCATCGGCATTCCTACAAATATCTACGAATTTCACCT CTACACTTGTAGTTCCGCCCACCTCTCCAGTACTCTAGAAAGACAGTTT |
| | | CCAACGCAATACGGAGTTGAGCCCCGCATTTTCACATCAGACTTATCTT TCCGCCTAGACGCGCTTTACGCCCAATAAATCCGGATAACGCTTGCGA CATACGTATTACCGCGGCTGCTGGCACGTATTTAGCCGTCGCTTCTTCT GTTGGTACCGTCACTTTCTTCTTCCCAACTGAAAGCACTTTACAATCCG AAGACCTTCATCGTGCACACAGAATTGCTGGATCAGGCTTGTGGCCCA TTGTCCAATATTCCCCA |
| 44 | Ad_denovo10 591 | CCTGTTTGCTCCCCACGCTTTCGCGCTTCAGCGTCAGTATCTGTCCAGT GAGCTGACTTCTCCATCGGCATTCCTACAAATATCTACGAATTTCACCT CTACACTTGTAGTTCCGCCCACCTCTCCAGTACTCTAGATTGGCAGTTT CCAACGCAATACGGAGTTGAGCCCCGCATTTTAACATCAGACTTACCA ATCCGCCTAGACGCGCTTTACGCCCAATAAATCCGGATAACGCTTGCG ACATACGTATTACCGCGGCTGCTGGCACGTATTTAGCCGTCGCTTCTTC TGTTGGTACCGTCACTTACTTCTTCCCAACTGAAAGCACTTTACAATCC GAAGACCTTCATCGTGCACACAGAATTGCTGGATCAGACTTTTGGTCC ATTGTCCAATATTCCCCA |
| 45 | Ad_denovo94 95 | CCTGTTCGCTACCCATGCTTTCGAGCCTCAGCGTCAGTTGCAGACCAGA GAGCCGCCTTCGCCACTGGTGTTCTTCCATATATCTACGCATTCCACCG CTACACATGGAGTTCCACTCTCCTCTTCTGCACTCAAGTTCAACAGTTT CTGATGCAATTCTCCGGTTGAGCCGAAGGCTTTCACATCAGACTTATTG AACCGCCTGCACTCGCTTTACGCCCAATAAATCCGGACAACGCTTGCC ACCTACGTATTACCGCGGCTGCTGGCACGTAGTTAGCCGTGACTTTCTA AGTAATTACCGTCAAATAAAGGCCAGTTACTACCTCTATCTTTCTTCAC TACCAACAGAGCTTTACGAGCCGAAACCCTTCTTCACTCACGCGGCGT TGCTCCATCAGACTTTCGTCCATTGTGGAAGATTCCCTA |
| 46 | Ad_denovo12 10 | CCTGTTTGCTCCCCACGCTTTCGAGCCTCAACGTCAGTTACTGTCCAGT AAGCCGCCTTCGCCACTGGTGTTCCTCCTAATATCTACGCATTTCACCG CTACACTAGGAATTCCACTTACCTCTCCAGCACTCTAGATGAACAGTTT CCAATGCAGTCCCGGGGTTGAGCCCCGGGTTTTCACATCAGACTTGCC CATCCGTCTACGCTCCCTTTACACCCAGTAAATCCGGATAACGCTTGCA CCATACGTATTACCGCGGCTGCTGGCACGTATTTAGCCGGTGCTTCTTA GTCAGGTACCGTCATTATCTTCCCTGCTGATAGAGCTTTACATACCGAA ATACTTCATCGCTCACGCGGCGTCGCTGCATCAGGGTTTCCCCCATTGT GCAATATTCCCCA |
| 47 | Ad_denovo88 89 | CCTATTTGCTCCCCACGCTTTCGTGCCTCAGCGTCAGATGCAGGCCAGA CGGCCGCCTTCGCCACCGGTGTTCTTCCATATATCTACGCATTTTACCG CTACACATGGAGTTCCACCGTCCTCTCCTGCTCTCCAGCCGGACAGTTT CCGCAGCCCCTGAAGGTTGAGCCTCCAGTTTTACTGCGGACTTGCCCG GCCGCCTGCGCACCCTTTACGCCCAATCATTCCGGATAACGCTCGCCA CCTACGTATTACCGCGGCTGCTGGCACGTAGTTAGCCGTGACTTCCTCG CCGGGTACCATCACTCAGAAAGCTTTCCACTCTTTCTGCCTTTTGTCCC CGGCAACAGAGCTTTACGATCCGAAGACCTTCCTCGCTCACGCGGCAT TGCTCGTTCAGGGTTCCCCCCATTGACGAAAATTCCCTA |
| 48 | Ad_denovo57 29 | CCTGTTTGCTCCCCACGCTTTCGAGCCTCAGCGTCAGTTACAGACCAGA GAGCCGCTTTCGCCACCGGTGTTCCTCCATATATCTACGCATTTCACCG CTACACATGGAATTCCACTCTCCCCTTCTGCACTCAAGTTTAACAGTTT CCAAAGCGAACAATGGTTAAGCCACTGCCTTTAACTTCAGACTTATTA AACCGCCTGCGCTCGCTTTACGCCCAATAAATCCGGACAACGCTCGGG ACCTACGTATTACCGCGGCTGCTGGCACGTAGTTAGCCGTCCCTTTCTG GTAAGTTACCGTCACTGTGTGAACTTTCCACTCTCACACACGTTCTTCT CTTACAACAGAGCTTTACGATCCGAAAACCTTCTTCACTCACGCGGCG TTGCTCGGTCAGGGTTGCCCCCATTGCCGAAGATTCCCTA |
| 49 | Ad_denovo73 22 | CCCGTTCGCTACCCTAGCTTTCGAGCCTCAGCGTCAGTTACAGTCCAGA AAGCCGCCTTCGCCACTGGTGTTCTTCCCAATATCTACGCATTTCACCG CTACACTGGGAATTCCGCTTTCCTCTCCTGCACTCAAGAAAATCAGTTC GGACCCCCTCACGAGGTTGAGCCCCGCACTTTTAAGATCCGCTTAATTT CCCGCCTGCGCTCCCTTTACGCCCAATAATTCCGGACAACGCTTGCCAC CTACGTATTACCGCGGCTGCTGGCACGTAGTTAGCCGTGGCTTCCTCGT TAAGTACCGTCAAATACTTACTGTATTATAATAAGCATCCTTCGCCCTT AACAACAGAACTTTACGATCCGAAGACCTTCTTCGTTCACGCGGCGTT GCTCCGTCAGGCTTTCGCCCATTGCGGAAGATTCCCCA |
| 50 | Ad_denovo21 83 | CCTGTTTGCTCCCCACGCTTTCGCGCCTCAGTGTCAGTTACAGACCAGG AAGCCGCCTTCGCCACTGGTGTTCCTCCATATCTCTACGCATTTCACCG CTACACATGGAATTCCACTTCCCTCTTCTGCACTCAAGTCGACCAGTTT CCAATGACCCTCCACGGTTAAGCCGTGGGCTTTCACATCAGACTTAAT CAACCACCTGCGCGCTCTTTACGCCCAATAATTCCGGATAACGCTCGC CACCTACGTATTACCGCGGCTGCTGGCACGTAGTTAGCCGTGGCTTTCT CATAAGGTACCGTCACACTCTAGCCATTTCCTACTAAAGTCGTTCTTCC |
| | | CTTATAACAGAATTTTACAACCCGAAGGCCTTCATCATTCACGCGGCG TTGCTCGGTCAGGCTTTCGCCCATTGCCGAAGATTCCCTA |
| 51 | Ad_denovo75 91 | CCTGTTTGCTCCCCACGCTTTCGAGCCTCAACGTCAGTTACCGTCCAGT AAGCCGCCTTCGCCACTGGTGTTCCTCCTAATATCTACGCATTTCACCG CTACACTAGGAATTCCGCTTACCCCTCCGGCACTCAAGTATGACAGTTT CCAATGCAGTCCACAGGTTGAGCCCATGCCTTTCACATCAGACTTGCC ACACCGTCTACGCTCCCTTTACACCCAGTAAATCCGGATAACGCTTGCC CCCTACGTATTACCGCGGCTGCTGGCACGTAGTTAGCCGGGGCTTCTTA GTCAGGTACCGTCACTATCTTCCCTGCTGATAGAAGTTTACATACCGAG ATACTTCTTCCTTCACGCGGCGTCGCTGCATCAGGGTTTCCCCCATTGT GCAATATTCCCCA |
| 52 | Ad_denovo30 16 | CCTGTTTGCTCCCCACGCTTTCGAGCCTCAACGTCAGTTATCGTCCAGT AAGCCGCCTTCGCCACTGGTGTTCCTCCTAATATCTACGCATTTCACCG CTACACTAGGAATTCCACTTACCCCTCCGACACTCTAGACTGACAGTTT CCAATGCAGTCCCGGGGTTGAGCCCCGGGTTTTCACATCAGACTTGCC AGTCCGTCTACGCTCCCTTTACACCCAGTAAATCCGGATAACGCTTGCC CCCTACGTATTACCGCGGCTGCTGGCACGTAGTTAGCCGGGGCTTCTTA GTCAGGTACCGTCATTTTCTTCCCTGCTGATAGAGCTTTACATACCGAA ATACTTCTTCGCTCACGCGGCGTCGCTGCATCAGGGTTTCCCCCATTGT GCAATATTCCCCA |
| 53 | Ad_denovo68 87 | CCTGTTTGCTACCCACGCTTTCGCGCTTTAGCGTCAGTATCTGTCCAGT AGGCTGGCTTCCCCATCGGCATTCCTACAAATATCTACGAATTTCACCT CTACACTTGTAGTTCCGCCTACCTCTCCAGTACTCTAGTTTGGCAGTTT CCAACGCAATACGGAGTTGAGCCCCGCATTTTCACATCAGACTTACCA AACCGCCTAGACGCGCTTTACGCCCAATAAATCCGGATAACGCTTGCG ACATACGTATTACCGCGGCTGCTGGCACGTATTTAGCCGTCGCTTCTTC TGTTGGTACCGTCACTTTCTTCTTCCCAACTGAAAGCACTTTACATTCC GAAAAACTTCATCGTGCACACAGAATTGCTGGATCAGACTTTTGGTCC ATTGTCCAATATTCCCCA |
| 54 | Ad_denovo17 67 | CCTGTTTGCTCCCCACGCTTTCGAGCCTCAGCGTCAGTTACAGACCAGA GAGCCGCCTTCGCCACTGGTGTTCCTCCATATATCTACGCATTTCACCG CTACACATGGAATTCCACTCTCCTCTTCTGCACTCAAGTCTCCCAGTTT CCAATGACCCTCCCCGGTTGAGCCGGGGGCTTTCACATCAGACTTAAG AAACCGCCTGCGCTCGCTTTACGCCCAATAAATCCGGACAACGCTTGC |
| | | CACCTACGTATTACCGCGGCTGCTGGCACGTAGTTAGCCGTGGCTTTCT GGTTAGATACCGTCAAGGATGAACAGTTACTCTCATCCTTGTTCTTCT CTAACAACAGAGTTTTACGATCCGAAAACCTTCTTCACTCACGCGGCG TTGCTCGGTCAGACTTTCGTCCATTGCCGAAGATTCCCTA |
| 55 | Ad_denovo45 38 | CCTGTTTGCTCCCCACGCTTTCGAGCCTCAACGTCAGTCATCGTCCAGT AAGCCGCCTTCGCCACTGGTGTTCCTCCTAATATCTACGCATTTCACCG CTACACTAGGAATTCCACTTACCTCTCCGACACTCTAGAAAAACAGTTT CCAATGCAGTCCCGGGGTTGAGCCCCGGGTTTTCACATCAGACTTGCC TCTCCGTCTACGCTCCCTTTACACCCAGTAAATCCGGATAACGCTTGCA CCATACGTATTACCGCGGCTGCTGGCACGTATTTAGCCGGTGCTTCTTA GTCAGGTACCGTCATTTTCTTCCCTGCTGATAGAGCTTTACATACCGAA ATACTTCATCGCTCACGCGGCGTCGCTGCATCAGGGTTTCCCCCATTGT GCAATATTCCCCA |
| 56 | Addenovo10 142 | CCTGTTCGCTCCCCACGCTTTCGTGCCTCAGCGTCAGTTACAGTCCAGA AAGCCGCCTTCGCTACTGGTGTTCCTCCGAATCTCTACGCATTTCACCG CTACACTCGGAATTCCGCTTTCCTCTCCTGCACTCTAGCAACTCAGTTT CAGATGCAGTCTCAAGGTTGAGCCCTGAGTTTTCACATCTGACTTAAAT CGCCGCCTACGCACCCTTTACACCCAGTAATTCCGGATAACGCTTGCCC CCTACGTATTACCGCGGCTGCTGGCACGTAGTTAGCCGGGGCTTCTTA GTCAGGTACCGTCATGGTTTCTTCCCTGCTGATAGAAGTTTACGATCCG AAGACCTTCATCCTTCACGCGGCGTCGCTGCATCAGGCTTGCGCCCATT GTGCAATATTCCCCA |
| 57 | Ad_denovo63 04 | CCTATTTGCTCCCCACGCTTTCGGGACTGAGCGTCAGTTATGCGCCAGA TCGTCGCCTTCGCCACTGGTGTTCCTCCATATATCTACGCATTTCACCG CTACACATGGAATTCCACGATCCTCTCACACACTCTAGCTCTACGGTTT CCATGGCTTACCGAAGTTAAGCTTCGATCTTTCACCACAGACCCTTAGT GCCGCCTGCTCCCTCTTTACGCCCAATAATTCCGGATAACGCTCGCCAC CTACGTATTACCGCGGCTGCTGGCACGTAGTTAGCCGTGGCTTTCTTAT AGAGTACCGTCACTTGGATATCATTCCCTATATCCACCGTTCTTCCTCT ATGACAGAAGTTTACATAACGAATTACTTCTTCCTTCACGCGGCGTTGC TCGGTCAGGGTTTCCCCCATTGCCGAAAATTCCCTA |
| 58 | Ad_denovo10 124 | CCTGTTTGCTACCCACGCTTTCGAACCTCAGCGTCAGTTACAGACCAGA GAGCCGCTTTCGCCACTGGTGTTCTTCCATATATCTACGCATTTCACCG CTACACATGGAGTTCCACTCTCCTCTTCTGCACTCAAGTCTCCCAGTTT |
| | | CCAATGCACTACTCCGGTTAAGCCGAAGGCTTTCACATCAGACTTAAA AGACCGCCTGCGTTCCCTTTACGCCCAATAAATCCGGATAACGCTTGC CACCTACGTATTACCGCGGCTGCTGGCACGTAGTTAGCCGTGGCTTTCT GGTTAGATACCGTCGAAACGTGAACAGTTACTCTCACGCACTTTCTTCT CTAACAACAGGGTTTACGATCCGAAGACCTTCTTCACCCACGCGGCG TTGCTCCATCAGGCTTTCGCCCATTGTGGAAGATTCCCTA |
| 59 | Ad_denovo87 23 | CCTGTTTGCTCCCCACGCTTTCGAGCCTCAACGTCAGTTACCGTCCAGT AAGCCGCCTTCGCCACTGGTGTTCCTCCTAATATCTACGCATTTCACCG CTACACTAGGAATTCCGCTTACCTCTCCGGCACTCTAGAAGCACAGTTT CCAATGCAGTCCCGTGGTTGAGCCTCGGGTTTTCACATCAGACTTGCAC TTCCGTCTACGCTCCCTTTACACCCAGTAAATCCGGATAACGCTTGCCC CCTACGTATTACCGCGGCTGCTGGCACGTAGTTAGCCGGGGCTTCTTAC TCAGGTACCGTCATTTTCTTCCCTGCTGATAGAAGTTTACATACCGAAA TACTTCATCCTTCACGCGGCGTCGCTGCATCAGGGTTTCCCCCATTGTG CAATATTCCCCA |
| 60 | Ad_denovo43 16 | CCTGTTCGCTCCCCCAGCTTTCGCGCCTCAGCGTCAGTGGCGGCCCAGC AGGCTGCCTTCGCCATCGGTGTTCTTCCCGATATCTGCGCATTCCACCG CTACACCGGGAATTCCGCCTGCCTCTACCGCACTCGAGCCGCCCAGTC CGGAACCCGGCCCGAGGTTGAGCCCCGGGATTAGAGGTTCCGCTTAGG CGGCCGCCTACGCGCGCTTTACGCCCAATGAATCCGGATAACGCTCGC CCCCTACGTATTACCGCGGCTGCTGGCACGTAGTTAGCCGGAGCTTCTT CTGCAGGTACCGTCTATGTCTTCCCTGCTGAAAGCGGTTTACAACCCGA AGGCCTTCGTCCCGCACGCGGCGTTGCTGCGTCAGGGTTTCCCCCATTG CGCAAAATTCCCCA |
| 61 | Ad_denovo18 38 | CCTGTTTGCTACCCACGCTTTCGAGCCTCAGCGTCAGTTAAAGCCCAGC AGGCCGCCTTCGCCACTGGTGTTCCTCCTAATATCTACGCATTTCACCG CTACACTAGGAATTCCGCCTGCCTCTACTTCACTCAAGAAAAACAGTTT TGAAAGCAGCTCATGGGTTGAGCCCATGCATTTCACTTCCAACTTGCTT TCCCGCCTACGCTCCCTTTACACCCAGTAATTCCGGACAACGCTCGCTC CCTACGTATTACCGCGGCTGCTGGCACGTAGTTAGCCGGAGCTTCCTTC TTCGGTACCGTCACTTTCTTCGTCCCGAATGACAGAGGTTTACAACCCG AAGGCCGTCTTCCCTCACGCGGCGTCGCTGCATCAGGCTTTCGCCCATT GTGCAATATCCCCCA |
| 62 | Ad_denovo46 6 | CCTGTTTGCTCCCCACGCTTTCGCACCTCAGCGTCAGTATTGCCCAGG AGACCGCCTTCGCCTCTGGTGTTCTTTCAGATATCTACGCATTCCACCG CTACACCTGAAATTCCATCTCCCTCTGCAATACTCTAGAAAAGTAGTTC AAAACGACCAACCCAGGTTGAGCCCGGGTATTTCACGCCTTGCTTGCT TTTCCGCCTACATGCCCTTTACGCCCAGTTATTCCGATTAACGCTTGCA CCCTCCGTATTACCGCGGCTGCTGGCACGGAGTTAGCCGGTGCTTCTTC TGTGGGTAACGTCAGTGTTTCCTCCCCACTGAAAGTGCTTTACAACCCG AAGGCCTTCTTCACACACGCGGCATGGCTGCATCAGGGTTTCCCCCATT GTGCAATATTCCCTA |
| 63 | Ad_denovo81 47 | CCTGTTTGCTCCCCACGCTTTCGTGCCTCAGTGTCAGTTACAGTCCAGA GAGCCGCCTTCGCAACTGGTATTCCTCCTAATATCTACGCATTTCACCG CTACACTAGGAATTCTACTCTCCTCTCCTGCACTCAAGTTTCTCAGTTT CAAAGGCTTACTACGGTTGAGCCGTAGCCTTTCACCTCTGACTTAAGA AACCACCTACGCACCCTTTACGCCCAGTAATTCCGGATAACGCTAGCC CCCTACGTATTACCGCGGCTGCTGGCACGTAGTTAGCCGGGGCTTCCTC CTCAAGTACCGTCATTATCTTCCTTGAGGACAGAGCTTTACGACCCGA AGGCCTTCATCGCTCACGCGGCGTTGCTGCATCAGGCTTTCGCCCATTG TGCAATATTCCCCA |
| 64 | Ad_denovo63 99 | CCTGTTTGCTCCCCACGCTTTCGAGCCTCAACGTCAGTCATCGTCCAGA AAGCCGCCTTCGCCACTGGTGTTCCTCCTAATATCTACGCATTTCACCG CTACACTAGGAATTCCGCTTTCCTCTCCGACACTCTAGCCTGACAGTTC CAAATGCAGTCCCGGGGTTGAGCCCCGGGCTTTCACATCTGGCTTGCC ATGCCGTCTACGCTCCCTTTACACCCAGTAAATCCGGATAACGCTTGCC CCCTACGTATTACCGCGGCTGCTGGCACGTAGTTAGCCGGGGCTTCTTA GTCAGGTACCGTCATTTTCTTCCCTGCTGATAGAGCTTTACATACCGAA ATACTTCATCGCTCACGCGGCGTCGCTGCATCAGGGTTTCCCCCATTGT GCAATATTCCCCA |
| 65 | Ad_denovo29 00 | CCTGTTTGATACCCGCACTTTCGAGCCTCAGCGTCAGTTACACTCCAGA TACCTGCCTTCGCGATCGGAGTTCCTCATGATATCTGAGCATTTCACCG CTACACCATGAATTCCAGTATCTCTGCGTGTACTCAAGACTCCCAGTAT CAACTGCAGTCCGACGGTTGAGCCGCCGTATTTCACAACTGACTTAAG AGTCCGCCTGCGCTCCCTTTAAACCCAATAAATCCGGATAACGCCTGG ACCTTCCGTATTACCGCGGCTGCTGGCACGGAATTAGCCGGTCCTTTTT CTGATGGTACATACAAAACGGTACACGTACCGCACTTTATTCCCAACT |
| | | AAAAGCAGTTTACAACCCAGAGGGCCGTCATCCTGCACGCTACTTGGC TGGTTCAGACTTCCGTCCATTGACCAATATTCCTCA |
| 66 | Ad_denovo10 042 | CCTGTTTGCTCCCCACGCTTTCGAGCCTCAACGTCAGTTACCGTCCAGT AAGCCGCCTTCGCCACTGGTGTTCCTCCTAATATCTACGCATTTCACCG CTACACTAGGAATTCCGCTTACCTCTCCGGCACTCTAGATGGACAGTTT CCAAAGCAGTCCAGGGGTTGAGCCCCTGCCTTTCACTTCAGACTTGCC CGTCCGTCTACGCTCCCTTTACACCCAGTAAATCCGGATAACGCTTGCC CCCTACGTATTACCGCGGCTGCTGGCACGTAGTTAGCCGGGGCTTCTTA GTCAGGTACCGTCATTGTCTTCCCTGCTGATAGAAGTTTACATACCGAG ATACTTCTTCCTTCACGCGGCGTCGCTGCATCAGGGTTTCCCCCATTGT GCAATATTCCCTA |
| 67 | Ad_denovo73 70 | CCTGTTTGCTCCCCACGCTTTCGCACCTCAGCGTCAGTATTGCCCAGG AGACCGCCTTCGCCTCTGGTGTTCTTTCAGATATCTACGCATTCCACCG CTACACCTGAAATTCCATCTCCCTCTGCAATACTCTAGAAAGGTAGTTC AAAATGACCAACCCAGGTTGAGCCCAGGTCTTTCACATCTTGCTTGCCT TTCCGCCTACATGCCCTTTACGCCCAGTTATTCCGATTAACGCTTGCAC CCTCCGTATTACCGCGGCTGCTGGCACGGAGTTAGCCGGTGCTTCTTCT GTGGGTAACGTCAGTATTTCCTCCCCACTGAAAGTGCTTTACAACCCG AAGGCCTTCTTCACACACGCGGCATGGCTGCATCAGGGTTTCCCCCATT GTGCAATATTCCCCA |
| 68 | Ad_denovo68 61 | CCTGTTCGCTACCCATGCTTTCGAGCCTCAGCGTCAGTTGCAGACCAGA CAGCCGCCTTCGCCACTGGTGTTCTTCCATATATCTACGCATTCCACCG CTACACATGGAGTTCCACTGTCCTCTTCTGCACTCAAGTCGCCCGGTTT CCGATGCACTTCTTCGGTTAAGCCGAAGGCTTTCACATCAGACCTAAG CAACCGCCTGCGCTCGCTTTACGCCCAATAAATCCGGATAACGCTTGC CACCTACGTATTACCGCGGCTGCTGGCACGTAGTTAGCCGTGACTTTCT GGTTGGATACCGTCACTGCGTGAACAGTTACTCTCACGCACGTTCTTCT CCAACAACAGAGCTTTACGAGCCGAAACCCTTCTTCACTCACGCGGTG TTGCTCCATCAGGCTTGCGCCCATTGTGGAAGATTCCCTA |

### Example 3 - A method of detecting the biological age of the microbiome (data from a study of adult, senior and geriatric dogs)

The faecal microbiota was analysed in a cohort of 41 adult Beagle dogs aged between 3.8 and 15.0 years. The study cohort included 13 animals assigned to the adult group (aged 3.8-6.2 years), 20 dogs assigned to the senior group (aged 8.2-12.9 years) and 8 dogs assigned to the geriatric group (aged 14.6-15.0 years).

### Methods

Following DNA extraction from the faecal sample and Illumina sequencing of the V4-V6 region using DNA oligonucleotide primers (sequence (319F: CAAGCAGAAG ACGGCATACG AGATGTGACT GGAGTTCAGA CGTGTGCTCT TCCGATCT and 806R: AATGATACGG CGACCACCGA GATCTACACT CTTTCCCTAC ACGACGCTCT TCCGATCT) the resulting DNA sequences were clustered at 98% identity and abundant taxa (representing >0.001 of the total sequences) were then assessed for their relative proportions. Bacterial taxon data were analysed for correlations between samples and the detection and abundance of the OTUs (bacterial taxa) present using partial least squares discriminate analysis (PLSDA).

### Method

The method involves using the combination of bacterial taxa below to detect the biological age of the microbiome. After DNA sequencing the relative abundance data obtained from the test sample is clustered with the control dataset and the cluster in which the sample sits (Figure 6) describes the biological age of the sample. An example analysis supporting detection of biological age of the microbiome compared to control samples is given in Figure 6. This figure demonstrates separation of the canine geriatric microbiome samples.

**Table 3.1: Bacterial taxa (OTUs) with variable importance in projection (VIP) scores >1 contributing to separation of life stage groups in partial least squares analysis. P=PLSDA order**

| **P** | **OTU** | **Phylum** | **Taxon designation** |
|---|---|---|---|
| P1 | Ad_denovo9495 | Firmicutes | *Lactobacillus johnsonii* / *Lactobacillus taiwanensis*/ *Lactobacillus gasseri* |
| P2 | Ad_denovo10124 | Firmicutes | *Lactobacillus murinus*/*Lactobacillus animalis* / *Lactobacillus apodemi* |
| P3 | Ad_denovo9530 | Proteobacteria | *Escherichia coli* /*Shigella flexneri* |
| P4 | Ad_denovo6887 | Fusobacteria | *Fusobacterium mortiferum* |
| P5 | Ad_denovo3832 | Fusobacteria | *Fusobacterium sp.* |
| P6 | Ad_denovo2900 | Bacteroidetes | *Prevotella sp.* |
| P7 | Ad_denovo75 | Bacteroidetes | *Bacteroides sp.* |
| P8 | Ad_denovo6281 | Bacteroidetes | *Bacteroides sp.* |
| P9 | Ad_denovo466 | Proteobacteria | *Anacerobiospirillum sp.* |
| P10 | Ad_denovo7849 | Bacteroidetes | *Prevotella sp.* |
| P11 | Ad_denovo7322 | Firmicutes | *Megamonas sp.* |
| P12 | Ad_denovo3299 | Firmicutes | *Turicibacter sp.* |
| P13 | Ad_denovo8147 | Firmicutes | *Clostridium sp.K39* / *[Clostridium] dakarense*/ *Peptostreptococcaceae TM5* |
| P14 | Ad_denovo2806 | Firmicutes | *Clostridium celatum* / *Clostridium sp. BG-C151* / *Clostridium disporicum*/*perfringens* |
| P15 | Ad_denovo2183 | Firmicutes | *Turicibacter sp.* |

Taxa can also be described by the following DNA sequences (covers any organism with these DNA sequences between 100% and 98% similarity / identity to the sequences below).

**Table 3.2. DNA sequences for bacterial taxa most influential in separation of life stage groups in partial least squares analysis. S= SEQ ID NO**

| **Taxon reference** | **S** | **16S rDNA partial DNA sequence** |
|---|---|---|
| Ad_denovo9495 | 45 | CCTGTTCGCTACCCATGCTTTCGAGCCTCAGCGTCAGTTGCAGACC AGAGAGCCGCCTTCGCCACTGGTGTTCTTCCATATATCTACGCATT CCACCGCTACACATGGAGTTCCACTCTCCTCTTCTGCACTCAAGTTC AACAGTTTCTGATGCAATTCTCCGGTTGAGCCGAAGGCTTTCACAT CAGACTTATTGAACCGCCTGCACTCGCTTTACGCCCAATAAATCCG GACAACGCTTGCCACCTACGTATTACCGCGGCTGCTGGCACGTAGT TAGCCGTGACTTTCTAAGTAATTACCGTCAAATAAAGGCCAGTTAC TACCTCTATCTTTCTTCACTACCAACAGAGCTTTACGAGCCGAAAC |
| | | CCTTCTTCACTCACGCGGCGTTGCTCCATCAGACTTTCGTCCATTGT GGAAGATTCCCTA |
| Ad_denovo10124 | 58 | CCTGTTTGCTACCCACGCTTTCGAACCTCAGCGTCAGTTACAGACC AGAGAGCCGCTTTCGCCACTGGTGTTCTTCCATATATCTACGCATTT CACCGCTACACATGGAGTTCCACTCTCCTCTTCTGCACTCAAGTCTC CCAGTTTCCAATGCACTACTCCGGTTAAGCCGAAGGCTTTCACATC AGACTTAAAAGACCGCCTGCGTTCCCTTTACGCCCAATAAATCCGG ATAACGCTTGCCACCTACGTATTACCGCGGCTGCTGGCACGTAGTT AGCCGTGGCTTTCTGGTTAGATACCGTCGAAACGTGAACAGTTACT CTCACGCACTTTCTTCTCTAACAACAGGGTTTACGATCCGAAGAC CTTCTTCACCCACGCGGCGTTGCTCCATCAGGCTTTCGCCCATTGTG GAAGATTCCCTA |
| Ad_denovo9530 | 35 | CCTGTTTGCTCCCCACGCTTTCGCACCTGAGCGTCAGTCTTCGTCCA GGGGGCCGCCTTCGCCACCGGTATTCCTCCAGATCTCTACGCATTT CACCGCTACACCTGGAATTCTACCCCCCTCTACGAGACTCAAGCTT GCCAGTATCAGATGCAGTTCCCAGGTTGAGCCCGGGGATTTCACAT CTGACTTAACAAACCGCCTGCGTGCGCTTTACGCCCAGTAATTCCG ATTAACGCTTGCACCCTCCGTATTACCGCGGCTGCTGGCACGGAGT TAGCCGGTGCTTCTTCTGCGGGTAACGTCAATGAGCAAAGGTATTA ACTTTACTCCCTTCCTCCCCGCTGAAAGTACTTTACAACCCGAAGG CCTTCTTCATACACGCGGCATGGCTGCATCAGGCTTGCGCCCATTG TGCAATATTCCCCA |
| Ad_denovo6887 | 53 | CCTGTTTGCTACCCACGCTTTCGCGCTTTAGCGTCAGTATCTGTCCA GTAGGCTGGCTTCCCCATCGGCATTCCTACAAATATCTACGAATTT CACCTCTACACTTGTAGTTCCGCCTACCTCTCCAGTACTCTAGTTTG GCAGTTTCCAACGCAATACGGAGTTGAGCCCCGCATTTTCACATCA GACTTACCAAACCGCCTAGACGCGCTTTACGCCCAATAAATCCGGA TAACGCTTGCGACATACGTATTACCGCGGCTGCTGGCACGTATTTA GCCGTCGCTTCTTCTGTTGGTACCGTCACTTTCTTCTTCCCAACTGA AAGCACTTTACATTCCGAAAAACTTCATCGTGCACACAGAATTGCT GGATCAGACTTTTGGTCCATTGTCCAATATTCCCCA |
| Ad_denovo3832 | 69 | CCTGTTTGCTCCCCATGCTTTCGCGCTTCAGCGTCAGTATCTGTCCA GTGAGCTGACTTCTCTATCGGCATTCCTACAAATATCTACGAATTT CACCTCTACACTTGTAGTTCCGCCCACCTCTCCAGTACTCTAGTCTG |
| | | GCAGTTTCCAACGCAATACGGAGTTGAGCCCCGCATTTCCACATCA GACTTACCAGACCGCCTAGACGCGCTTTACGCCCAATAAATCCGGA TAACGCTTGCGACATACGTATTACCGCGGCTGCTGGCACGTATTTA GCCGTCGCTTCTTCTATCGGTACCATCACTTTCTTTTTCCCGATTGA AAGCACTTTACAATCCTAAGACCGTCATCGTGCACACAGAATTGCT GGATCAGACTTTTGGTCCATTGTCCAATATTCCCCA |
| Ad_denovo2900 | 65 | CCTGTTTGATACCCGCACTTTCGAGCCTCAGCGTCAGTTACACTCC AGATACCTGCCTTCGCGATCGGAGTTCCTCATGATATCTGAGCATT TCACCGCTACACCATGAATTCCAGTATCTCTGCGTGTACTCAAGAC TCCCAGTATCAACTGCAGTCCGACGGTTGAGCCGCCGTATTTCACA ACTGACTTAAGAGTCCGCCTGCGCTCCCTTTAAACCCAATAAATCC GGATAACGCCTGGACCTTCCGTATTACCGCGGCTGCTGGCACGGAA TTAGCCGGTCCTTTTTCTGATGGTACATACAAAACGGTACACGTAC CGCACTTTATTCCCAACTAAAAGCAGTTTACAACCCAGAGGGCCGT CATCCTGCACGCTACTTGGCTGGTTCAGACTTCCGTCCATTGACCA ATATTCCTCA |
| Ad_denovo75 | 34 | CCTGTTTGATACCCGCACTTTCGAGCATCAGCGTCAGTTACGGTCC AGCAAGCTGCCTTCGCAATCGGAGTTCTTCGTGATATCTAAGCATT TCACCGCTACACCACGAATTCCGCCTGCCTTTACCGCACTCAAGAA CTCCAGTATCAACTGCAATTTTACGGTTGAGCCGCAAACTTTCACA ACTGACTTAAAATTCCGCCTACGCTCCCTTTAAACCCAATAAATCC GGATAACGCTCGGATCCTCCGTATTACCGCGGCTGCTGGCACGGAG TTAGCCGATCCTTATTCATACGGTACATACAAAAAGCCACACGTGG CTAACTTTATTCCCGTATAAAAGAAGTTTACAACCCATAGGGCAGT CATCCTTCACGCTACTTGGCTGGTTCAGACTCTCGTCCATTGACCA ATATTCCTCA |
| Ad_denovo6281 | 36 | CCTGTTTGATACCCGCACTTTCGAGCATCAGCGTCAGTTACGGTCC AGTAAGCTGCCTTCGCAATCGGAGTTCTTCGTGATATCTAAGCATT TCACCGCTACACCACGAATTCCGCCTACCTATACCGCACTCAAGAA ATCCAGTATCAACTGCAATTTTACGGTTGAGCCGCAAACTTTCACA ACTGACTTAAACTTCCGCCTACGCTCCCTTTAAACCCAATAAATCC GGATAACGCTCGGATCCTCCGTATTACCGCGGCTGCTGGCACGGAG TTAGCCGATCCTTATTCATACGGTACATACAAAAAAGCACACGTGC TTCACTTTATTCCCGTATAAAAGAAGTTTACAACCCATAGGGCAGT |
| | | CATCCTTCACGCTACTTGGCTGGTTCAGACTCTCGTCCATTGACCA ATATTCCTCA |
| Ad_denovo466 | 62 | CCTGTTTGCTCCCCACGCTTTCGCACCTCAGCGTCAGTATTTGCCCA GGAGACCGCCTTCGCCTCTGGTGTTCTTTCAGATATCTACGCATTCC ACCGCTACACCTGAAATTCCATCTCCCTCTGCAATACTCTAGAAAA GTAGTTCAAAACGACCAACCCAGGTTGAGCCCGGGTATTTCACGCC TTGCTTGCTTTTCCGCCTACATGCCCTTTACGCCCAGTTATTCCGAT TAACGCTTGCACCCTCCGTATTACCGCGGCTGCTGGCACGGAGTTA GCCGGTGCTTCTTCTGTGGGTAACGTCAGTGTTTCCTCCCCACTGA AAGTGCTTTACAACCCGAAGGCCTTCTTCACACACGCGGCATGGCT GCATCAGGGTTTCCCCCATTGTGCAATATTCCCTA |
| Ad_denovo7849 | 70 | CCTGTTCGATACCCGCACTTTCGAGCTTCAGCGTCAGTTGCGCTCC AGTGAGCTGCCTTCGCAATCGGAGTTCTTCGTGATATCTAAGCATT TCACCGCTACACCACGAATTCCGCCCACTTTGTGCGTACTCAAGGA AACCAGTTCGCGCTGCAGTGCAGACGTTGAGCGTCTACATTTCACA ACACGCTTAATCTCCGGCCTACGCTCCCTTTAAACCCAATAAATCC GGATAACGCCCGGACCTTCCGTATTACCGCGGCTGCTGGCACGGA ATTAGCCGGTCCTTATTCATAAGGTACATGCAAAAAGAGTCACGAC TCCCACTTTATTCCCTTATAAAAGCAGTTTACAACCCATAGGGCCG TCATCCTGCACGCTACTTGGCTGGTTCAGACTCTCGTCCATTGACC AATATTCCTCA |
| Ad_denovo7322 | 49 | CCCGTTCGCTACCCTAGCTTTCGAGCCTCAGCGTCAGTTACAGTCC AGAAAGCCGCCTTCGCCACTGGTGTTCTTCCCAATATCTACGCATT TCACCGCTACACTGGGAATTCCGCTTTCCTCTCCTGCACTCAAGAA AATCAGTTCGGACCCCCTCACGAGGTTGAGCCCCGCACTTTTAAGA TCCGCTTAATTTCCCGCCTGCGCTCCCTTTACGCCCAATAATTCCGG ACAACGCTTGCCACCTACGTATTACCGCGGCTGCTGGCACGTAGTT AGCCGTGGCTTCCTCGTTAAGTACCGTCAAATACTTACTGTATTAT AATAAGCATCCTTCGCCCTTAACAACAGAACTTTACGATCCGAAGA CCTTCTTCGTTCACGCGGCGTTGCTCCGTCAGGCTTTCGCCCATTGC GGAAGATTCCCCA |
| Ad_denovo3299 | 39 | CCTGTTTGCTCCCCACGCTTTCGCGCCTCAGTGTCAGTTGCAGACC AGGAAGCCGCCTTCGCCACTGGTGTTCCTCCATATCTCTACGCATT TCACCGCTACACATGGAATTCCACTTCCCTCTTCTGCACTCAAGTC |
| | | AACCAGTTTCCAATGACCCTCCACGGTTAAGCCGTGGGCTTTCACA TCAGACTTAATTAACCACCTGCGCGCTCTTTACGCCCAATAATTCC GGATAACGCTTGCCACCTACGTATTACCGCGGCTGCTGGCACGTAG TTAGCCGTGGCTTTCTCATAAGGTACCGTCAATTGATAGTCATTTCC TCCTATCACCTTTCTTCCCTTATAACAGAATTTACAACCCGAAGGC CTTCTTCATTCACGCGGCGTTGCTCGGTCAGGCTTTCGCCCATTGCC GAAGATTCCCTA |
| Ad_denovo8147 | 63 | CCTGTTTGCTCCCCACGCTTTCGTGCCTCAGTGTCAGTTACAGTCCA GAGAGCCGCCTTCGCAACTGGTATTCCTCCTAATATCTACGCATTT CACCGCTACACTAGGAATTCTACTCTCCTCTCCTGCACTCAAGTTTC TCAGTTTCAAAGGCTTACTACGGTTGAGCCGTAGCCTTTCACCTCT GACTTAAGAAACCACCTACGCACCCTTTACGCCCAGTAATTCCGGA TAACGCTAGCCCCCTACGTATTACCGCGGCTGCTGGCACGTAGTTA GCCGGGGCTTCCTCCTCAAGTACCGTCATTATCTTCCTTGAGGACA GAGCTTTACGACCCGAAGGCCTTCATCGCTCACGCGGCGTTGCTGC ATCAGGCTTTCGCCCATTGTGCAATATTCCCCA |
| Ad_denovo2806 | 42 | CCTGTTTGCTCCCCACGCTTTCGAGCCTCAGCGTCAGTTACAGTCC AGAGAATCGCCTTCGCCACTGGTGTTCTTCCTAATCTCTACGCATTT CACCGCTACACTAGGAATTCCATTCTCCTCTCCTGCACTCTAGACTT CCAGTTTGAAATGCAGCACCCAAGTTGAGCCCGGGTATTTCACATC TCACTTAAAAGTCCGCCTACGCTCCCTTTACGCCCAGTAAATCCGG ACAACGCTCGCCACCTACGTATTACCGCGGCTGCTGGCACGTAGTT AGCCGTGGCTTCCTCCTCAGGTACCGTCATTATCGTCCCTGAAGAC AGAGCTTTACAACCCGAAGGCCGTCATCACTCACGCGGCGTTGCTG CATCAGGGTTTCCCCCATTGTGCAATATTCCCCA |
| Ad_denovo2183 | 50 | CCTGTTTGCTCCCCACGCTTTCGCGCCTCAGTGTCAGTTACAGACC AGGAAGCCGCCTTCGCCACTGGTGTTCCTCCATATCTCTACGCATT TCACCGCTACACATGGAATTCCACTTCCCTCTTCTGCACTCAAGTC GACCAGTTTCCAATGACCCTCCACGGTTAAGCCGTGGGCTTTCACA TCAGACTTAATCAACCACCTGCGCGCTCTTTACGCCCAATAATTCC GGATAACGCTCGCCACCTACGTATTACCGCGGCTGCTGGCACGTAG TTAGCCGTGGCTTTCTCATAAGGTACCGTCACACTCTAGCCATTTCC TACTAAAGTCGTTCTTCCCTTATAACAGAATTTTACAACCCGAAGG |
| | | CCTTCATCATTCACGCGGCGTTGCTCGGTCAGGCTTTCGCCCATTGC CGAAGATTCCCTA |

### Example 4: Puppy Microbiota Blautia spp., Clostridium hiranonsis and Megamonas

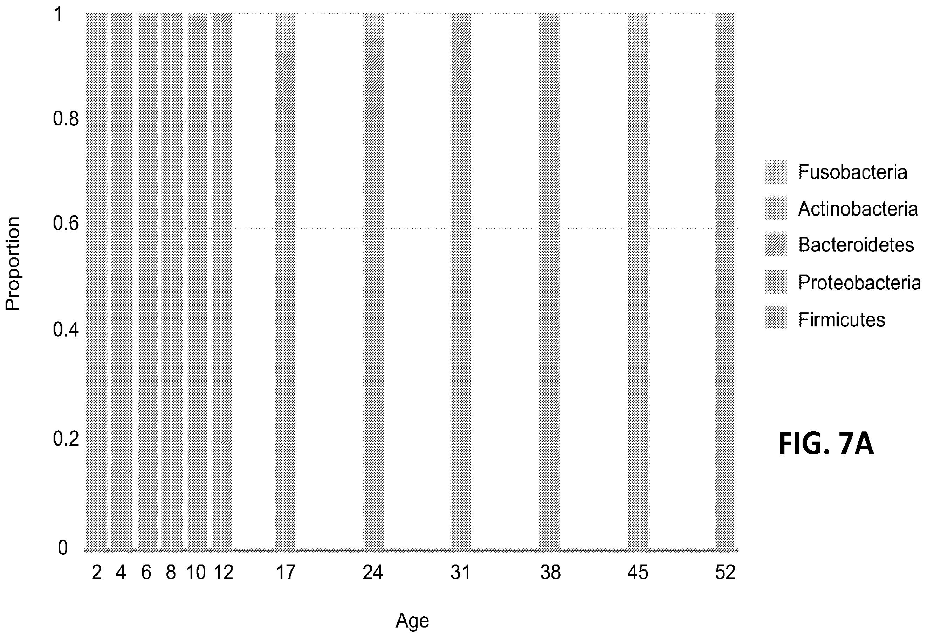

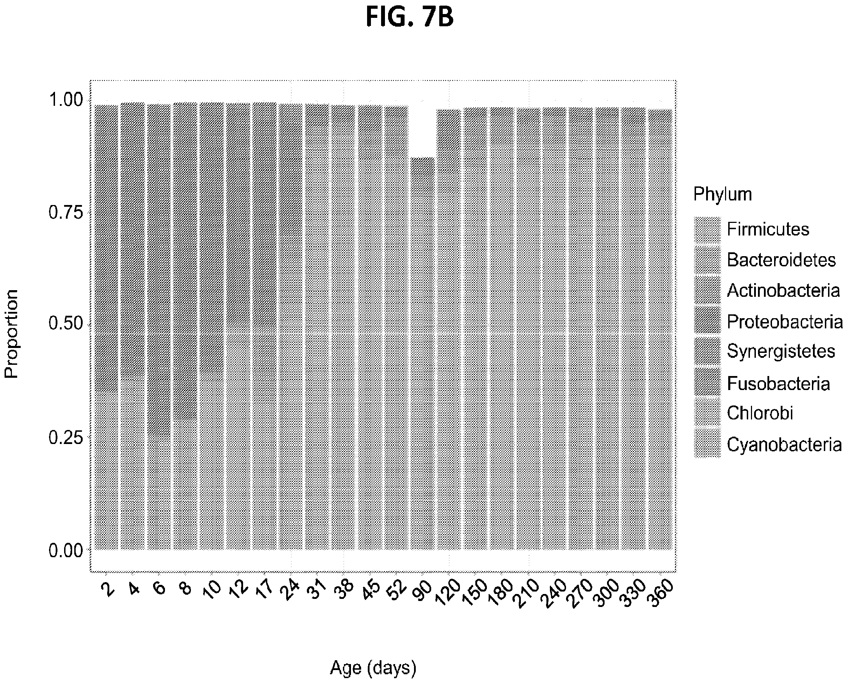

A recent study of the puppy faecal microbiota described changes in the bacterial communities detected within the faeces of healthy puppies during the first year of life. The microbiota detected within the faeces of healthy puppies during the first year of life demonstrated that in the period before weaning, the most common types of bacteria belong to the Phylum Proteobacteria (Figures 7A and 7B). After weaning bacteria from the phylum Firmicutes were the most abundant detected. The diversity of the bacterial community also increased after weaning.

The initial elements of the puppy microbiota are likely from a maternal source and include *Staphylococcus aureus* and *Bifidobacterium longum,* which is known to be able to exploit the oligosaccharides present in the maternal milk, and a *Clostridium* sensu stricto 1 sp., amongst others. The presence of these taxa suggests that they are able to exploit the environment of the neonatal gut, given the availability of a source of nutrients from maternal milk, and the tolerance of various environmental stressors such as an unfavourable pH.

Following weaning, a number of species become more prevalent in the neonatal gut. The most notable examples are *Megamonas sp.* and *Blautia* sp. both of which are prolific fermenters of complex carbohydrates and producers of short chain fatty acids. In general, these species are associated with a healthy gut microbiome due to their production of short chain fatty acids, and their decreased abundance in dogs with diarrhoea [20,21] and canine IBD [22]. Following this, a large and sustained increase in *Clostridium hiranonsis* was observed, such that this becomes the most abundant taxa at all sampling points from Day 52 onwards until the final sampling point at Day 360. This species is also associated with a healthy gut microbiota, being involved in deconjugation of bile acids and decreased in cases of canine chronic enteropathy [22] and having a reported ability to inhibit the pathogen *Clostridium difficile* via secondary bile acids [23]. Overall, the increased abundance of *Blautia* spp., *Clostridium hiranonsis* and *Megamonas* spp. post-weaning indicate a healthy microbiota in puppies and adult dogs.

### Example 5: Allobacullum, Peptostreptococcus and core Bifidobacterium, Lactobacillus, and Enterococcus.

In a study of 22 dogs receiving a course of metronidazole prophylaxis for clinical signs of gastrointestinal dysbiosis, the faecal microbiota was assessed prior to, during, and following treatment. The study aimed to assess the extent, variability, and longevity of metronidazole treatment on the faecal microbiota in dogs. Metronidazole treatment was associated with a reduction in diarrhoea within the cohort. Assessment of the faecal microbiota by 16S rRNA gene amplicon sequencing revealed reduced Shannon diversity and altered community composition during and immediately following treatment. While the animals received metronidazole, a core microbiota, dominated by OTU (sequence type) assigned to the lactic acid bacteria (*Bifidobacterium, Lactobacillus, and Enterococcus*) was observed across the cohort. This core microbiota representative of the organisms associated with metronidazole treatment was enriched for operational taxonomic units assigned to the genera *Bifidobacterium, Lactobacillus, and Enterococcus.* Diversity and species richness of the faecal microbiota increased to a post-treatment plateau around 4 weeks following the cessation of treatment. The increase in microbial diversity was associated with an apparent evolution within the microbial community composition of individuals, characterised by consistent signatures at both the OTU and genus taxonomic levels.Metronidazole treatment was associated with reduced microbial diversity, establishment of a core microbiota, and conserved features indicative of a consistent hierarchy in the evolution of gut microbiota community composition during the re-establishment of microbial diversity across individuals. The core microbiota associated with metronidazole treatment was enriched for sequences assigned to the lactic acid bacteria suggestive of innate resistance and the capability to perform activities essential to gut microbiome function. The composition of the microbiota during and immediately following treatment was dominated by lactic acid bacteria from the genera *Lactobacillus, Bifidobacterium,* and *Enterococcus.* The enhanced relative abundance of these genera, considered to be associated with gastrointestinal health in humans, is therefore likely to be responsible for the clinical resolution of dysbiosis and, by inference from their consistent representation across the cohort, can represent a healthy core microbiota naturally resistant to metronidazole and capable of performing the functions of the microbiome and restoring the gut microbiota and physiology. In the 1-2 weeks following the completion of antibiotic prophylaxis, a change in the genera represented was apparent with sequence types assigned to *Allobaculum, Clostridium,* and *Peptostreptococcus* spp. increasing in abundance as well as OTUs assigned to the genera *Blautia.* These bacteria therefore form the first organisms to recolonise the gut.

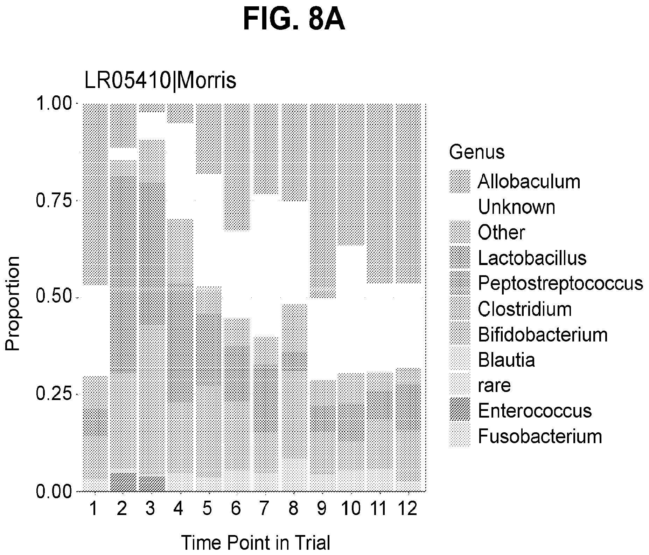

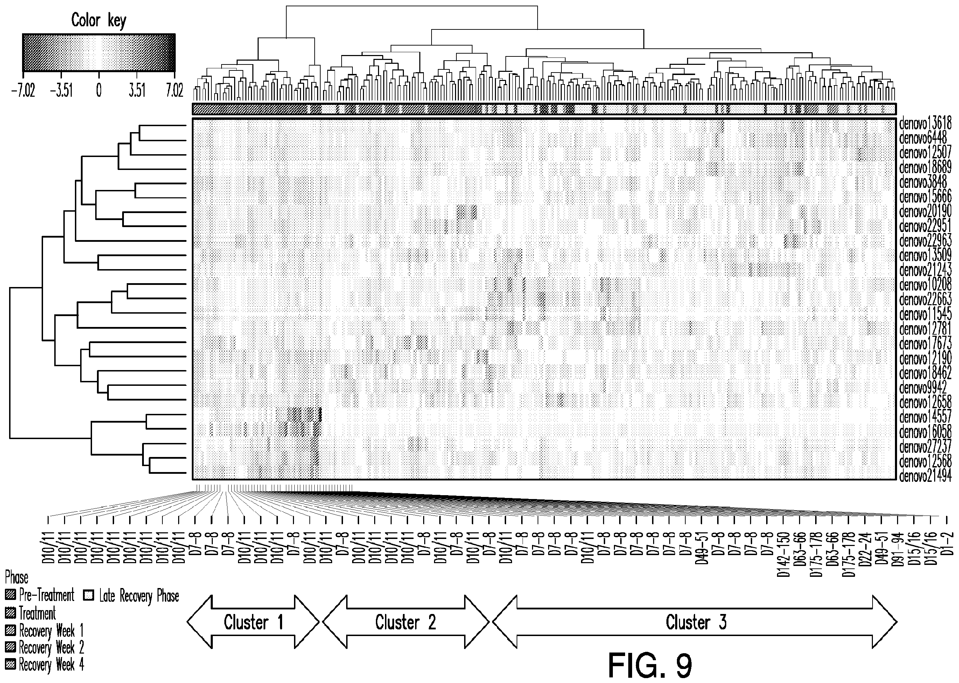

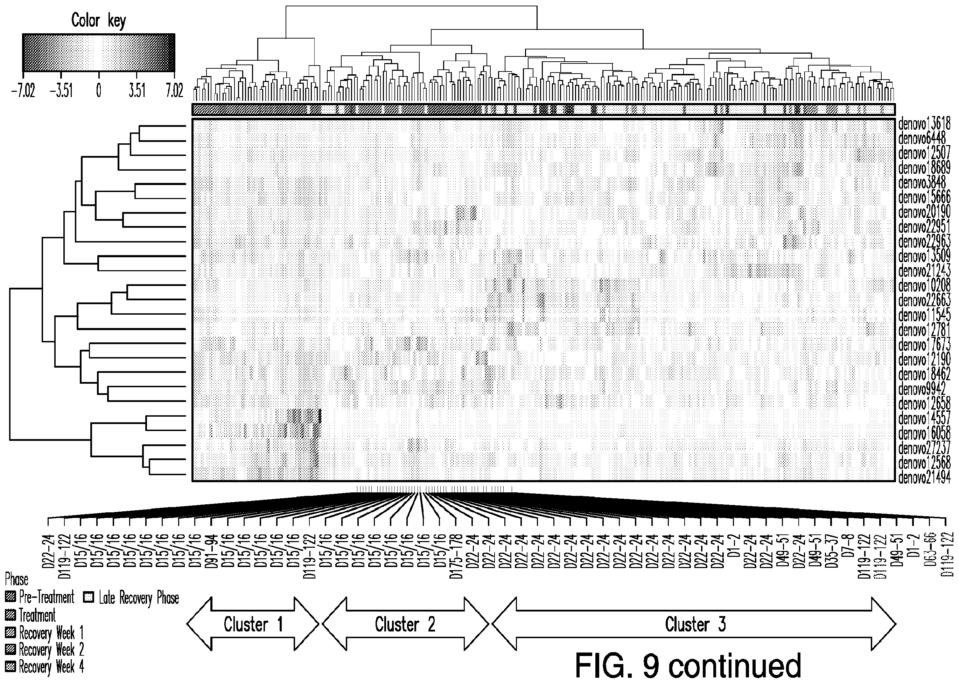

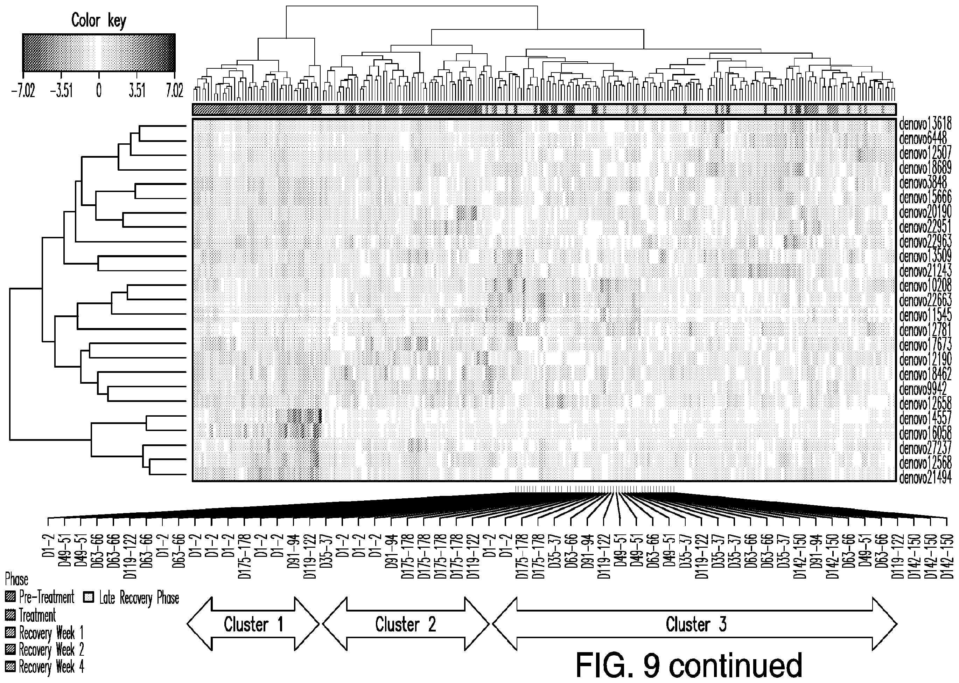

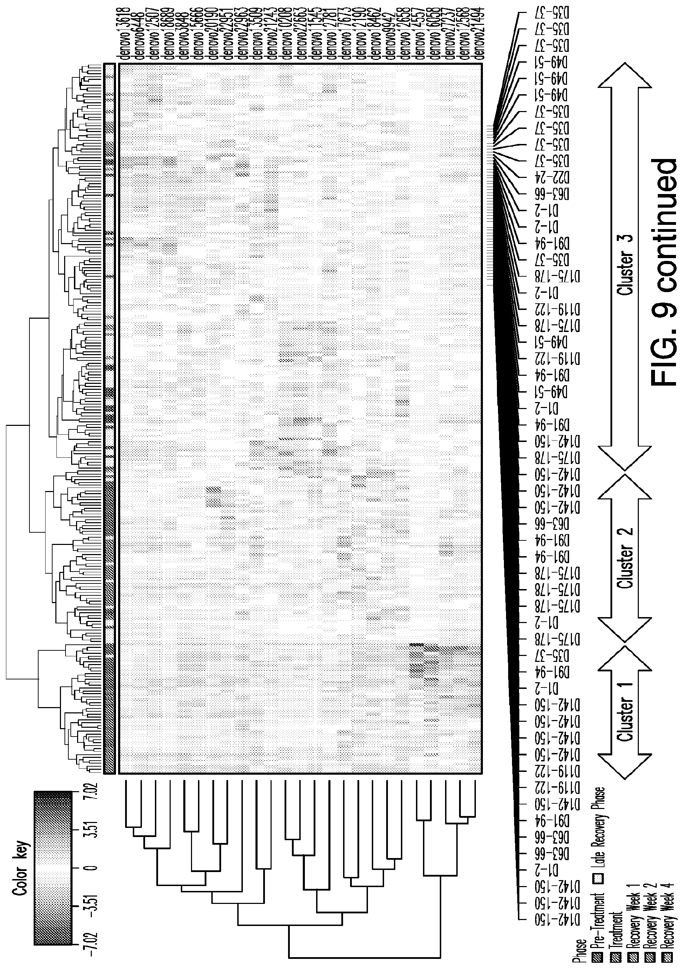

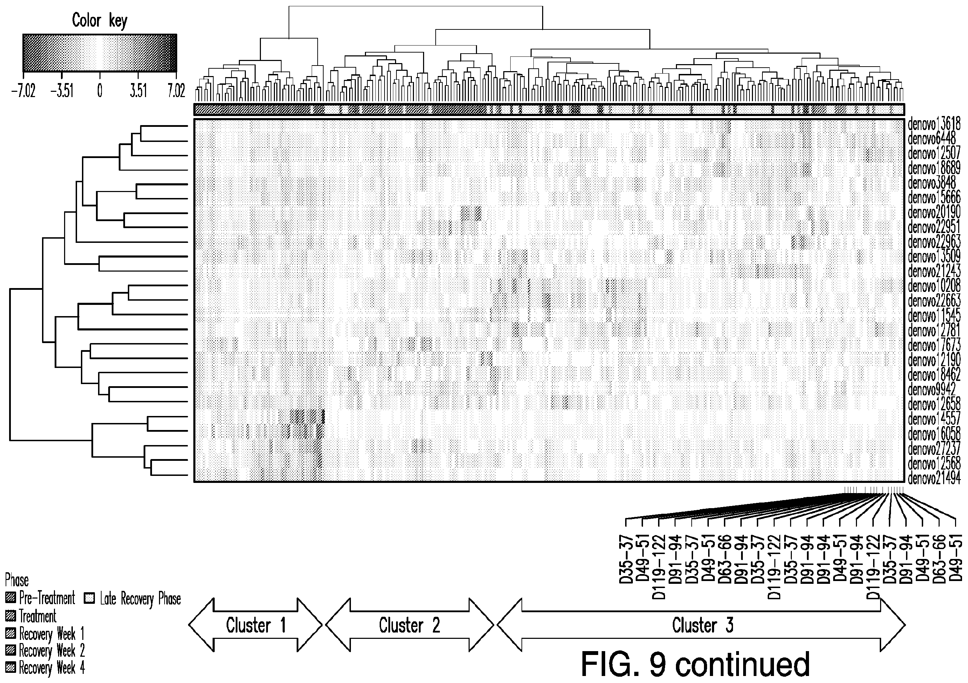

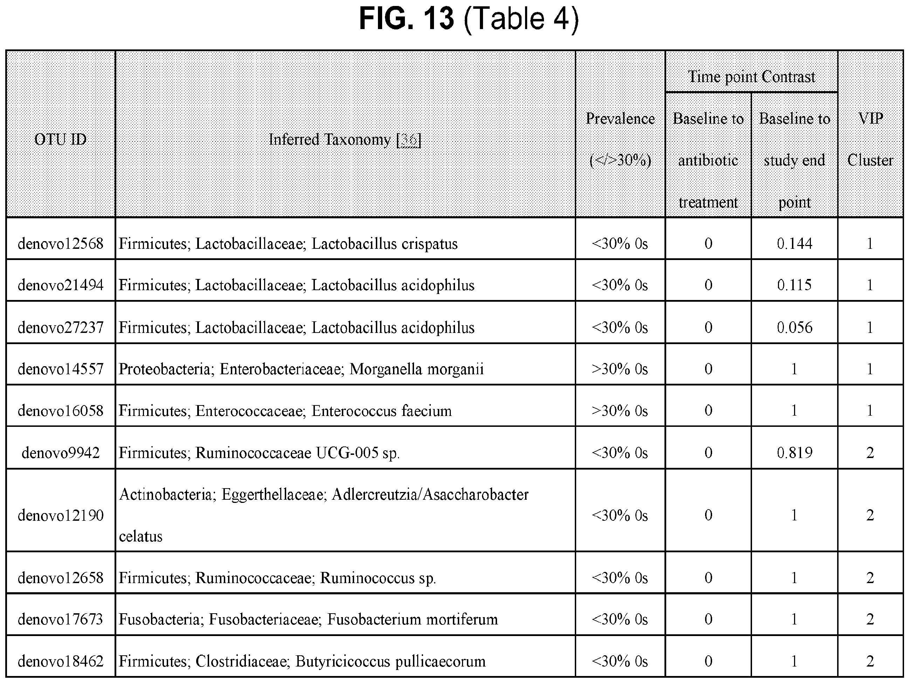

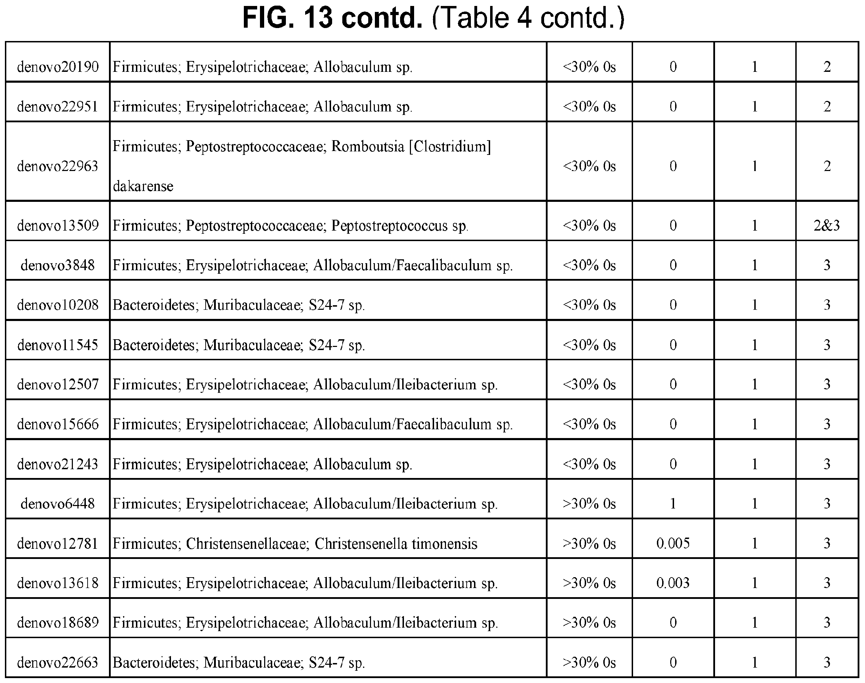

By visual assessment of stacked plots, the treatment and first recovery time points (t=2 and t=3) dominated by lactic acid bacteria were followed in subsequent time points by the completion of treatment. *Peptostreptococcus* and *Allobaculum* genera, before return to a complexity similar to that observed during the baseline phase (Figures 8A-8H). This subset of OTUs best describes those most influential in driving the separation of samples into clusters associated with treatment phase based on their relative abundance profiles in faeces samples from the cohort. The subset comprised 9 OTUs assigned to the genus *Allobaculum,* 3 assigned to *Lactobacillus,* 3 to S24-7, and individual OTUs from the genera *Christensenella, Peptostreptococcus, Romboutsia, Morganella, Adlercreutzia*/*Asaccharobacter, Enterococcus,* and *Butyricicoccus* as well as 2 OTUs assigned to the family Ruminococcaceae (Figure 9 and Figure 13 (Table 4)). During and immediately following metronidazole treatment the relative abundance of three predominant OTUs were influential in the clustering, these were all assigned to the genus *Lactobacillus.* Additionally, two more minor OTUs detected in less than 30% of samples also influenced the clustering of samples into antibiotic and first sampling 2-3 days post-antibiotic therapy based on VIP score. These OTUs were assigned to the genera *Enterococcus* and *Morganella* (Enterobacteriaceae family). All OTUs in the second cluster influential in the early recovery phase during the first two weeks after treatment were prevalent, being detected in greater than 30% of the population. Taxonomic assignment of those OTUs driving the clustering of samples in this early recovery phase following the completion of antibiotic therapy described 2 OTUs from the family Ruminococcaceae and genus *Allobaculum* and one each from the family Eggerthellaceae and the genera *Butyricicoccus, Fusobacterium, Romboutsia,* and *Peptostreptococcus.* Finally, a third cluster defined by PLSDA VIP scores contained samples from the baseline and post-treatment phase 28 days after the completion of antibiotic treatment. OTUs represented at increased abundance within this cluster and prevalent being represented in more than 30% of the sample set included 4 Erysipelotrichaceae sp. assigned to the genus *Allobaculum* and 2 OTUs from the group S24-7, likely Muribaculaceae sp. A further group of lower prevalence OTUs were detected in less than 30% of the samples and included 3 OTUs assigned to the Erysipelotrichaceae genus *Allobaculum*/*Ileibacterium,* and one OTU each assigned to the S24-7 group and *Christensenella* genus. Clusters 1 and 2 (Table 4) therefore represent basic core microbiota with health associated species associated with the restoration of clinical health.

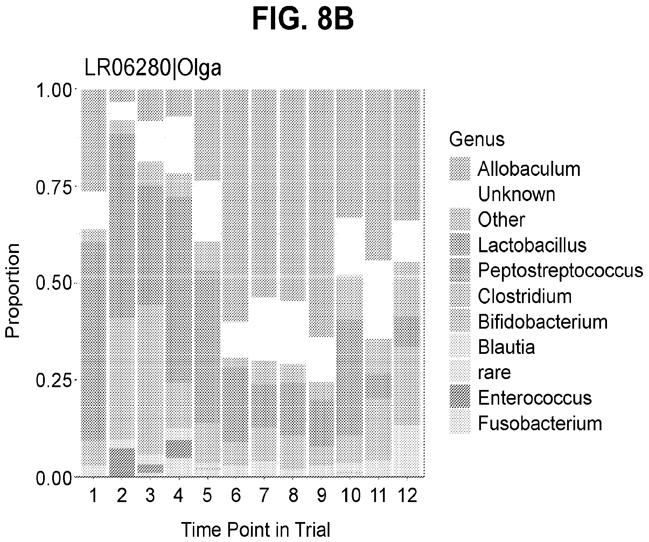

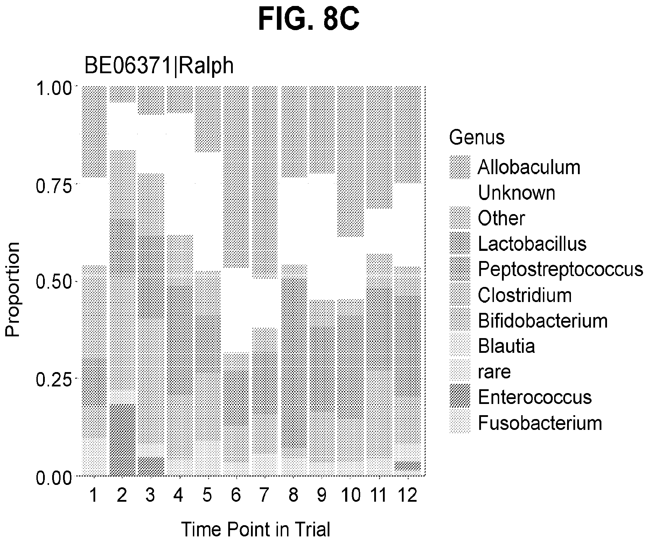

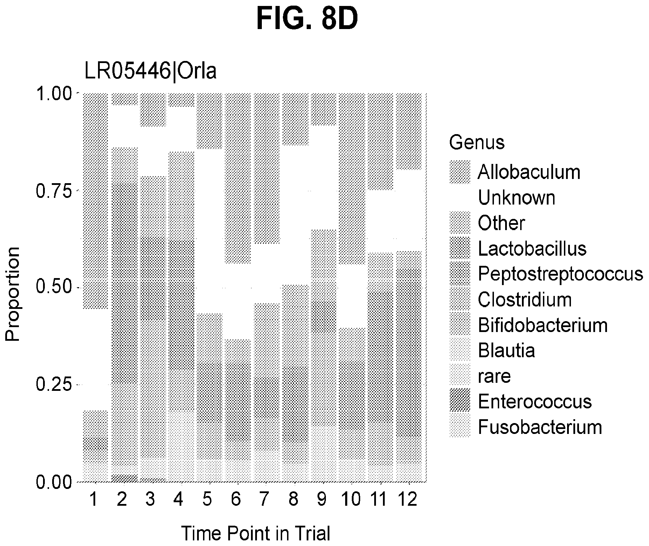

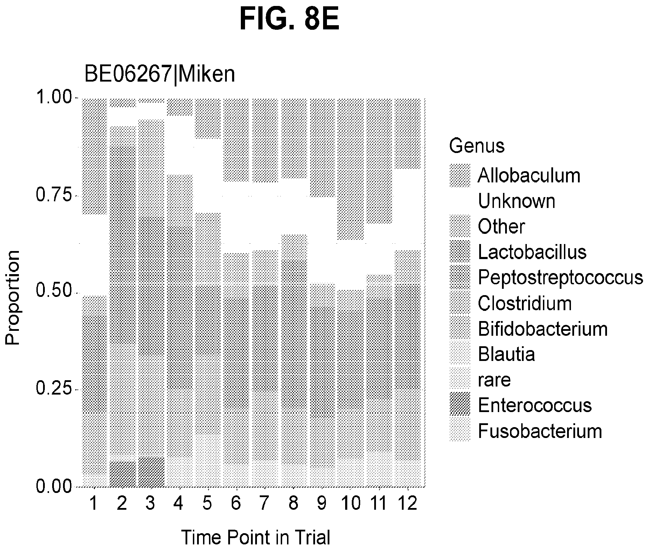

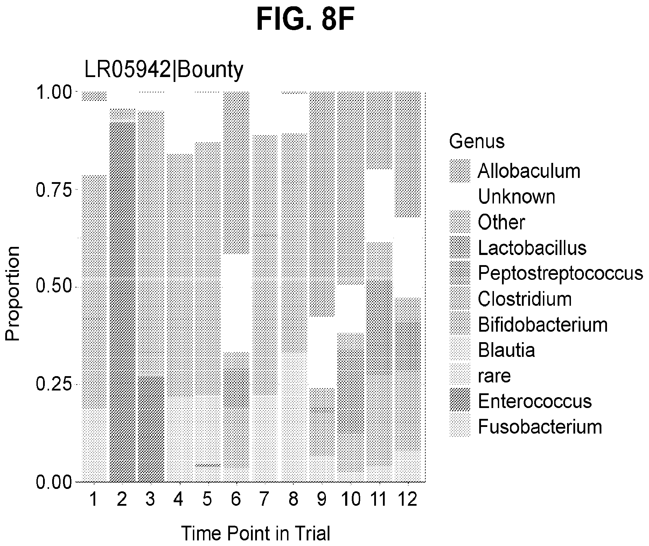

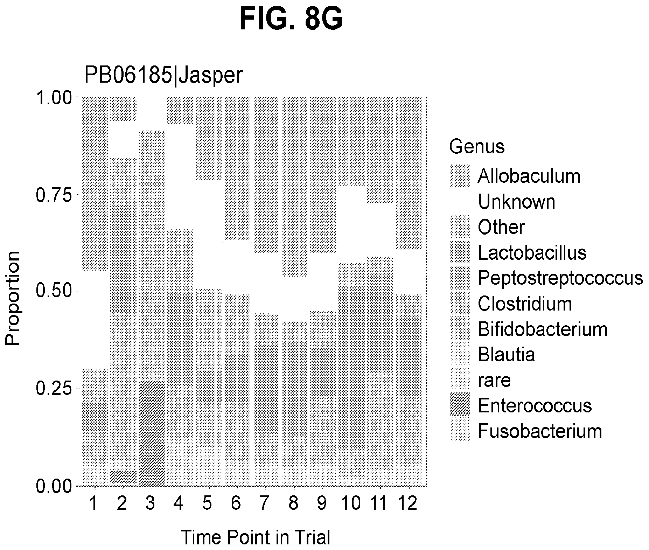

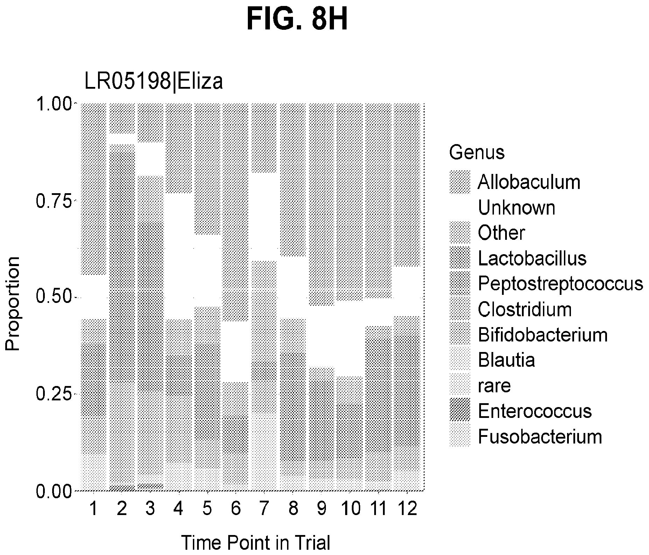

### Detailed description of Figure 8

Stacked bar plots from eight representative dogs within the cohort demonstrating the distribution in the abundant taxonomic groups at each sampling point. Phylogenetic assignment to the genus level is shown as determined by DNA sequence of the 16S rRNA gene v4 region. Abundance is expressed as a proportion relative to the total sequences for the sample. Sequences not assigned a nearest hit in the Green genes database (version 12_10) were collated into the 'Unknown' group; sequences of low abundance for visualisation and those representing less than 0.001% of sequences within the sample were assigned to Other and Rare groups respectively. Pretreatment phase: *t=1 - Baseline;* Treatment phase: *t=2 - Antibiotic administered;* Recovery phase: *t=3 - Early week 1; t=4 - Late week 1; t=5 - week 2; t=6 - week 4; t=7 - week 6; t=8 - week 8; t=9 - month 3; t=10 - month 4; t=11 - month 5; t=12 - month* 6. Genera designations for the eight taxa that were most abundant throughout the study. Taxa that were observed that were not able to be expressed due to low level abundance were classified as Other; those that are not found as commonly were termed Rare; unclassified genera were Unknown.

### Detailed description of Figure 9

Partial least Square discriminate analysis (PLS-DA) correlation plot based on likeness in bacterial abundance data for the 25 OTUs displaying the greatest influence on clustering of the samples (variable importance in projection scores >1). Sample and OTU descriptors have been replaced for ease of visualisation with a colour guide (see key for details). Faeces samples are represented in vertical rows while bacterial OTUs are represented by horizontal rows within the heat plot. The heat map results are read in a similar manner to correlations although values are not constrained to (-1, 1). Dark red or blue sections on the heatmap indicate positively and negatively correlated groups of measurements respectively.

### REFERENCES

[1] Frank et al. (2007) Proc. Natl. Acad. Sci. USA 104, 13780-13785.
[2] Gevers et al. (2014) Cell Host Microbe 15, 382-392.
[3] Ni et al. (2017) Sci. Transl. Med. 9, eaah6888.
[4] Kostic et al. (2013) Cell Host Microbe 14, 207-215.
[5] Johnson and Foster (2018) Nature Reviews Microbiology, Oct;16(10):647-655
[6] Kirchoff et al. (2018) PeerJ Preprints 6:e26990v1
[7] Sharon et al. (2013) Genome research, 23(1), pp.111-120.
[8] Hart et al. (2015) PLoS One. Nov 24;10(11):e0143334
[9] WO2018/006080
[10] Gennaro (2000) Remington: The Science and Practice of Pharmacy. 20th edition, ISBN: 0683306472.
*[11]* Molecular Biology Techniques: An Intensive Laboratory Course, (Ream et al., eds., 1998, Academic Press).
*[12]* Methods In Enzymology (S. Colowick and N. Kaplan, eds., Academic Press, Inc.)
*[13]* Handbook of Experimental Immunology, Vols. I-IV (D.M. Weir and C.C. Blackwell, eds, 1986, Blackwell Scientific Publications)
[14] Sambrook et al. (2001) Molecular Cloning: A Laboratory Manual, 3rd edition (Cold Spring Harbor Laboratory Press).
*[15]* Handbook of Surface and Colloidal Chemistry (Birdi, K.S. ed., CRC Press, 1997)
[16] Ausubel et al. (eds) (2002) Short protocols in molecular biology, 5th edition (Current Protocols).
*[17]* PCR (Introduction to Biotechniques Series), 2nd ed. (Newton & Graham eds., 1997, Springer Verlag)
*[18]* Current Protocols in Molecular Biology (F.M. Ausubel et al., eds., 1987) Supplement 30
[19] Smith & Waterman (1981) Adv. Appl. Math. 2: 482-489.
[20] Guard, B.C., et al., Characterization of microbial dysbiosis and metabolomic changes in dogs with acute diarrhea. PLoS One, 2015. 10(5): p. e0127259.
[21] Bresciani, F., et al., Effect of an extruded animal protein-free diet on fecal microbiota of dogs with food-responsive enteropathy. J Vet Intern Med, 2018. 32(6): p. 1903-1910.
[22] Minamoto, Y., et al., Alteration of the fecal microbiota and serum metabolite profiles in dogs with idiopathic inflammatory bowel disease. Gut Microbes, 2015. 6(1): p. 33-47.

Although the presently disclosed subject matter and its advantages have been described in detail, it should be understood that various changes, substitutions and alterations can be made herein without departing from the scope of the invention as defined by the appended claims. Moreover, the scope of the present application is not intended to be limited to the particular embodiments of the process, machine, manufacture, composition of matter, means, methods and steps described in the specification. As one of ordinary skill in the art will readily appreciate from the disclosure of the presently disclosed subject matter, processes, machines, manufacture, compositions of matter, means, methods, or steps, presently existing or later to be developed that perform substantially the same function or achieve substantially the same result as the corresponding embodiments described herein can be utilized according to the presently disclosed subject matter.

## Claims

1. A method of determining the microbiome biological age status of a canid, comprising
(a) detecting one or more bacterial taxa in a sample obtained from the canid;
(b) correlating the one or more bacterial taxa in the sample to a control data set ; and
(c) determining the microbiome biological age status,
wherein the method further comprises comparing the microbiome biological age status of the canid to the chronological age of the canid.

2. The method of claim 1, wherein the control data set comprises microbiome data of canids at different times or life stages, optionally wherein the control data set comprises microbiome data from at least two lifestages of a canid selected from the list consisting of a puppy, an adult canid, a senior canid and a geriatric canid, optionally wherein the control data set comprises microbiome data from all of puppies, adults, seniors and geriatrics.

3. The method of claim 1 or claim 2, wherein the method comprises quantitating the one or more bacterial taxa.

4. The method of any preceding claim, wherein the bacterial taxa are bacterial species from genera selected from the group consisting of: *Lactobacillus, Terrisporobacter, Turicibacter, Bacteroides, Bifidobacterium, Allobaculum, Blautia, Cetobacterium, Clostridium, Dubosiella, Escherichia, Phascolarctobacterium, Streptococcus, Megamonas, Ruminococcus, Fusobacterium, Enterococcus, Lachnoclostridium, Epulopiscium, Erysipelotrichaceae, Dorea, Adlercreutzia, Anaerobiospirillum, Romboutsia, Peptostreptococcaceae, and Prevotella,* or bacterial species from the families Erysipelotrichaceae Peptostreptococcaceae or Lachnospiraceae, optionally wherein the bacterial taxa are bacterial species selected from the group consisting of *Adlercreutzia equolifaciens, Anaerobiospirillum sp., Bacteroides sp., Blautia sp., Blautia [Ruminococcus] gnavus, Blautia [Ruminococcus] torques, Blautia obeum, Blautia producta, Cetobacterium somerae, Clostridium, Clostridium celatum, Clostridium dakarense, Clostridium hiranonsis, Clostridium perfringens, Clostridium sp., Clostridium sp.K39, Dorea sp., Dubosiella newyorkensis, Enterococcus asini, Enterococcus casseliflavus, Enterococcus haemoperoxidus, Enterococcus sulfureus, Erysipelotrichaceae sp., Escherichia coli* /*Shigella flexneri, Fusobacterium mortiferum, Lachnoclostridium sp., Lachnospiraceae sp., Lactobacillus animalis, Lactobacillus apodemi, Lactobacillus gasseri, Lactobacillus hamsteri, Lactobacillus helveticus, Lactobacillus johnsonii, Lactobacillus murinus, Lactobacillus reuteri, Lactobacillus taiwanensis, Megamonas sp., Niameybacter massiliensis, Peptostreptococcaceae TM5, Phascolarctobacterium sp., Prevotella sp., Romboutsia sp., Ruminococcus gauvreauii group sp., Streptococcus alactolyticus, Streptococcus luteciae, Terrisporobacter sp.,* and *Turicibacter sp,* optionally wherein the bacterial species has a 16S rDNA with at least about 95%, at least about 96%, at least about 97%, at least about 98%, at least about 99% or 100% identity to the sequence of any one of SEQ ID NOs: 32-68.

5. The method of claim 4, wherein the method comprises the detection of one or more bacterial species from genera selected from the group consisting of *Phascolarctobacterium, Bacteroides* and *Escherichia* /*Shigella,* optionally wherein the method comprises the detection of one or more bacterial species selected from the group consisting of *Phascolarctobacterium sp., Bacteroides sp.* and *Escherichia coli*/*Shigella flexneri,* optionally wherein the bacterial species have a 16S rDNA with at least about 95%, at least about 96%, at least about 97%, at least about 98%, at least about 99% or 100% identity to the sequence of any one of SEQ ID NOs: 32-36.

6. The method of claim 5, further comprising: (i) the detection of *Lactobacillus helveticus, Lactobacillus hamsteri, Terrisporobacter sp., Turicibacter sp., Blautia obeum* and *Blautia sp,* optionally wherein the bacterial species have a 16S rDNA with at least about 95%, at least about 96%, at least about 97%, at least about 98%, at least about 99% or 100% identity to the sequence of any one of SEQ ID NOs: 37-41; and/or (ii) the detection of *Clostridium celatum, Clostridium sp., Clostridium disporicum, Clostridium perfringens, Cetobacterium somerae, Lactobacillus johnsonii, Blautia torques, Dubosiella newyorkensis, Streptococcus alactolyticus, Megamonas sp.,* and *Turicibacter sp,* optionally wherein the bacterial species have a 16S rDNA with at least about 95%, at least about 96%, at least about 97%, at least about 98%, at least about 99% or 100% identity to the sequence of any one of SEQ ID NOs: 32, and 42-50, optionally wherein the method comprises the detection of all of SEQ ID NOs: 32, and 42-50.

7. The method of any preceding claim, wherein the bacterial species are from bacterial genera selected from the group consisting of *Clostridium, Collinsella, Enterococcus, Eubacterium, Escherichia-Shigella, Flavonifractor, Klebsiella, Lachnoclostridium, Lachnospiraceae, Lactobacillus, Paeniclostridium, Pediococcus, Peptoclostridium, Romboutsia, Staphylococcus,* and *Tyzzerella,* optionally wherein the bacterial species are selected from the group consisting of *Clostridium sp, Collinsella sp., Enterococcus sp., Escherichia-Shigella sp., Flavonifractor sp., Shigella dysenteriae, Lachnoclostridium sp., Lachnospiraceae sp., Lactobacillus sp., Eubacterium sp., Pediococcus sp., Peptoclostridium sp., Romboutsia sp., Staphylococcus sp.,* and *Tyzzerella sp,* optionally wherein the bacterial species have a 16S rDNA with at least about 95%, at least about 96%, at least about 97%, at least about 98%, at least about 99% or 100% identity to the sequence of any one of SEQ ID NOs: 3-31.

8. The method of claim 7, wherein the bacterium is of the species *Blautia [Ruminococcus] gnavus,* optionally wherein the bacterium has a 16S rDNA with at least about 95%, at least about 96%, at least about 97%, at least about 98%, at least about 99% or 100% identity with the sequence of SEQ ID NO: 20.

9. The method of claim 7 or claim 8, wherein at least 5, at least 10, at least 15, at least 20, at least 25 or at least 28 different taxa are detected and/or quantitated.

10. The method of any one of claims 7-9, comprising: (i) detecting or quantitating *Ruminococcus gnavus, Escherichia sp., Staphylococcus,* and *Flavonifractor,* optionally wherein the bacterial species have a 16S rDNA with at least about 95%, at least about 96%, at least about 97%, at least about 98%, at least about 99% or 100% identity to the sequence of any one of SEQ ID NOs: 13, 16, 17, 20, and 29; and/or (ii) detecting or quantitating *Romboutsia sp., Peptoclostridium sp., Bacteroides sp., Megamonas sp.* and *Collinsella sp,* optionally wherein the bacterial species have a 16S rDNA with at least about 95%, at least about 96%, at least about 97%, at least about 98%, at least about 99% or 100% identity to the sequence of any one of SEQ ID NOs: 10, 15, 22, 26, and 27; and/or (iii) detecting or quantitating *Clostridium sp., Megamonas sp., Paeniclostridium sp, Tyzzerella sp., Romboutsia sp.,* and *Blautia sp,* optionally wherein the bacterial species have a 16S rDNA with at least about 95%, at least about 96%, at least about 97%, at least about 98%, at least about 99% or 100% identity to the sequence of any one of SEQ ID NOs: 3, 4, 8, 9, 14, 18, 19, 21, 23, and 24.

11. A method of monitoring a canid, comprising a step of determining the microbiome biological age status of the animal by the method of any preceding claim on at least two time points, optionally wherein the two time points are 6 months apart.

12. The method of any preceding claim, wherein the control data set is representative of the microbiome of puppies, a microbiome from an adult canid, a microbiome from a senior canid or a microbiome from a geriatric canid.

13. A method of monitoring the microbiome biological age status in a canid that has received a dietary change, a supplement, a functional food, a nutraceutical or a pharmaceutical composition or an exercise/physical activity regimen, which is able to change the microbiome composition, comprising determining the microbiome biological age status by the method of any preceding claim, optionally wherein the microbiome age is determined before and after administration of the composition, optionally wherein the composition comprises bacteria.

14. The method of any preceding claim, wherein: (i) the bacterial species is detected by means of DNA sequencing, RNA sequencing, protein sequence homology or other biological marker indicative of the bacterial species; and/or (ii) the canid's microbiome biological age status is compared to its biological age and the canid's owner is notified when there is a discrepancy; and/or (iii) the sample is a faecal sample, or a sample from the gastrointestinal tract, optionally wherein the sample is a ileal, jejunal, duodenal or colonic sample; and/or (iv) the canid is a dog.

## Patentansprüche

1. Verfahren zur Bestimmung des biologischen Alters eines Mikrobioms eines Caniden, Folgendes umfassend
(a) Nachweisen eines Bakterientaxons oder mehrerer Bakterientaxa in einer Probe, die dem Caniden entnommen wurde;
(b) Korrelieren des einen Bakterientaxons oder der mehreren Bakterientaxa in der Probe mit einem Kontrolldatensatz; und
(c) Bestimmen des biologischen Alters des Mikrobioms,
wobei das Verfahren ferner das Vergleichen des biologischen Alters des *Mikrobioms* des Caniden mit dem chronologischen Alter des Caniden umfasst.

2. Verfahren nach Anspruch 1, wobei der Kontrolldatensatz Mikrobiomdaten von Caniden zu unterschiedlichen Zeitpunkten oder Lebensphasen umfasst, wobei der Kontrolldatensatz optional Mikrobiomdaten von zumindest zwei Lebensphasen eines Caniden umfasst, die aus der Liste ausgewählt sind, die aus einem Welpen, einem erwachsenen Caniden, einem älteren Caniden und einem geriatrischen Caniden besteht, wobei der Kontrolldatensatz optional Mikrobiomdaten sowohl der Welpen als auch der Erwachsenen, Älteren und Geriatrischen umfasst.

3. Verfahren nach Anspruch 1 oder Anspruch 2, wobei das Verfahren das Quantifizieren des einen Bakterientaxons oder der mehreren Bakterientaxa umfasst.

4. Verfahren nach einem der vorhergehenden Ansprüche, wobei die Bakterientaxa bakterielle Arten aus Gattungen sind, die aus der Gruppe ausgewählt sind, die sich aus Folgendem zusammensetzt: *Lactobacillus, Terrisporobacter, Turicibacter, Bacteroides, Bifidobacterium, Allobaculum, Blautia , Cetobacterium, Clostridium, Dubosiella* , *Escherichia, Phascolarctobacterium, Streptococcus, Megamonas, Ruminococcus, Fusobacterium, Enterococcus, Lachnoclostridium, Epulopiscium, Erysipelotrichaceae, Dorea, Adlercreutzia, Anaerobiospirillum, Romboutsia, Peptostreptococcaceae und Prevotella.* oder Bakterienarten der Familien Erysipelotrichaceae Peptostreptococcaceae oder Lachnospiraceae, wobei die Bakterientaxa optional Bakterienarten sind, die ausgewählt sind aus der Gruppe bestehend aus *Adlercreutzia equolifaciens, Anaerobiospirillum sp., Bacteroides sp., Blautia sp., Blautia [Ruminococcus] gnavus, Blautia [Ruminococcus] torques, Blautia obeum, Blautiaproducta, Cetobacterium somerae, Clostridium, Clostridium celatum , Clostridium dakarense, Clostridium hiranonsis, Clostridium perfringens, Clostridium sp., Clostridium sp. K39, Dorea sp., Dubosiella newyorkensis, Enterococcus asini, Enterococcus casseliflavus, Enterococcus haemoperoxidus, Enterococcus sulfureus, Erysipelotrichaceae sp., Escherichia coli*/*Shigella flexneri, Fusobacterium mortiferum, Lachnoclostridium sp., Lachnospiraceae sp., Lactobacillus animalis, Lactobacillus apodemi₁ Lactobacillus gasser,₁ Lactobacillus hamsteri, Lactobacillus helveticus, Lactobacillus johnsonii, Lactobacillus murinus, Lactobacillus reuteri, Lactobacillus taiwanensis, Megamonas sp., Niameybacter massiliensis, Peptostreptococcaceae TM5 , Phascolarctobacterium sp., Prevotella sp. , Romboutsia sp., Ruminococcus gauvreauii group sp., Streptococcus alactolyticus, Streptococcus luteciae, Terrisporobacter sp.* und *Turicibacter sp.,* wobei die Bakterienarten optional eine 16S rDNA mit zumindest etwa 95 %, zumindest etwa 96 %, zumindest etwa 97 %, zumindest etwa 98 %, zumindest etwa 99 % oder 100 % Identität mit der Sequenz einer der SEQ ID NRn : 32-68 aufweisen.

5. Verfahren nach Anspruch 4, wobei das Verfahren den Nachweis *von* einer oder mehreren Bakterienarten aus Gattungen umfasst, die aus der Gruppe ausgewählt sind, die aus *Phascolarctobacterium, Bacteroides* und *Escherichia*/*Shigella* besteht, wobei das Verfahren optional den Nachweis *von* einer oder mehreren Bakterienarten umfasst, die aus der Gruppe ausgewählt sind, die aus *Phascolarctobacterium sp., Bacteroides sp.* und *Escherichia coli*/*Shigella flexneri* besteht, wobei die Bakterienarten optional eine 16S rDNA mit zumindest etwa 95 %, zumindest etwa 96 %, zumindest etwa 97 %, zumindest etwa 98 %, zumindest etwa 99 % oder 100 % Identität mit der Sequenz einer der SEQ ID NRn: 32-36 aufweisen.

6. Verfahren nach Anspruch 5, ferner umfassend: (i) den Nachweis von *Lactobacillus helveticus, Lactobacillus hamsteri, Terrisporobacter sp., Turicibacter sp., Blautia obeum* und *Blautia sp,* wobei die Bakterienarten optional eine 16S rDNA mit zumindest etwa 95 %, zumindest etwa 96 %, zumindest etwa 97 %, zumindest etwa 98 %, zumindest etwa 99 % oder 100 % Identität mit der Sequenz einer der SEQ ID NRN: 37-41 aufweisen; und/oder (ii) den Nachweis von *Clostridium celatum,Clostridium sp., Clostridium disporicum, Clostridium perfringens, Cetobacterium somerae, Lactobacillus johnsonii, Blautia torques, Dubosiella newyorkensis, Streptococcus alactolyticus, Megamonas sp.* und *Turicibacter sp.,* wobei die Bakterienarten optional eine 16S rDNA mit zumindest etwa 95 %, zumindest etwa 96 %, zumindest etwa 97 %, zumindest etwa 98 %, zumindest etwa 99 % oder 100 % Identität mit der Sequenz einer der SEQ ID NRN: 32 und 42-50 aufweisen, wobei das Verfahren den Nachweis aller SEQ ID NRN: 32 und 42-50 umfasst.

7. Verfahren nach einem der vorhergehenden Ansprüche,wobei die Bakterienarten aus Bakteriengattungen stammen, die aus der Gruppe ausgewählt sind, die aus *Clostridium, Collinsella,Enterococcus, Eubacterium, Escherichia-Shigella, Flavonifractor, Klebsiella, Lachnoclostridium, Lachnospiraceae, Lactobacillus, Paeniclostridium, Pediococcus, Peptoclostridium, Romboutsia, Staphylococcus* und *Tyzzerella* besteht, wobei die Bakterienarten optional ausgewählt sind aus der Gruppe bestehend aus *Clostridium sp, Collinsella sp., Enterococcus sp., Escherichia- Shigella sp., Flavonifractor sp., Shigella dysenteriae, Lachnoclostridium sp., Lachnospiraceae sp., Lactobacillus sp., Eubacterium sp., Pediococcus sp.,Peptoclostridium sp., Romboutsia sp., Staphylococcus sp.* und *Tyzzerella sp,* wobei die Bakterienarten optional eine 16S rDNA mit zumindest etwa 95 %, zumindest etwa 96 %, zumindest etwa 97 %, zumindest etwa 98 %, zumindest etwa 99 % oder 100 % Identität mit der Sequenz einer der SEQ ID NRn: 3-31 aufweisen.

8. Verfahren *nach* Anspruch 7, wobei das Bakterium von der Art *Blautia [Ruminococcus] gnavus ist,* wobei das Bakterium optional eine 16S rDNA mit zumindest etwa 95 %, zumindest etwa 96 %, zumindest etwa 97 %, zumindest etwa 98 %, zumindest etwa 99 % oder 100 % Identität mit der Sequenz der SEQ ID NR: 20 aufweist.

9. Verfahren nach Anspruch 7 oder Anspruch 8, wobei zumindest 5, zumindest 10, zumindest 15, zumindest 20, zumindest 25 oder zumindest 28 verschiedene Taxa nachgewiesen und/oder quantifiziert werden.

10. Verfahren nach einem der Ansprüche 7-9, Folgendes umfassend: (i) Nachweisen oder Quantifizieren von *Ruminococcus gnavus, Escherichia sp., Staphylococcusn* und *Flavonifractor,* wobei die Bakterienarten optional eine 16S rDNA mit zumindest etwa 95 %, zumindest etwa 96 %, zumindest etwa 97 %, zumindest etwa 98 %, zumindest etwa 99 % oder 100 % Identität mit der Sequenz einer der SEQ ID NRn:13,16,17, 20 und 29 aufweisen; und/oder (ii) Nachweisen oder Quantifizieren von *Romboutsia sp., Peptoclostridium sp., Bacteroides sp., Megamonas sp.* und *Collinsella sp,* wobei die Bakterienarten optional eine 16S rDNA mit zumindest etwa 95%, zumindest etwa 96 %, zumindests etwa 97 %, zumindest etwa 98 %, zumindest etwa 99 % oder 100 % Identität mit der Sequenz einer der SEQ ID NRN:10, 15, 22, 26 und 27 **aufweisen;** und/oder (iii) Nachweisen oder Quantifizieren von *Clostridium sp., Megamonas sp., Paeniclostridium sp, Tyzzerella sp., Romboutsia sp.* und *Blautia sp,* wobei die Bakterienarten optional eine 16S rDNA mit zumindest etwa 95 %, zumindest etwa 96 %, zumindest etwa 97 %, zumindest etwa 98 %, zumindest etwa 99 % oder 100 % Identität mit der Sequenz einer der SEQ ID NRn: 3, 4, 8, 9, 14, 18, 19, 21, 23 und 24 aufweisen.

11. Verfahren zur Überwachung eines Caniden, umfassend einen Schritt des Bestimmens des biologischen Alters des Mikrobioms des Tiers durch das Verfahren nach einem der vorhergehenden Ansprüche zu zumindest zwei Zeitpunkten, wobei die zwei Zeitpunkte wahlweise 6 Monate auseinanderliegen.

12. Verfahren nach einem der vorangehenden Ansprüche, wobei der Kontrolldatensatz repräsentativ für das Mikrobiom von Welpen, ein Mikrobiom eines erwachsenen Caniden, ein Mikrobiom eines älteren Caniden oder ein Mikrobiom eines geriatrischen Caniden ist.

13. Verfahren zur Überwachung des biologischen Alters des Mikrobioms in einem Caniden, der eine Ernährungsumstellung, ein Nahrungsergänzungsmittel, ein funktionelles Nahrungsmittel, eine nutrazeutische oder pharmazeutische Zusammensetzung oder ein Trainings-/Bewegungsprogramm erhalten hat, das die Mikrobiomzusammensetzung ändern kann, umfassend das Bestimmen des biologischen Alters des Mikrobioms durch das Verfahren nach einem der vorhergehenden Ansprüche, wobei das Alter des Mikrobioms optional vor und nach der Verabreichung der Zusammensetzung bestimmt wird, wobei die Zusammensetzung optional Bakterien umfasst.

14. Verfahren nach einem der vorhergehenden Ansprüche,wobei: (i) die Bakterienart mittels DNA-Sequenzierung, RNA-Sequenzierung, Proteinsequenzhomologie oder anderer biologischer Marker, die die Bakterienart anzeigen, nachgewiesen wird; und/oder (ii) das biologische Alter des Mikrobioms des Caniden mit seinem biologischen Alter verglichen wird und der Besitzer des Caniden benachrichtigt wird, wenn eine Abweichung vorliegt; und/oder (iii) die Probe eine Fäkalprobe oder eine Probe aus dem Gastrointestinaltrakt ist, wobei die Probe optional eine Ileal-, Jejunal-, Duodenal- oder Kolonprobe ist; und/oder (iv) der Canide ein Hund ist.

## Revendications

1. Procédé de détermination de l'état d'âge biologique du microbiome d'un canidé, comprenant
(a) la détection d'une ou plusieurs taxons bactériens dans un échantillon obtenu à partir du canidé ;
(b) la mise en corrélation des un ou plusieurs taxons bactériens dans l'échantillon avec un ensemble de données de contrôle; et
(c) la détermination de l'état d'âge biologique du microbiome, dans lequel le procédé comprend en outre la comparaison de l'état d'âge biologique du *microbiome* du canidé à l'âge chronologique du canidé.

2. Procédé selon la revendication 1, dans lequel l'ensemble de données de contrôle comprend des données de microbiomes de canidés à différents moments ou stades de vie, facultativement dans lequel l'ensemble de données de contrôle comprend des données de microbiomes provenant d'au moins deux stades de vie d'un canidé choisis dans la liste constituée d'un chiot, d'un canidé adulte, d'un canidé âgé et d'un canidé gériatrique, facultativement dans lequel l'ensemble de données de contrôle comprend des données de microbiomes provenant de tous les chiots, canidés adultes, âgés et gériatriques.

3. Procédé selon la revendication 1 ou la revendication 2, dans lequel le procédé comprend la quantification des un ou plusieurs taxons bactériens.

4. Procédé selon l'une quelconque des revendications précédentes, dans lequel les taxons bactériens sont des espèces bactériennes provenant de genres choisis dans le groupe constitué de : *Lactobacillus, Terrisporobacter, Turicibacter, Bacteroides, Bifidobacterium, Allobaculum, Blautia, Cetobacterium, Clostridium, Dubosiella, Escherichia, Phascolarctobacterium, Streptococcus, Megamonas, Ruminococcus, Fusobacterium, Enterococcus, Lachnoclostridium, Epulopiscium, Erysipelotrichaceae, Dorea, Adlercreutzia, Anaerobiospirillum, Romboutsia, Peptostreptococcaceae* et *Prevotella.* ou des espèces bactériennes des familles Erysipelotrichaceae Peptostreptococcaceae ou Lachnospiraceae, facultativement dans lequel les taxons bactériens sont des espèces bactériennes choisies dans le groupe constitué de *Anaerobiospirillum sp., Bacteroides sp., Blautia sp., Blautia [Ruminococcus] gnavus, Blautia [Ruminococcus] torques, Blautia obeum, Blautiaproducta, Cetobacterium somerae, Clostridium, Clostridium celatum , Clostridium dakarense, Clostridium hiranonsis, Clostridium perfringens, Clostridium sp., Clostridium sp.K39, Dorea sp., Dubosiella newyorkensis, Enterococcus asini, Enterococcus casseliflavus, Enterococcus haemoperoxidus, Enterococcus sulfureus, Erysipelotrichaceae sp., Escherichia coli* /*Shigella flexneri, Fusobacterium mortiferum, Lachnoclostridium sp., Lachnospiraceae sp., Lactobacillus animalis, Lactobacillus apodemil Lactobacillus gasser,1 Lactobacillus hamsteri, Lactobacillus helveticus, Lactobacillus johnsonii, Lactobacillus murinus, Lactobacillus reuteri, Lactobacillus taiwanensis, Megamonas sp., Niameybacter massiliensis, Peptostreptococcaceae TM5, Phascolarctobacterium sp., Prevotella sp., Romboutsia sp., Ruminococcus gauvreauii group sp., Streptococcus alactolyticus, Streptococcus luteciae, Terrisporobacter sp. et Turicibacter sp.,* facultativement dans lequel l'espèce bactérienne présente un ADNr 16S avec au moins environ 95 %, au moins environ 96 %, au moins environ 97 %, au moins environ 98 %, au moins environ 99 % ou 100 % d'identité avec la séquence de l'une quelconque des SEQ ID N0s: 32-68.

5. Procédé selon la revendication 4, dans lequel le procédé comprend la détection d'une ou de plusieurs espèces bactériennes à partir de genres choisis dans le groupe constitué par *Phascolarctobacterium, Bacteroides* et *Escherichia* /*Shigella,* facultativement dans lequel le procédé comprend la détection d'une ou plusieurs espèces bactériennes choisies dans le groupe constitué par *Phascolarctobacterium sp., Bacteroides sp.* et *Escherichia coli*/*Shigella flexneri,* facultativement dans lequel les espèces bactériennes présentent un ADNr 16S avec au moins environ 95 %, au moins environ 96 %, au moins environ 97 %, au moins environ 98 %, au moins environ 99 % ou 100 % d'identité avec la séquence de l'une quelconque des SEQ ID N° : 32-36.

6. Procédé selon la revendication 5, comprenant en outre : (i) la détection de *Lactobacillus helveticus, Lactobacillus hamsteri, Terrisporobacter sp., Turicibacter sp., Blautia obeum* et *Blautia sp,* facultativement dans lequel les espèces bactériennes présentent un ADNr 16S avec au moins environ 95 %, au moins environ 96 %, au moins environ 97 %, au moins environ 98 %, au moins environ 99 % ou 100 % d'identité avec la séquence de l'une quelconque des SEQ ID N° : 37-41 ; et/ou (ii) la détection de *Clostridium celatum* , *Clostridium sp., Clostridium disporicum, Clostridium perfringens, Cetobacterium somerae, Lactobacillus johnsonii, Blautia torques, Dubosiella newyorkensis, Streptococcus alactolyticus, Megamonas sp.* et *Turicibacter sp.* facultativement dans lequel les espèces bactériennes présentent un ADNr 16S avec au moins environ 95 %, au moins environ 96 %, au moins environ 97 %, au moins environ 98 %, au moins environ 99 % ou 100 % d'identité avec la séquence de l'une quelconque des SEQ ID N°: 32 et 42-50, facultativement dans lequel le procédé comprend la détection de la totalité de SEQ ID N° : 32 et 42-50.

7. Procédé selon l'une quelconque des revendications précédentes,dans lequel les espèces bactériennes proviennent de genres bactériens choisis dans le groupe constitué de *Clostridium, Collinsella* , *Enterococcus, Eubacterium, Escherichia-Shigella, Flavonifractor, Klebsiella, Lachnoclostridium* , *Lachnospiraceae, Lactobacillus , Paeniclostridium, Pediococcus, Peptoclostridium, Romboutsia, Staphylococcus et Tyzzerella,* facultativement dans lequel les espèces bactériennes sont choisies dans le groupe constitué de *Clostridium sp, Collinsella sp., Enterococcus sp. , Escherichia- Shigella sp.* , *Flavonifractor sp. , Shigella dysenteriae, Lachnoclostridium sp. , Lachnospiraceae sp., Lactobacillus sp., Eubacterium sp.*, *Pediococcus sp. , Peptoclostridium sp., Romboutsia sp., Staphylococcus sp. et Tyzzerella sp.,* facultativement dans lequel les espèces bactériennes présentent un ADNr 16S avec au moins environ 95 %, au moins environ 96 %, au moins environ 97 %, au moins environ 98 %, au moins environ 99 % ou 100 % d'identité avec la séquence de l'une quelconque des SEQ ID N°: 3-31.

8. *Procédé* selon la revendication 7, dans lequel la bactérie est de l'espèce *Blautia [Ruminococcus] gnavus,* facultativement dans lequel la bactérie présente un ADNr 16S avec au moins environ 95 %, au moins environ 96 %, au moins environ 97 %, au moins environ 98 %, au moins environ 99 % ou 100 % d'identité avec la séquence de SEQ ID N°: 20.

9. Procédé selon la revendication 7 ou la revendication 8, dans lequel au moins 5, au moins 10, au moins 15, au moins 20, au moins 25 ou au moins 28 taxons différents sont détectés et/ou quantifiés.

10. *Procédé* selon l'une quelconque des revendications 7 à 9, comprenant : (i) la détection ou la quantification de *Ruminococcus gnavus, Escherichia sp., Staphylococcus* et *Flavonifractor,* facultativement dans lequel les espèces bactériennes présentent un ADNr 16S avec au moins environ 95 %, au moins environ 96 %, au moins environ 97 %, au moins environ 98 %, au moins environ 99 % ou 100 % d'identité avec la séquence de l'une quelconque des SEQ ID N° : 13,16,17, 20, et 29 ; et/ou (ii) la détection ou la quantification de *Romboutsia sp., Peptoclostridium sp., Bacteroides sp., Megamonas sp.* et *Collinsella sp,* facultativement dans lequel les espèces bactériennes présentent un ADNr 16S avec au moins environ 95 %, au moins environ 96 %, au moins environ 97 %, au moins environ 98 %, au moins environ 99 % ou 100 % d'identité avec la séquence de l'une quelconque des SEQ ID N° : 10,15, 22, 26, et 27 ; et/ou (iii) la détection ou la quantification de *Clostridium sp., Megamonas sp., Paeniclostridium sp., Tyzzerella sp., Romboutsia sp.* et *Blautia sp,* facultativement dans lequel les espèces bactériennes présentent un ADNr 16S avec au moins environ 95 %, au moins environ 96 %, au moins environ 97 %, au moins environ 98 %, au moins environ 99 % ou 100 % d'identité avec la séquence de l'une quelconque des SEQ ID N° : 3, 4, 8, 9,14,18,19,21 , 23 et 24.

11. Procédé de surveillance d'un canidé, comprenant une étape de détermination de l'état d'âge biologique du microbiome de l'animal par le procédé selon l'une quelconque des revendications précédentes à au moins deux points temporels, facultativement dans lequel les deux points temporels sont espacés de 6 mois.

12. Procédé selon l'une quelconque des revendications précédentes, dans lequel l'ensemble de données de contrôle est représentatif du microbiome des chiots, d'un microbiome d'un canidé adulte, d'un microbiome d'un canidé âgé ou d'un microbiome d'un canidé gériatrique.

13. Procédé de surveillance de l'état d'âge biologique du microbiome chez un canidé ayant reçu un changement de régime alimentaire, un supplément, un aliment fonctionnel, un nutraceutique ou une composition pharmaceutique ou un régime d'exercice/activité physique, qui est capable de changer la composition de microbiome, comprenant la détermination de l'état d'âge biologique du microbiome par le procédé selon l'une quelconque des revendications précédentes, facultativement dans lequel l'âge du microbiome est déterminé avant et après l'administration de la composition, facultativement dans lequel la composition comprend des bactéries.

14. Procédé selon l'une quelconque des revendications précédentes, dans lequel : (i) l'espèce bactérienne est détectée au moyen d'un séquençage d'ADN, de séquençage d'ARN, d'homologie de séquence protéique ou d'un autre marqueur biologique indicatif de l'espèce bactérienne ; et/ou (ii) l'état d'âge biologique du microbiome du canidé est comparé à son âge biologique et le propriétaire du canidé est averti en cas de divergence ; et/ou (iii) l'échantillon est un échantillon fécal, ou un échantillon provenant du tractus gastro-intestinal, facultativement dans lequel l'échantillon est un échantillon iléal, jéjunal, duodénal ou du côlon ; et/ou (iv) le canidé est un chien.
